# EUROPEAN PATENT APPLICATION

(11) **EP 4 814 116 A1**
(43) Date of publication of application: **30.09.2026**
(21) Application number: 24894239.3
(22) Date of filing: 22.11.2024
(51) Int. Cl.: C12N 15/09, A01K 67/0275, A61K 31/7125, A61K 48/00, A61P 43/00, C12N 15/11

(54) **COMPOSITION FOR NUCLEOTIDE SEQUENCE EDITING, AND NUCLEOTIDE SEQUENCE EDITING METHOD USING SAME**

(30) Priority: 24.11.2023 JP 2023199332
(71) Applicant: Hiroshima University, Higashi-Hiroshima-shi Hiroshima 739-8511 (JP); Daiichi Sankyo Company, Limited, Tokyo 103-8426 (JP)
(72) Inventor: KAMIYA, Hiroyuki, Hiroshima 739-8511 (JP); KAWAI, Hidehiko, Hiroshima 739-8511 (JP); KOIZUMI, Makoto, Tokyo 103-8426 (JP); SHOJI, Takao, Tokyo 103-8426 (JP)
(74) Representative: Marks & Clerk LLP
(86) International application number: PCT/JP2024/041539
(87) International publication number: WO 2025/110249

(57) **Abstract**

Provided is a composition and others, the composition containing a first single-stranded polynucleotide and a third single-stranded polynucleotide, wherein the first single-stranded polynucleotide is 50 to 200 nucleotides in length, and has a base sequence having a sequence identity of 80% or more with a partial base sequence of an editing target strand in a target region present in an intracellular double-stranded DNA and containing at least one mutation for editing selected from the group consisting of deletion, substitution, and insertion of one or more nucleotides as compared with a base sequence of the editing target strand, and the third single-stranded polynucleotide is 15 to 200 nucleotides in length, and has a base sequence having a sequence identity of 90% or more with a base sequence in the editing target strand 5' or 3' to a position corresponding to the mutation for editing in the first single-stranded polynucleotide, wherein, in the editing target strand, the base sequence having sequence identity with the first single-stranded polynucleotide and the base sequence having sequence identity with the third single-stranded polynucleotide are overlapping, adjacent, or separated by one to nine bases.

## Description

### TECHNICAL FIELD

The present invention relates to a composition for base sequence editing, a base sequence editing method using the composition, and others.

### BACKGROUND ART

Since CRISPR-Cas9 was reported in 2013, many researchers have come to conduct genome editing (NPLs 1 and 2). Genome editing techniques themselves had existed prior to that, and methods with ZFN (zinc-finger nuclease) or transcription activator-like effector nuclease (TALEN) had been known; but it had not been easy to design and construct a system for editing desired sites in genomes, and hence those methods had been used by only a small number of researchers. In editing with CRISPR-Cas9, the sequence of a nucleic acid called guide RNA specifies cleavage positions. This enables editing with CRISPR-Cas9 just by using different guide RNAs for different target sites, and hence CRISPR-Cas9 has been widely used as an experimental technique. Moreover, CRISPR-Cas9 has begun to be applied to improvement or the like of agricultural crops and domestic animals.

When CRISPR-Cas9 or another artificial nuclease cleaves a DNA at a target site in the genome, an error is generated at high frequency in repairing in the cell, and by utilizing this phenomenon a gene of interest can be knocked out (making the functions lost). If a donor DNA containing a sequence having homology with a cleavage site is present when the cleavage occurs, the DNA may be incorporated though the frequency is low, and by utilizing this phenomenon a desired sequence can be inserted and a base substitution mutation or the like can be introduced. However, involving DNA break, genome editing with such an artificial nuclease causes a mutation at an off-target site or an unexpected mutation at an on-target site (NPLs 3 and 4), and hence attempts to apply them to healthcare need to be made with extreme care. In addition, the intellectual property rights to the techniques are possessed by foreign organizations, and thus the techniques are not readily accessible for domestic companies in Japan.

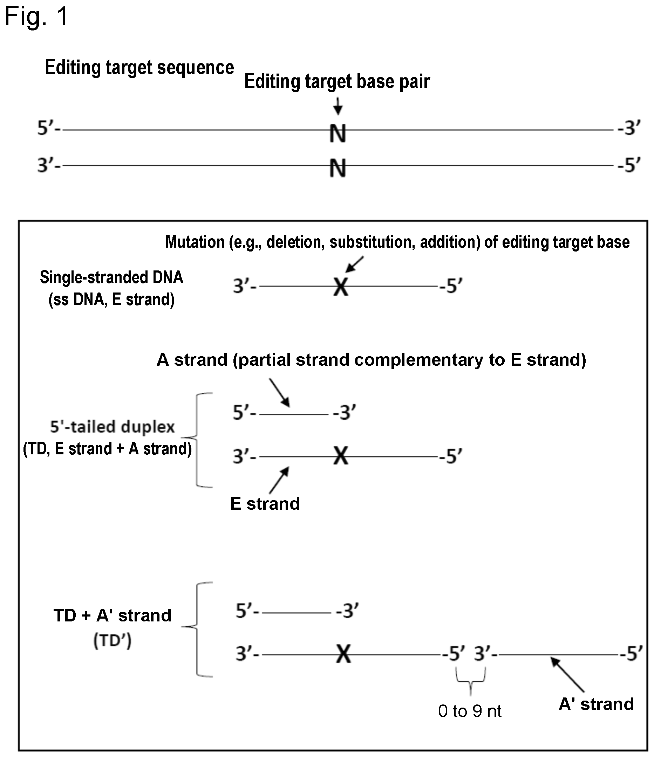

Meanwhile, a base sequence editing method with a single-stranded DNA (ss DNA) or a 5'-tailed duplex (TD) (PTLs 1 and 2), the method developed by Kamiya et al., is known as a base sequence editing method without use of any artificial nuclease (Fig. 1). Regarding the base sequence editing method with a TD, Kawai et al. examined the influence of the lengths of editor strands (an E strand, a long strand containing a mutation for a target sequence) of TDs on editing efficiency, and found that use of a TD with an E strand having a length of 100 nucleotides or less gave higher editing efficiency than use of a TD with an E strand having a length of several hundreds of nucleotides (NPLs 5 and 6).

PTL 3 describes a genome editing technique with a polynucleotide in a single-stranded form. A polynucleotide for editing that is used in the technique has a primary editing site, and it is also disclosed that a polynucleotide for promoting editing that has a region overlapping with complementary sequences in a 5'-end part and 3'-end part of the polynucleotide for editing is used. PTL 3 also discloses that the polynucleotide for editing contains a nucleotide analog having high affinity for DNA as a constituent unit, and that enhanced editing activity was found only in the case that the nucleotide analog having high affinity for DNA was contained at a specific site in the 5'-end part and 3'-end part of the polynucleotide for editing.

### CITATION LIST

### PATENT LITERATURE

PTL 1: International Publication No. WO 2005/075657
PTL 2: International Publication No. WO 2006/064813
PTL 3: International Publication No. WO 2023/190848

### NON PATENT LITERATURE

NPL 1: L. Cong, F. A. Ran, D. Cox, S. Lin, R. Barretto, N. Habib, P. D. Hsu, X. Wu, W. Jiang, L. A. Marraffini, F. Zhang. Multiplex genome engineering using CRISPR/Cas systems. Science 339, 819-823 (2013)
NPL 2: P. Mali, L. Yang, K. M. Esvelt, J. Aach, M. Guell, J. E. DiCarlo, J. E. Norville, G. M. Church. RNA-guided human genome engineering via Cas9. Science 339, 823-826 (2013)
NPL 3: Y. Fu, J. A. Foden, C. Khayter, M. L. Maeder, D. Reyon, J. K. Joung, J. D. Sander. High-frequency off-target mutagenesis induced by CRISPR-Cas nucleases in human cells. Nat. Biotechnol. 31, 822-826 (2013)
NPL 4: M. Kosicki, K. Tomberg, A. Bradley. Repair of double-strand breaks induced by CRISPR-Cas9 leads to large deletions and complex rearrangements. Nat. Biotechnol. 36, 765-771 (2018)
NPL 5: H. Kawai, K. Yazama, Y. Yanai, R. Kamitsubo, H. Kamiya. Gene correction by 5'-tailed duplexes with short editor oligodeoxyribonucleotides. J. Biosci. Bioengng. 132, 552-559 (2021)
NPL 6: H. Kawai, R. Kamitsubo, H. Kamiya. Correction of monomeric enhanced green fluorescent protein (mEGFP) gene by short 5'-tailed duplexes. J. Biosci. Bioengng. 134, 175-181 (2022)

### SUMMARY OF INVENTION

### TECHNICAL PROBLEM

Further enhanced editing efficiency has been demanded for base sequence editing techniques without use of any nuclease such as the base sequence editing method disclosed in PTL 1 or 2 with ss DNAs or TDs.

### SOLUTION TO PROBLEM

The present inventors found that substantially enhanced editing efficiency is achieved with an E strand or TD (E strand + A strand) to which an additional nucleic acid strand (A' strand) having sequence identity with an editing target strand with which the E strand has sequence identity has been added. In addition, it was found that introduction of a specific chemical modification at a specific position in an E strand or an A strand gives substantially enhanced editing efficiency. The present inventors, through further diligent study, have completed the present invention.

Specifically, the present invention includes the following.
[1] A composition containing a first single-stranded polynucleotide and a third single-stranded polynucleotide, wherein
   the first single-stranded polynucleotide is 50 to 200 nucleotides in length, and has a base sequence having a sequence identity of 80% or more with a partial base sequence of an editing target strand in a target region present in an intracellular double-stranded DNA and containing at least one mutation for editing selected from the group consisting of deletion, substitution, and insertion of one or more nucleotides as compared with a base sequence of the editing target strand, and
   the third single-stranded polynucleotide is 15 to 200 nucleotides in length, and has a base sequence having a sequence identity of 90% or more with a base sequence in the editing target strand 5' or 3' to a position corresponding to the mutation for editing in the first single-stranded polynucleotide, wherein, in the editing target strand, the base sequence having sequence identity with the first single-stranded polynucleotide and the base sequence having sequence identity with the third single-stranded polynucleotide are overlapping, adjacent, or separated by one to nine bases.
   Preferably, the composition contains neither an exogenous nuclease nor a polynucleotide capable of expressing it, and is not used in combination with any of them.
[2] The composition according to [1], wherein the position of the mutation for editing in the first single-stranded polynucleotide is present within a range of 25 to 75% from the 5' end, with the full length from the 5' end to the 3' end defined as 100%.
[3] The composition according to [2], wherein the position of the mutation for editing in the first single-stranded polynucleotide is present within a range of 49 to 51% from the 5' end, with the full length from the 5' end to the 3' end defined as 100%.
[4] The composition according to any of [1] to [3], wherein the first single-stranded polynucleotide is 70 to 100 nucleotides in length.
[5] The composition according to any of [1] to [4], wherein, in the first single-stranded polynucleotide, a sequence excluding the position of the mutation for editing and a region of 10 bases from the 5' end and a region of 10 bases from the 3' end from the first single-stranded polynucleotide consists of a base sequence 100% identical to a corresponding base sequence in the editing target strand.
[6] The composition according to any of [1] to [5], wherein the base sequence of the third single-stranded polynucleotide has a sequence identity of 90% or more with a base sequence in the editing target strand 5' to the position corresponding to the mutation for editing in the first single-stranded polynucleotide.
[7] The composition according to any of [1] to [6], wherein, in the editing target strand, the base sequence having sequence identity with the first single-stranded polynucleotide and the base sequence having sequence identity with the third single-stranded polynucleotide are adjacent.
[8] The composition according to any of [1] to [7], wherein the third single-stranded polynucleotide is 55 to 75 nucleotides in length.
[9] The composition according to any of [1] to [8], wherein the third single-stranded polynucleotide consists of a base sequence 100% identical to a corresponding base sequence in the editing target strand.
[10] The composition according to any of [1] to [9], having at least one of the following features:
   (a) at least one nucleotide in a region consisting of the position of the mutation for editing and a 10-base sequence 5' adjacent to the position of the mutation for editing and a 10-base sequence 3' adjacent to the mutation for editing in the first single-stranded polynucleotide is a sugar-modified nucleotide; and
   (b) the first single-stranded polynucleotide contains at least one modified internucleotide bond, and the modified internucleotide bond is absent in a range from a third base 5' to the position of the mutation for editing to a third base 3' to the position of the mutation for editing.
[11] The composition according to [10], wherein the sugar-modified nucleotide is a nucleotide containing a 2'-O,4'-C-methylene cross-linkage or a 2'-O,4'-C-ethylene cross-linkage in the ribose ring.
[12] The composition according to [10] or [11], wherein the first single-stranded polynucleotide contains one to four modified internucleotide bonds within 13 bases from the 5' end and/or the 3' end.
   Here, preferably, the modified internucleotide bond or modified internucleotide bonds is/are each a phosphorothioate bond, an alkylphosphonate bond, or a phosphotriester bond. More preferably, the first single-stranded polynucleotide contains one to four modified internucleotide bonds within five nucleotides (more preferably, two modified internucleotide bonds within three nucleotides or one modified internucleotide bond within two nucleotides) from the 3' end, and the modified internucleotide bonds are each a phosphorothioate bond.
[13] The composition according to any of [1] to [12], further containing a second single-stranded polynucleotide, wherein
   the second single-stranded polynucleotide is 20 to 200 nucleotides in length, contains a base sequence complementary to a partial base sequence of a region not containing the mutation for editing in the first single-stranded polynucleotide, and is capable of forming a double strand with the first single-stranded polynucleotide.
[14] The composition according to [13], wherein the second single-stranded polynucleotide contains a base sequence complementary to a part within a region 3' to the position of the mutation for editing in the base sequence of the first single-stranded polynucleotide.
[15] The composition according to [13] or [14], wherein the molar concentration of the third single-stranded polynucleotide is equal to or higher than the molar concentration of the second single-stranded polynucleotide.
[16] The composition according to any of [13] to [15], wherein the second single-stranded polynucleotide contains at least one modified internucleotide bond.
[17] The composition according to [16], wherein the second single-stranded polynucleotide contains one or two modified internucleotide bonds within three bases from the 5' end and/or the 3' end or one modified internucleotide bond within two bases from the 5' end and/or the 3' end.
   Preferably, the second single-stranded polynucleotide contains one or two modified internucleotide bonds within three nucleotides from the 3' end or one modified internucleotide bond within two nucleotides from the 3' end.
[18] The composition according to any of [10] to [17], wherein the modified internucleotide bond or modified internucleotide bonds is/are each a phosphorothioate bond, an alkylphosphonate bond, or a phosphotriester bond.
[19] The composition according to any of [1] to [19], wherein an abnormal base sequence causative of a disease is contained in the target region, and the base sequence of the first single-stranded polynucleotide contains the mutation for editing so that the abnormal base sequence is repaired and thereby the disease is treated or prevented.
[20] A pharmaceutical composition for treating or preventing a disease due to an abnormal base sequence contained in a target region present in an intracellular double-stranded DNA, the pharmaceutical composition containing the composition according to [19].
[21] A method for treating or preventing a disease due to an abnormal base sequence contained in a target region present in an intracellular double-stranded DNA, the method including administering a therapeutically or prophylactically effective amount of the composition according to [19] to a subject.
[22] A method for producing an isolated cell having a mutation introduced thereinto or an organism possessing a cell having a mutation introduced thereinto, the method including:
   introducing the composition according to any of [1] to [19] into an isolated cell or a cell constituting a living body of an organism, thereby introducing a mutation into a base sequence in a target region present in an intracellular double-stranded DNA.
   Here, the method may be performed *in vitro*.
[23] Use of the composition according to any of [1] to [19] in the manufacture of a medicament for treating or preventing a disease due to an abnormal base sequence contained in a target region present in an intracellular double-stranded DNA.

The present invention also includes the following.
[a1] A composition containing a first single-stranded polynucleotide, wherein
   the first single-stranded polynucleotide is 50 to 200 nucleotides in length, and has a base sequence having a sequence identity of 80% or more with a partial base sequence of an editing target strand in a target region present in an intracellular double-stranded DNA and containing at least one mutation for editing selected from the group consisting of deletion, substitution, and insertion of one or more nucleotides as compared with a base sequence of the editing target strand, and the composition has at least one of the following features:
   (a) at least one nucleotide in a region consisting of the position of the mutation for editing and a 10-base sequence 5' adjacent to the position of the mutation for editing and a 10-base sequence 3' adjacent to the position of the mutation for editing in the first single-stranded polynucleotide is a sugar-modified nucleotide; and
   (b) the first single-stranded polynucleotide contains at least one modified internucleotide bond, and the modified internucleotide bond is absent in a range from a third base 5' to the position of the mutation for editing to a third base 3' to the position of the mutation for editing.
   Preferably, the composition contains neither an exogenous nuclease nor a polynucleotide capable of expressing it, and is not used in combination with any of them.
[a2] The composition according to [a1], wherein the sugar-modified nucleotide is a nucleotide containing a 2'-O,4'-C-methylene cross-linkage or a 2'-O,4'-C-ethylene cross-linkage in the ribose ring.
[a3] The composition according to [a1] or [a2], wherein the first single-stranded polynucleotide contains one to four modified internucleotide bonds within 13 bases from the 5' end and/or the 3' end.
   Here, preferably, the modified internucleotide bond or modified internucleotide bonds is/are each a phosphorothioate bond, an alkylphosphonate bond, or a phosphotriester bond. More preferably, the first single-stranded polynucleotide contains one to four modified internucleotide bonds within five bases (more preferably, two modified internucleotide bonds within three bases or one modified internucleotide bond within two bases) from the 3' end, and the modified internucleotide bonds are each a phosphorothioate bond.
[a4] The composition according to any of [a1] to [a3], wherein the position of the mutation for editing in the first single-stranded polynucleotide is present within a range of 25 to 75% from the 5' end, with the full length from the 5' end to the 3' end defined as 100%.
[a5] The composition according to [a4], wherein the position of the mutation for editing in the first single-stranded polynucleotide is present within a range of 49 to 51% from the 5' end, with the full length from the 5' end to the 3' end defined as 100%.
[a6] The composition according to any of [a1] to [a5], wherein the first single-stranded polynucleotide is 70 to 100 nucleotides in length.
[a7] The composition according to any of [a1] to [a6], further containing a second single-stranded polynucleotide, wherein
   the second single-stranded polynucleotide is 20 to 200 nucleotides in length, contains a base sequence complementary to a partial base sequence of a region not containing the mutation for editing in the first single-stranded polynucleotide, and is capable of forming a double strand with the first single-stranded polynucleotide.
[a8] The composition according to [a7], wherein the second single-stranded polynucleotide contains at least one modified internucleotide bond.
[a9] The composition according to [a8], wherein the second single-stranded polynucleotide contains one or two modified internucleotide bonds within three bases from the 5' end and/or the 3' end or one modified internucleotide bond within two bases from the 5' end and/or the 3' end.
   Preferably, the second single-stranded polynucleotide contains one or two modified internucleotide bonds within three bases from the 3' end or one modified internucleotide bond within two bases from the 3' end.
[a10] The composition according to any of [a7] to [a9], wherein the second single-stranded polynucleotide contains a base sequence complementary to a part within a region 3' to the position of the mutation for editing in the base sequence of the first single-stranded polynucleotide.
[a11] The composition according to [a1], [a3], [a8], or [a9], wherein the modified internucleotide bond or modified internucleotide bonds is/are each a phosphorothioate bond, an alkylphosphonate bond, or a phosphotriester bond.
[a12] The composition according to any of [a1] to [a11], further containing a third single-stranded polynucleotide, wherein
   the third single-stranded polynucleotide is 15 to 200 nucleotides in length, and has a base sequence having a sequence identity of 90% or more with a base sequence in the editing target strand 5' or 3' to a position corresponding to the mutation for editing in the first single-stranded polynucleotide, wherein, in the editing target strand, the base sequence having sequence identity with the first single-stranded polynucleotide and the base sequence having sequence identity with the third single-stranded polynucleotide are overlapping, adjacent, or separated by one to nine bases.
[a13] The composition according to [a12], wherein the base sequence of the third single-stranded polynucleotide has a sequence identity of 90% or more with a base sequence in the editing target strand 5' to the position corresponding to the mutation for editing in the first single-stranded polynucleotide.
[a14] The composition according to [a12] or [a13], wherein, in the editing target strand, the base sequence having sequence identity with the first single-stranded polynucleotide and the base sequence having sequence identity with the third single-stranded polynucleotide are adjacent.
[a15] The composition according to any of [a12] to [a14], wherein the third single-stranded polynucleotide is 55 to 75 nucleotides in length.
[a16] The composition according to any of [a12] to [a15], wherein the third single-stranded polynucleotide consists of a base sequence 100% identical to a corresponding base sequence in the editing target strand.
[a17] The composition according to any of [a12] to [a16], wherein the molar concentration of the third single-stranded polynucleotide is equal to or higher than the molar concentration of the second single-stranded polynucleotide.
[a18] The composition according to any of [a1] to [a17], wherein an abnormal base sequence causative of a disease is contained in the target region, and the base sequence of the first single-stranded polynucleotide contains the mutation for editing so that the abnormal base sequence is repaired and thereby the disease is treated or prevented.
[a19] A pharmaceutical composition for treating or preventing a disease due to an abnormal base sequence contained in a target region present in an intracellular double-stranded DNA, the pharmaceutical composition containing the composition according to [a18].
[a20] A method for treating or preventing a disease due to an abnormal base sequence contained in a target region present in an intracellular double-stranded DNA, the method including administering a therapeutically or prophylactically effective amount of the composition according to [a18] to a subject.
[a21] A method for producing an isolated cell having a mutation introduced thereinto or an organism possessing a cell having a mutation introduced thereinto, the method including:
   introducing the composition according to any of [a1] to [a18] into an isolated cell or a cell constituting a living body of an organism, thereby introducing a mutation into a base sequence in a target region present in an intracellular double-stranded DNA.
   Here, the method may be performed *in vitro*.
[a22] Use of the composition according to any of [a1] to [a18] in the manufacture of a medicament for treating or preventing a disease due to an abnormal base sequence contained in a target region present in an intracellular double-stranded DNA.

The present invention also includes the following.
[b1] A pharmaceutical composition containing a third single-stranded polynucleotide for use in combination with a composition containing a first single-stranded polynucleotide, wherein
   the first single-stranded polynucleotide is 50 to 200 nucleotides in length, and has a base sequence having a sequence identity of 80% or more with a partial base sequence of an editing target strand in a target region present in an intracellular double-stranded DNA and containing at least one mutation for editing selected from the group consisting of deletion, substitution, and insertion of one or more nucleotides as compared with a base sequence of the editing target strand,
   the third single-stranded polynucleotide is 15 to 200 nucleotides in length, and has a base sequence having a sequence identity of 90% or more with a base sequence in the editing target strand 5' or 3' to a position corresponding to the mutation for editing in the first single-stranded polynucleotide, wherein, in the editing target strand, the base sequence having sequence identity with the first single-stranded polynucleotide and the base sequence having sequence identity with the third single-stranded polynucleotide are overlapping, adjacent, or separated by one to nine bases, and
   an abnormal base sequence causative of a disease is contained in the target region, and the base sequence of the first single-stranded polynucleotide contains the mutation for editing so that the abnormal base sequence is repaired and thereby the disease is treated or prevented.
   Preferably, the composition contains neither an exogenous nuclease nor a polynucleotide capable of expressing it, and is not used in combination with any of them.
[b2] The pharmaceutical composition according to [b1], wherein the base sequence of the third single-stranded polynucleotide has a sequence identity of 90% or more with a base sequence in the editing target strand 5' to the position corresponding to the mutation for editing in the first single-stranded polynucleotide.
[b3] The pharmaceutical composition according to [b1] or [b2], wherein, in the editing target strand, the base sequence having sequence identity with the first single-stranded polynucleotide and the base sequence having sequence identity with the third single-stranded polynucleotide are adjacent.
[b4] The pharmaceutical composition according to any of [b1] to [b3], wherein the third single-stranded polynucleotide is 55 to 75 nucleotides in length.
[b5] The composition according to any of [b1] to [b4], wherein the third single-stranded polynucleotide consists of a base sequence 100% identical to a corresponding base sequence in the editing target strand.
[b6] The pharmaceutical composition according to any of [b1] to [b5], wherein the position of the mutation for editing in the first single-stranded polynucleotide is present within a range of 25 to 75% from the 5' end, with the full length from the 5' end to the 3' end defined as 100%.
[b7] The pharmaceutical composition according to [b6], wherein the position of the mutation for editing in the first single-stranded polynucleotide is present within a range of 49 to 51% from the 5' end, with the full length from the 5' end to the 3' end defined as 100%.
[b8] The pharmaceutical composition according to any of [b1] to [b7], wherein the first single-stranded polynucleotide is 70 to 100 nucleotides in length.
[b9] The composition according to any of [b1] to [b8], wherein, in the first single-stranded polynucleotide, a sequence excluding the position of the mutation for editing and a region of 10 bases from the 5' end and a region of 10 bases from the 3' end from the first single-stranded polynucleotide consists of a base sequence 100% identical to a corresponding base sequence in the editing target strand.
[b10] The pharmaceutical composition according to any of [b1] to [b9], wherein
   the composition containing the first single-stranded polynucleotide further contains a second single-stranded polynucleotide, and
   the second single-stranded polynucleotide is 20 to 200 nucleotides in length, contains a base sequence complementary to a partial base sequence of a region not containing the mutation for editing in the first single-stranded polynucleotide, and is capable of forming a double strand with the first single-stranded polynucleotide.
[b11] The pharmaceutical composition according to [b10], wherein the second single-stranded polynucleotide contains a base sequence complementary to a part within a region 3' to the position of the mutation for editing in the base sequence of the first single-stranded polynucleotide.
[b12] The pharmaceutical composition according to [b10] or [b11], wherein the pharmaceutical composition is used in such a manner that the molar concentration of the third single-stranded polynucleotide is equal to or higher than the molar concentration of the second single-stranded polynucleotide.
[b13] The pharmaceutical composition according to any of [b1] to [b12] for treating or preventing a disease due to an abnormal base sequence contained in a target region present in an intracellular double-stranded DNA.
[b14] A method for treating or preventing a disease due to an abnormal base sequence contained in a target region present in an intracellular double-stranded DNA, the method including administering a therapeutically or prophylactically effective amount of the pharmaceutical composition according to [b13] to a subject together with the composition containing the first single-stranded polynucleotide simultaneously, separately, or consecutively.
[b15] A method for producing an isolated cell having a mutation introduced thereinto or an organism possessing a cell having a mutation introduced thereinto, the method including:
   introducing the pharmaceutical composition according to any of [b1] to [b13] into an isolated cell or a cell constituting a living body of an organism together with the composition containing the first single-stranded polynucleotide simultaneously, separately, or consecutively, thereby introducing a mutation into a base sequence in a target region present in an intracellular double-stranded DNA.
   Here, the method may be performed *in vitro*.
[b16] Use of the pharmaceutical composition according to any of [b1] to [b12] in the manufacture of a medicament for treating or preventing a disease due to an abnormal base sequence contained in a target region present in an intracellular double-stranded DNA.

### ADVANTAGEOUS EFFECTS OF INVENTION

The present invention enables desired base editing for intracellular DNAs in editing methods with ss DNAs or TDs.

### BRIEF DESCRIPTION OF DRAWINGS

[Fig. 1] Fig. 1 is a schematic diagram of polynucleotides to be used in a base sequence editing method with single-stranded DNAs (ss DNAs) or 5'-tailed duplexes (TDs) as a conventional technique, and first to third single-stranded polynucleotides in a specific embodiment of the present invention.
[Fig. 2] Fig. 2 shows experimental results in Example 1, in which examination was performed on change in editing efficiency caused by adding an A' strand with use of copGFP gene as a target gene in the base sequence editing method with TDs. An enlarged view of a fluorescence microscope image with addition of the A' strand is shown in an area indicated by an arrow symbol.
[Fig. 3] Fig. 3 is a graph representing the experimental results in Example 1 in editing efficiency (%). Bars indicate SEM (n = 3).
[Fig. 4] Fig. 4 shows experimental results in Example 2, in which examination was performed on change in editing efficiency caused by adding an A' strand with use of mEGFP gene as a target gene in the base sequence editing method with TDs. An enlarged view of a fluorescence microscope image with addition of the A' strand is shown in an area indicated by an arrow symbol.
[Fig. 5] Fig. 5 is a graph representing the experimental results in Example 2 in editing efficiency (%). Bars indicate SEM (n = 3).
[Fig. 6] Fig. 6 is a graph showing results in Example 3 (3-1). Shown in the parentheses for each sample name in the abscissa are the modified strand and the positions and number of PS modifications. Bars indicate SEM (n = 3).
[Fig. 7] Fig. 7 is a graph showing results in Example 3 (3-2). Shown in the parentheses for each sample name in the abscissa are the modified strand and the positions and number of PS modifications. Bars indicate SEM (n = 3).
[Fig. 8] Fig. 8 is a graph showing results in Example 3 (3-3). Shown in the parentheses for each sample name in the abscissa are the positions of PS modifications in an E strand. Bars indicate SEM. **: P < 0.01; ***: P < 0.001 (n = 3); n-TD' vs others.
[Fig. 9] Fig. 9 is a graph showing results in Example 3 (3-4). Shown in the parentheses for each sample name in the abscissa are the modified strand and the positions of P-Me modifications or P-OEt modifications. n = 3.
[Fig. 10] Fig. 10 is a graph showing results in Example 3 (3-5). Shown in the parentheses for each sample name in the abscissa are the positions and number of P-OEt modifications or PS modifications in an A strand. Bars indicate SEM. *: P < 0.05 (n = 3).
[Fig. 11] Fig. 11 is a graph showing results in Example 3 (3-6). Shown in the parentheses for each sample name in the abscissa are the positions of LNA modifications in an E strand. Bars indicate SEM. *: P < 0.05; *: *P < 0.01; ***: P < 0.001 (n = 3); n-TD' vs others.
[Fig. 12] Fig. 12 is a graph showing results in Example 3 (3-7). Shown in the parentheses for each sample name in the abscissa are the positions of LNA modifications in an E strand. The six samples on the right side were provided with one P-OEt modification in an A strand. Bars indicate SEM. *: P < 0.05; **: P < 0.01 (n = 3).
[Fig. 13] Fig. 13 is a graph showing results in Example 4.
[Fig. 14] Fig. 14 is a graph showing results in Example 5. The abscissa shows the molar ratios among an E strand, an A strand, and an A' strand (E strand:A strand:A' strand) in samples.
[Fig. 15] Fig. 15A is a diagram illustrating the arrangement of constituents of nucleic acids for editing that were used in Example 6 relative to a target region. Fig. 15B is a graph showing results in Example 6. Shown in the parentheses for each sample name in the abscissa is the number of gaps. Bars indicate SEM. *: P < 0.05; ***: P < 0.001 (n = 3).
[Fig. 16] Fig. 16A is a diagram illustrating the arrangement of constituents of nucleic acids for editing that were used in Example 7 relative to a target region. Fig. 16B is a graph showing results in Example 7. Shown in the parentheses for each sample name in the abscissa is the number of bases overlapping between an E strand and an A' strand. Bars indicate SEM. *: P < 0.05; **: P < 0.01 (n = 3).

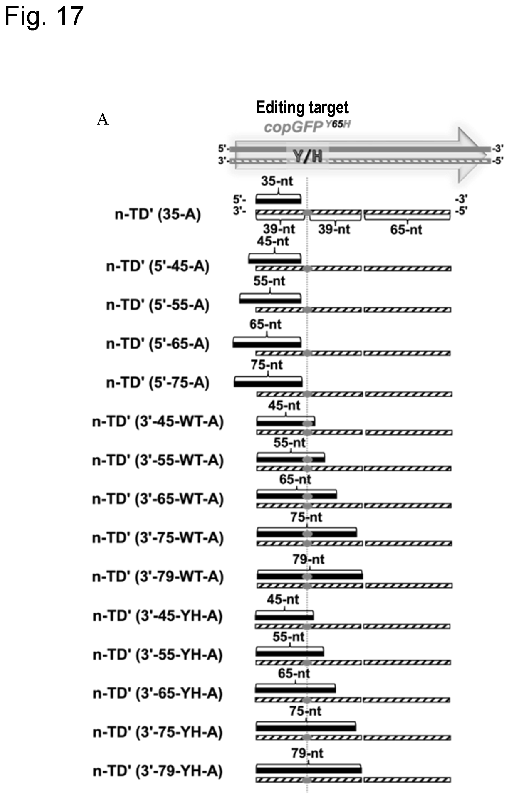

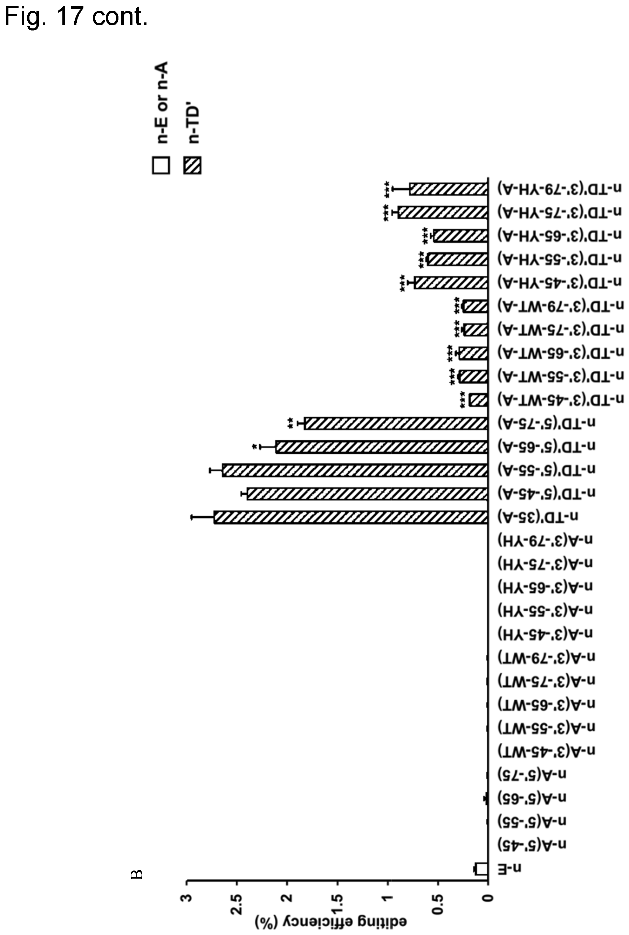

[Fig. 17] Fig. 17A is a diagram illustrating the arrangement of constituents of nucleic acids for editing that were used in Example 8 relative to a target region. Fig. 17B is a graph showing results in Example 8. Shown in the parentheses for each sample name in the abscissa are the direction of elongation of the length of an A strand (in the 5' or 3' direction), the length of the A strand, and, if the A strand contained a base sequence complementary to a part containing a mutation in an E strand, whether the base sequence was wild-type (WT) or mutated (Y/H). Bars indicate SEM. *: P < 0.05; **: P < 0.01; ***: P < 0.001 (n = 3); n-TD' (35A) vs others.

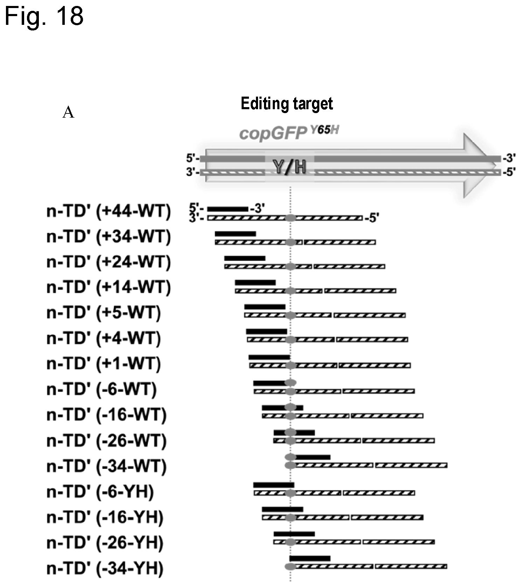

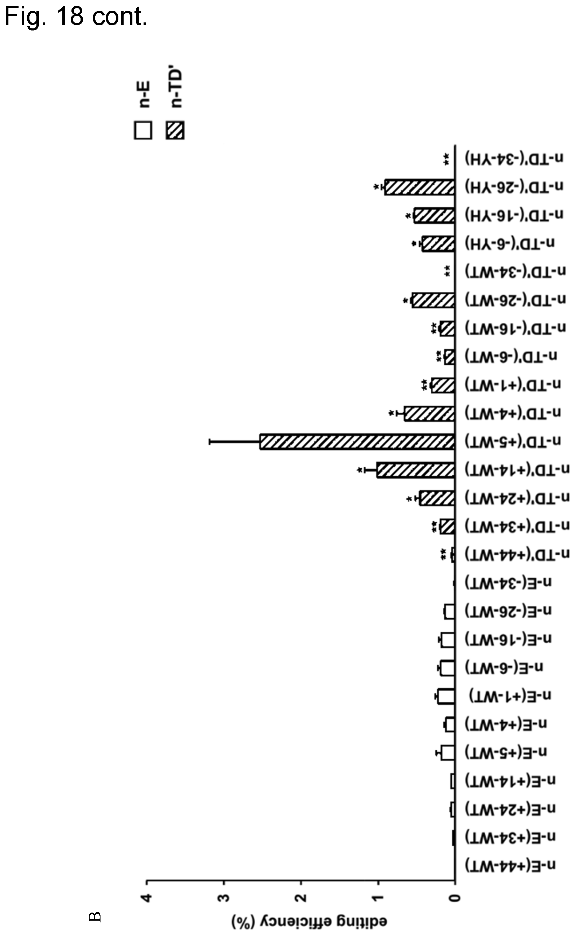

[Fig. 18] Fig. 18A is a diagram illustrating the arrangement of constituents of nucleic acids for editing that were used in Example 9 relative to a target region. Fig. 18B is a graph showing results in Example 9. Shown in the parentheses for each sample name in the abscissa are the position of a mutation, with the position of a base in an E strand complementary to the base at the 3' end of an A strand defined as position -1, and, if the A strand contained a base sequence complementary to a part containing a mutation in the E strand, whether the base sequence was wild-type (WT) or mutated (Y/H). Bars indicate SEM. *: P < 0.05; **: P < 0.01 (n = 3); n-TD' (A 40-WT) vs others.
[Fig. 19] Fig. 19A is a diagram illustrating the arrangement of constituents of nucleic acids for editing that were used in Example 10 relative to a target region. Fig. 19B is a graph showing results in Example 10. Shown in the parentheses for each sample name in the abscissa is the molar ratio among a pDNA, an E strand, an A strand, and an A' strand (E strand:A strand:A' strand) in the sample. Bars indicate SEM. *: P < 0.05; ***: P < 0.001.
[Fig. 20] Fig. 20 shows results in Example 11. Shown are results of genome editing by using a TD with addition of an A' strand.
[Fig. 21] Fig. 21 is a graph showing results in Example 12. Bars indicate SEM. *P < 0.05 (n = 3).
[Fig. 22] Fig. 22 is a graph showing results in Example 13. Bars indicate SEM. *P < 0.05, **P < 0.01 (n = 3).
[Fig. 23] Fig. 23 is a graph showing results in Example 14.
[Fig. 24] Fig. 24 is a graph showing results in Example 15.
[Fig. 25] Fig. 25 is a diagram of a plasmid used in Example 16.
[Fig. 26] Fig. 26 is a graph showing results in Example 16. Shown are relative editing efficiencies as the efficiency of editing with AS n-TD' for pcDNA4_mPlum_T2A_copGFP Y/H having fluorescence-deficient copGFP incorporated therein is taken as 1. Bars indicate SEM. *P < 0.05, **P < 0.01, ***P < 0.001 (n = 3).
[Fig. 27] Fig. 27A is a diagram illustrating the arrangement of constituents of nucleic acids for editing that were used in Example 17-1 relative to a target region. Shown are an E strand having a length of 79 nucleotides and the arrangement and names of ODNs each having a length of 35 nucleotides. Fig. 27B is a graph representing experimental results in Example 17-1 in editing efficiency (%). Bars indicate SEM. *: P < 0.05; **: P < 0.01 (n = 3); n-E strand only vs others.
[Fig. 28] Fig. 28 is a graph representing experimental results in Example 17-2 in editing efficiency (%). Numerical values in the abscissa indicate doses in molar ratios among an E strand, an A strand, and an A' strand having a length of 35 nucleotides as the amount of introduction of a pDNA for editing efficiency analysis is taken as 1.
[Fig. 29] Fig. 29 is a graph representing experimental results in Example 17-3 in editing efficiency (%). Numerical values in the abscissa indicate doses in molar ratios among an E strand, an A strand, and an A' strand having a length of 35 nucleotides as the amount of introduction of a pDNA for editing efficiency analysis is taken as 1.
[Fig. 30] Fig. 30 is a graph representing experimental results in Example 17-4 in editing efficiency (%). Numerical values in the abscissa indicate doses in molar ratios among an E strand, an A strand, and an A' strand having a length of 35 nucleotides as the amount of introduction of a pDNA for editing efficiency analysis is taken as 1.
[Fig. 31] Fig. 31A is a diagram illustrating the arrangement of constituents of nucleic acids for editing that were used in Example 17-5 relative to a target region. Shown are an E strand having a length of 79 nucleotides, an A strand, and the arrangement and names of A' strands of different lengths. Fig. 31B is a graph representing experimental results in Example 17-5 in editing efficiency (%).
[Fig. 32] Fig. 32 is a graph representing experimental results in Example 17-6 in editing efficiency (%).

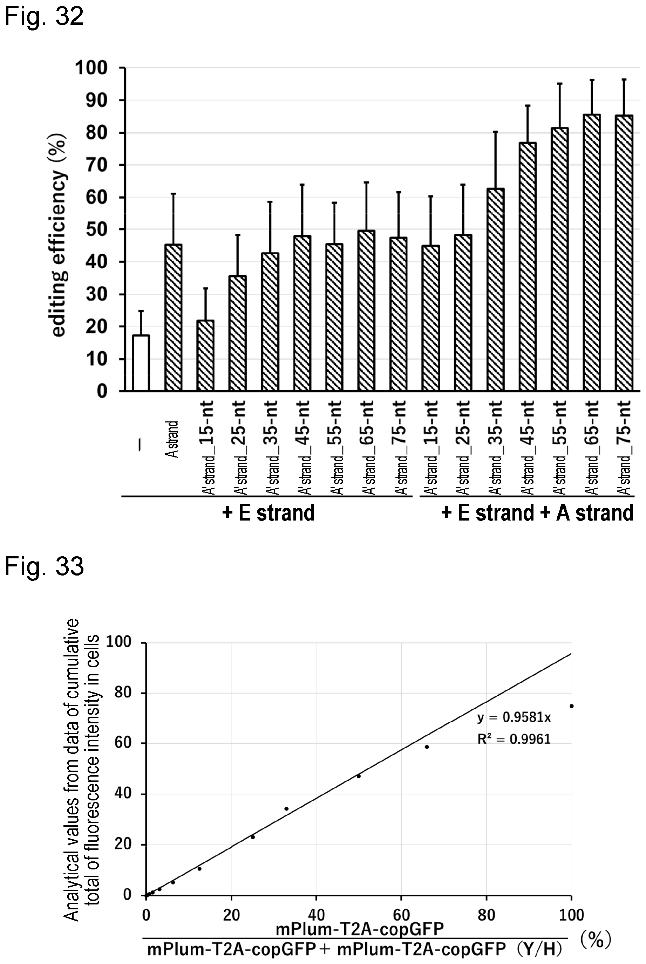

[Fig. 33] Fig. 33 is a graph for Example 17-7, in which analytical values from fluorescence intensity data are plotted on the ordinate, and the proportions (%) of an mPlum-T2A-copGFP (normal) expression vector plasmid in introduced plasmids (mPlum-T2A-copGFP (normal) expression vector plasmid and mPlum-T2A-copGFP (Y/H) expression vector plasmid) are plotted on the abscissa. The line is a regression line determined from analytical values for proportions of the copGFP (normal) expression vector up to 50%. y indicates the slope of the line, and R² indicates the coefficient of determination for the linear relationship.
[Fig. 34] Fig. 34 is a graph representing experimental results in Example 17-8 (editing efficiencies in Example 17-6 were determined by the method in Example 17-7) in editing efficiency (%).
[Fig. 35] Fig. 35 is a graph showing results in Example 18-1. Shown in the parentheses for each sample name in the abscissa are the positions of LNA modifications in an E strand.
[Fig. 36] Fig. 36 is a graph showing results in Example 18-2. Shown in the parentheses for each sample name in the abscissa are the positions of LNA modifications in the E strand.
[Fig. 37] Fig. 37 is a graph showing results in Example 18-3. Shown in the parentheses for each sample name in the abscissa are the positions of LNA modifications in an E strand.
[Fig. 38] Fig. 38 is a graph showing results in Example 18-4. Shown in the parentheses for each sample name in the abscissa are the positions of LNA modifications in an E strand. Bars indicate SEM. *: P < 0.05; **: P < 0.01 (n = 3); n-TD' vs others.
[Fig. 39] Fig. 39 is a graph showing results in Example 18-5. Shown in the parentheses for each sample name in the abscissa are the positions of LNA modifications in an E strand. Bars indicate SEM. *: P < 0.05; **: P < 0.01; ***: P < 0.05 (n = 3); n-TD' vs others. Shown are the positions of LNA modifications in an E strand.
[Fig. 40] Fig. 40 is a graph showing results in Example 18-6. Shown in the parentheses for each sample name in the abscissa are the positions of LNA modifications in an E strand.
[Fig. 41] Fig. 41 is a graph showing results in Example 18-7. Shown in the parentheses for each sample name in the abscissa are the positions of LNA modifications in an E strand.
[Fig. 42] Fig. 42A is a diagram illustrating the arrangement of constituents of nucleic acids for editing that were used in Example 18-8 relative to a target region. Figs. 42B to 42E are graphs showing results in Example 18-8. Shown in the parentheses for each sample name in the abscissa are/is the length of an A strand and/or the positions of LNA modifications in an E strand.
[Fig. 43] Fig. 43 is a graph showing results in Example 18-9. Shown in the parentheses for each sample name in the abscissa are the positions of LNA modifications in an E strand.
[Fig. 44] Fig. 44 is a graph showing results in Example 18-10. Shown in the parentheses for each sample name in the abscissa is the length of an A strand.
[Fig. 45] Fig. 45 is a series of graphs showing results in Example 18-11. Fig. 45A is a graph showing editing efficiency for copGFP gene. Fig. 45B is a graph showing editing efficiency for DMD gene. Fig. 45C is a graph showing editing efficiency for WRN gene.
[Fig. 46] Fig. 46 is a diagram illustrating the structure of pNGS2-K3.
[Fig. 47] Fig. 47 is a graph showing results in Example 19. n.s: no significant difference.
[Fig. 48] Fig. 48 is a graph showing results in Example 20.

### DESCRIPTION OF EMBODIMENTS

The following describes the present invention in detail. Embodiments described below are representative examples of those of the present invention, and the present invention is never interpreted narrowly based on those embodiments.

### 1. Definitions

Herein, the term "intracellular double-stranded DNA" means any double-stranded DNA existing in a cell that is present in a living body and constituting the living body or an isolated cell (e.g., a cultured cell). Double-stranded DNAs can contain a region encoding a protein or RNA product and a region to regulate expression thereof. An intracellular double-stranded DNA can be an endogenous double-stranded DNA (genomic DNA or mitochondrial DNA) or an exogenous double-stranded DNA (a foreign DNA, a viral genome, or the like introduced into a cell).

Herein, the term "target region" means a region of an intracellular double-stranded DNA, the region containing a base sequence into which a mutation is to be introduced. Such a target region may be any region of an intracellular double-stranded DNA, and can be, for example, a region containing part or the whole of a structural gene region or a regulatory gene region. A structural gene region is a region specifying the amino acid sequence of a protein, and the structural gene region of a eukaryotic cell can contain both exons and introns. Examples of regulatory gene regions include operator regions, promoter regions, and attenuator regions. In relation to genes encoded by a double-stranded DNA, one strand constituting a target region is occasionally referred to as a sense strand, and the other strand as an antisense strand.

Herein, the term "base sequence editing" refers to introducing an intended mutation into a base sequence. Herein, the term "mutation" means deletion, substitution, or insertion of one or more base(s) in a reference base sequence, or a combination of two or more of such deletion, substitution, and insertion. Such a reference base sequence is normally a natural or normal base sequence, but is not limited thereto.

Herein, the term "editing efficiency" refers to the proportion (percentage) of successfully edited cells in cells having a nucleic acid for editing introduced thereinto in base sequence editing for an intracellular double-stranded DNA, unless otherwise specified. In calculating editing efficiency in *in vitro* experiment as in Examples, a calculation method described in Example 1 with considering the copy number of a target region in cells and a calculation method described in Example 17-1 without considering the copy number of a target region in cells are both available.

Herein, the term "exogenous nuclease" means a nuclease that is not endogenously possessed by a biological species to be subjected to the base sequence editing of the present invention. Exogenous nucleases include various nucleases from different biological species to be subjected to the base sequence editing of the present invention, such as nucleases derived from noneukaryotic biological species, nucleases derived from nonmammalian biological species, and nucleases derived from biological species that are not any of humans, primates, mice, rats, dogs, cats, rabbits, horses, and bovines. Exogenous nucleases also include completely artificial nucleases such as zinc-finger nuclease (ZFN) and TALEN, and nucleases derived from bacteria such as Cas nuclease.

Herein, the term "Tailed Duplex" or "TD" refers to a double-stranded polynucleotide having a single-stranded moiety at the 5' end or the 3' end. TDs can be appropriately produced by a method known to those skilled in the art, for example, by mixing one single-stranded polynucleotide and another single-stranded polynucleotide having a base sequence complementary to a partial base sequence of the one single-stranded polynucleotide, or by hybridizing the two.

Herein, the term "single-stranded polynucleotide" refers to a molecule in which nucleotide monomers have been covalently bonded to form a chain, and DNAs, RNAs, and DNA/RNA chimeras are all acceptable as a single-stranded polynucleotide. Single-stranded polynucleotides may contain a modified nucleotide (a nucleotide having a modified base and/or a sugar-modified nucleotide) or a modified internucleotide bond.

Herein, the term "modified base" refers to a nucleotide base modified by substitution or addition of one or more atoms or groups. Modified bases structurally differ from natural or synthesized unmodified bases, but are functionally exchangeable with them. Modified bases include all modes of modification known in the art to which the present invention belongs. Examples of such modification include alkylation, halogenation, thiolation, amination, amidation, or acetylation, or any of various combinations of them. Examples of modified bases include 5-methylcytosine.

Herein, the term "sugar-modified nucleotide" refers to a nucleotide whose sugar moiety has been modified. Sugar-modified nucleotides include all modes of sugar modification known in the art to which the present invention belongs. Sugar-modified nucleotides, for example, for nucleotides having a ribose ring include 2'-modified nucleotides, 4'-thio-modified nucleotides, 4'-thio-2'-modified nucleotides, and bicyclic sugar-modified nucleotides.

Examples of 2'-modified nucleotides include halo, allyl, amino, azido, O-allyl, O-C1-C10 alkyl, OCF₃, O-(CH₂)₂-O-CH₃, 2'-O(CH₂)₂SCH₃, O-(CH₂)₂-O-N(Rm)(Rn), or O-CH₂-C(=O)-N(Rm)(Rn), wherein Rm and Rn are each individually an amino-protecting group or a substituted or unsubstituted C1-C10 alkyl. For 2'-O-methylguanosine, 2'-O-methyladenosine, 2'-O-methylcytidine, and 2'-O-methyluridine, commercially available amidite reagents can be used. 2'-O-Aminoethylguanosine, 2'-O-aminoethyladenosine, 2'-O-aminoethylcytidine, and 2'-O-aminoethyluridine can be synthesized with an amidite reagent described in a document (Blommers et al. Biochemistry (1998), 37, 17714-17725.). 2'-O-Propylguanosine, 2'-O-propyladenosine, 2'-O-propylcytidine, and 2'-O-propyluridine can be synthesized according to a document (Lesnik, E. A. et al. Biochemistry (1993), 32, 7832-7838.). For 2'-O-allylguanosine, 2'-O-allyladenosine, 2'-O-allylcytidine, and 2'-O-allyluridine, commercially available amidite reagents can be used. 2'-O-Methoxyethylguanosine, 2'-O-methoxyethyladenosine, 2'-O-methoxyethylcytidine, and 2'-O-methoxyethyluridine can be synthesized according to a patent (US6261840) or a document (Martin, P. Helv. Chim. Acta. (1995) 78, 486-504). 2'-O-Butylguanosine, 2'-O-butyladenosine, 2'-O-butylcytidine, and 2'-O-butyluridine can be synthesized with an amidite reagent described in a document (Lesnik, E. A. et al. Biochemistry (1993), 32, 7832-7838.). 2'-O-Pentylguanosine, 2'-O-pentyladenosine, 2'-O-pentylcytidine, and 2'-O-pentyluridine can be synthesized according to a document (Lesnik, E. A. et al. Biochemistry (1993), 32, 7832-7838.). For 2'-O-Propargylguanosine, 2'-O-propargyladenosine, 2'-O-propargylcytidine, and 2'-O-propargyluridine, commercially available amidite reagents can be used.

Examples of 4'-thio-modified nucleotides can include β-D-ribonucleotides whose 4'-oxygen atom has been replaced with a sulfur atom (Hoshika, S. et al. FEBS Lett. 579, p. 3115-3118, (2005); Dande, P. et al. J. Med. Chem. 49, p. 1624-1634 (2006); Hoshika, S. et al. ChemBioChem. 8, p. 2133-2138, (2007)).

Examples of 4'-thio-2'-modified nucleotides can include 4'-thio-2'-modified nucleotides possessing 2'-H or 2'-O-methyl (Matsugami, et al. Nucleic Acids Res. 36, 1805 (2008)).

Examples of bicyclic sugar-modified nucleotides can include nucleotides possessing a second ring formed by crosslinking two atoms of the ribose ring. Examples of bicyclic sugar modification can include 2'-O,4'-C-crosslinking modification such as 2',4'-BNA/LNA (bridged nucleic acids/locked nucleic acids) formed by crosslinking the 2'-oxygen atom and the 4'-carbon atom via a methylene chain (Obika, S. et al. Tetrahedron Lett., 38, p. 8735-(1997).; Obika, S. et al., Tetrahedron Lett., 39, p. 5401- (1998).; A. A. Koshkin, A.A. et al. Tetrahedron, 54, p. 3607 (1998).; Obika, S. Bioorg. Med. Chem., 9, p. 1001 (2001).), and ENA (2'-O,4'-C-ethylene-bridged nucleic acids) obtained by replacing the methylene chain for crosslinking in 2',4'-BNA/LNA with an ethylene chain, which is longer than the methylene chain by one carbon atom (Morita, K. et al. Bioorg. Med. Chem. Lett., 12, p. 73 (2002).; Morita, K. et al. Bioorg. Med. Chem., 11, p. 2211 (2003).). Other examples can include AmNA described in WO 2014/109384 or S-cEt (2',4'-constrained ethyl) described in a document (Seth, P. P. et al. J. Org. Chem (2010), 75, 1569-1581.). For modification of the ribose ring, the terms "2'-O,4'-C-methylene cross-linkage" and "4'-CH₂-O-2' cross-linkage" are synonymous.

Herein, the term "modified internucleotide bond" refers to a bond obtained by substituting or changing a bond via a phosphate moiety (i.e., a phosphodiester bond) between two naturally occurring nucleosides. That is, a polynucleotide containing a modified internucleotide bond has modification of a phosphate group of at least one nucleotide. Modified internucleotide bonds include all modes of modification known in the art to which the present invention belongs. Examples of modified internucleotide bonds include phosphorothioate bonds, phosphorodithioate bonds, alkylphosphonate bonds, boranophosphate bonds, phosphoroamidate bonds, and phosphotriester bonds. Alkylphosphonate bonds include a bond obtained through modification by replacing a non-crosslinking oxygen atom of a phosphodiester bond with a methyl group (P-CH₃; methylphosphonate bond). Phosphotriester bonds include a bond obtained through modification by replacing a non-bridging oxygen atom of a phosphodiester bond with an ethoxy group (P-OC₂H₅; ethylphosphotriester bond).

### 2. Base sequence editing with E strand and A' strand or E strand + A strand (Tailed Duplex (TD)) and A' strand

In an aspect, the present invention provides a composition containing a first single-stranded polynucleotide and a third single-stranded polynucleotide, wherein
the first single-stranded polynucleotide is 50 to 200 nucleotides in length, and has a base sequence having a sequence identity of 80% or more with a partial base sequence of an editing target strand in a target region present in an intracellular double-stranded DNA and containing at least one mutation for editing selected from the group consisting of deletion, substitution, and insertion of one or more nucleotides as compared with a base sequence of the editing target strand, and
the third single-stranded polynucleotide is 15 to 200 nucleotides in length, and has a base sequence having a sequence identity of 90% or more with a base sequence in the editing target strand 5' or 3' to a position corresponding to the mutation for editing in the first single-stranded polynucleotide, wherein, in the editing target strand, the base sequence having sequence identity with the first single-stranded polynucleotide and the base sequence having sequence identity with the third single-stranded polynucleotide are overlapping, adjacent, or separated by one to nine bases (hereinafter, also referred to as composition I of the present invention).

Composition I of the present invention, introduced into a cell, allows base sequence editing corresponding to a mutation for editing contained in the first single-stranded polynucleotide for a target region present in an intracellular double-stranded DNA. That is, composition I of the present invention can be used for introducing a mutation into a target region present in an intracellular double-stranded DNA. In addition, base sequence editing with composition I of the present invention does not need any exogenous nuclease. Accordingly, in a preferred embodiment, composition I of the present invention contains neither an exogenous nuclease nor a polynucleotide capable of expressing it, and is not used in combination with any of them.

In the present invention, the editing target strand is either one strand of the double strand of the target region, that is, the sense strand or the antisense strand. In a preferred mode, the editing target strand can be the antisense strand.

The base sequence of the first single-stranded polynucleotide (herein, also referred to as an E strand (Editor strand)) contains at least one mutation selected from the group consisting of deletion, substitution, and insertion of one or more base(s) as compared with a partial base sequence of an editing target strand in a target region present in an intracellular double-stranded DNA. Herein, the mutation contained in the first single-stranded polynucleotide is referred to as a mutation for editing. The mutation for editing can be, for example, selected from the group consisting of deletion, substitution, and insertion of 1 to 10 (10, 9, 8, 7, 6, 5, 4, 3, 2, or 1) nucleotides. The mutation for editing may be a combination of multiple different mutations for editing. If multiple mutations for editing are contained, they are preferably present closely within a range of several bases (e.g., within 10 bases, 9 bases, 8 bases, 7 bases, 6 bases, 5 bases, 4 bases, 3 bases, or 2 bases). In a more preferred mode, the base sequence of the first single-stranded polynucleotide can contain only one mutation for editing selected from the group consisting of deletion, substitution, and insertion of one nucleotide as compared with a partial base sequence of an editing target strand in a target region present in an intracellular double-stranded DNA.

The mutation for editing in the first single-stranded nucleotide tends to give higher editing efficiency in the case that the mutation for editing is present around the center than in the case that the mutation for editing is present in the vicinity of the 5' end or in the vicinity of the 3' end. Accordingly, in a preferred embodiment, the position of the introduction of the mutation for editing in the first single-stranded polynucleotide is within a range of 25 to 75%, for example, within a range of 30 to 70%, within a range of 35 to 65%, within a range of 40 to 60%, or within a range of 45 to 55%, more preferably within a range of 49 to 51%, from the 5' end, with the full length from the 5' end to the 3' end defined as 100%.

The length of the first single-stranded polynucleotide may be any length that is within a range of 50 to 200 nucleotides in length and allows base sequence editing without limitation, and can be, for example, 55 to 200 nucleotides in length, 60 to 200 nucleotides in length, 65 to 200 nucleotides in length, 70 to 200 nucleotides in length, 50 to 195 nucleotides in length, 55 to 195 nucleotides in length, 60 to 195 nucleotides in length, 65 to 195 nucleotides in length, 70 to 195 nucleotides in length, 50 to 190 nucleotides in length, 55 to 190 nucleotides in length, 60 to 190 nucleotides in length, 65 to 190 nucleotides in length, 70 to 190 nucleotides in length, 50 to 185 nucleotides in length, 55 to 185 nucleotides in length, 60 to 185 nucleotides in length, 65 to 185 nucleotides in length, 70 to 185 nucleotides in length, 50 to 180 nucleotides in length, 55 to 180 nucleotides in length, 60 to 180 nucleotides in length, 65 to 180 nucleotides in length, 70 to 180 nucleotides in length, 50 to 175 nucleotides in length, 55 to 175 nucleotides in length, 60 to 175 nucleotides in length, 65 to 175 nucleotides in length, 70 to 175 nucleotides in length, 50 to 170 nucleotides in length, 55 to 170 nucleotides in length, 60 to 170 nucleotides in length, 65 to 170 nucleotides in length, 70 to 170 nucleotides in length, 50 to 165 nucleotides in length, 55 to 165 nucleotides in length, 60 to 165 nucleotides in length, 65 to 165 nucleotides in length, 70 to 165 nucleotides in length, 50 to 160 nucleotides in length, 55 to 160 nucleotides in length, 60 to 160 nucleotides in length, 65 to 160 nucleotides in length, 70 to 160 nucleotides in length, 50 to 155 nucleotides in length, 55 to 155 nucleotides in length, 60 to 155 nucleotides in length, 65 to 155 nucleotides in length, 70 to 155 nucleotides in length, 50 to 150 nucleotides in length, 55 to 150 nucleotides in length, 60 to 150 nucleotides in length, 65 to 150 nucleotides in length, 70 to 150 nucleotides in length, 50 to 145 nucleotides in length, 55 to 145 nucleotides in length, 60 to 145 nucleotides in length, 65 to 145 nucleotides in length, 70 to 145 nucleotides in length, 50 to 140 nucleotides in length, 55 to 140 nucleotides in length, 60 to 140 nucleotides in length, 65 to 140 nucleotides in length, 70 to 140 nucleotides in length, 50 to 135 nucleotides in length, 55 to 135 nucleotides in length, 60 to 135 nucleotides in length, 65 to 135 nucleotides in length, 70 to 135 nucleotides in length, 50 to 130 nucleotides in length, 55 to 130 nucleotides in length, 60 to 130 nucleotides in length, 65 to 130 nucleotides in length, 70 to 130 nucleotides in length, 50 to 125 nucleotides in length, 55 to 125 nucleotides in length, 60 to 125 nucleotides in length, 65 to 125 nucleotides in length, 70 to 125 nucleotides in length, 50 to 120 nucleotides in length, 55 to 120 nucleotides in length, 60 to 120 nucleotides in length, 65 to 120 nucleotides in length, 70 to 120 nucleotides in length, 50 to 115 nucleotides in length, 55 to 115 nucleotides in length, 60 to 115 nucleotides in length, 65 to 115 nucleotides in length, 70 to 115 nucleotides in length, 50 to 110 nucleotides in length, 55 to 110 nucleotides in length, 60 to 110 nucleotides in length, 65 to 110 nucleotides in length, 70 to 110 nucleotides in length, 50 to 105 nucleotides in length, 55 to 105 nucleotides in length, 60 to 105 nucleotides in length, 65 to 105 nucleotides in length, 70 to 105 nucleotides in length, 50 to 100 nucleotides in length, 55 to 100 nucleotides in length, 60 to 100 nucleotides in length, 65 to 100 nucleotides in length, 70 to 100 nucleotides in length, 50 to 95 nucleotides in length, 55 to 95 nucleotides in length, 60 to 95 nucleotides in length, 65 to 95 nucleotides in length, 70 to 95 nucleotides in length, 50 to 90 nucleotides in length, 55 to 90 nucleotides in length, 60 to 90 nucleotides in length, 65 to 90 nucleotides in length, or 70 to 90 nucleotides in length, and preferably 50 to 100 nucleotides in length, 60 to 100 nucleotides in length, 70 to 100 nucleotides in length, or 60 to 90 nucleotides in length. The length of the first single-stranded polynucleotide can be, for example, 50 nucleotides or more in length, 55 nucleotides or more in length, 60 nucleotides or more in length, 65 nucleotides or more in length, or 70 nucleotides or more in length, and, 200 nucleotides or less in length, 195 nucleotides or less in length, 190 nucleotides or less in length, 185 nucleotides or less in length, 180 nucleotides or less in length, 175 nucleotides or less in length, 170 nucleotides or less in length, 165 nucleotides or less in length, 160 nucleotides or less in length, 155 nucleotides or less in length, 150 nucleotides or less in length, 145 nucleotides or less in length, 140 nucleotides or less in length, 135 nucleotides or less in length, 130 nucleotides or less in length, 125 nucleotides or less in length, 120 nucleotides or less in length, 115 nucleotides or less in length, 110 nucleotides or less in length, 105 nucleotides or less in length, 100 nucleotides or less in length, 95 nucleotides or less in length, or 90 nucleotides or less in length.

The base sequence of the first single-stranded polynucleotide has a sequence identity of 80% or more with a partial base sequence of an editing target strand in a target region present in an intracellular double-stranded DNA. Herein, a sequence that exhibits sequence identity with the first single-stranded polynucleotide in an editing target strand is also referred to as an "E-strand-correspondent sequence". In an embodiment, several nucleotides (e.g., 10 bases, 9 bases, 8 bases, 7 bases, 6 bases, 5 bases, 4 bases, 3 bases, 2 bases, or 1 base) differing from the E-strand-correspondent sequence or a sequence thereof may be contained at a site other than the mutation for editing (e.g., in the vicinity of the 5' end (a region of 10 bases, 7 bases, 5 bases, 4 bases, 3 bases, 2 bases, or 1 base from the end) or in the vicinity of the 3' end (a region of 10 bases, 7 bases, 5 bases, 4 bases, 3 bases, 2 bases, or 1 base from the end)), as long as enhanced editing efficiency is successfully obtained by the mutation for editing and no unintended mutation is introduced into the intracellular double-stranded DNA. The sequence identity of the base sequence of the first single-stranded polynucleotide with the E-strand-correspondent sequence can vary with the length of the first single-stranded polynucleotide and the mutation contained therein, and can be, for example, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, or 99% or more. In a particular embodiment, in the first single-stranded polynucleotide, a sequence excluding the position of the mutation for editing and a region of 10 bases from the 5' end and a region of 10 bases from the 3' end from the first single-stranded polynucleotide can consist of a base sequence 100% identical to the corresponding base sequence in the editing target strand. In a particular embodiment, the base sequence of the first single-stranded polynucleotide can differ from the E-strand-correspondent sequence only at the mutation for editing that has been introduced for base sequence editing. That is, the sequence of the first single-stranded polynucleotide with the mutation for editing excluded can consist of a base sequence 100% identical to the corresponding base sequence in the editing target strand.

An appropriate sequence in the editing target strand can be selected as the E-strand-correspondent sequence with considering the position of the site to be edited. In a mode of the present invention for the E-strand-correspondent sequence, selection may be made for the E-strand-correspondent sequence to give a higher GC content. In this case, the GC content of the first single-stranded polynucleotide can be, for example, 50%, 55% or more, 60% or more, or 65% or more.

In a mode, the first single-stranded polynucleotide can be a polynucleotide consisting of a base sequence in which, from the 5' end to the 3' end, a "5'-end sequence" of 0 to 10 bases, a "5' identical sequence" of 25 to 100 bases, a "mutated sequence" of 0 to 10 bases, a "3' identical sequence" consisting of 25 to 100 bases, and a "3'-end sequence" of 0 to 10 bases are disposed in this order. Here, the 5'-end sequence and the 3'-end sequence are each a base sequence that may contain mismatches of 10 or less bases with the corresponding base sequence in the E-strand-correspondent sequence. The 5' identical sequence and the 3' identical sequence are each a base sequence identical to the corresponding base sequence in the E-strand-correspondent sequence. In the 5'-end sequence, the base in contact with the 5' identical sequence is a base mismatching with the E-strand-correspondent sequence. In the 3'-end sequence, the base in contact with the 3' identical sequence is a base mismatching with the E-strand-correspondent sequence. The mutated sequence is a base sequence containing the mutation for editing, and a part of the mutated sequence may be a sequence or a base(s) identical to the corresponding sequence or base(s) in the E-strand-correspondent sequence. In the case that the mutation for editing is a substitution or an insertion, the mutated sequence is in contact with the 5' identical sequence or the 3' identical sequence at the base of the mutation for editing; in the case that the mutation for editing is a deletion, the mutated sequence is in contact with the 5' identical sequence or the 3' identical sequence at the deletion site. Preferably, the mismatches contained in the 5'-end sequence and the 3'-end sequence are each of one or less base (in the first single-stranded polynucleotide, the 5'-end sequence is absent if no mismatch is present within 10 bases from the 5' end, and the 3'-end sequence is absent if no mismatch is present within 10 bases from the 3' end), and further preferably neither the 5'-end sequence nor the 3'-end sequence is present. In a preferred embodiment, the ratio between the total length of the 5'-end sequence and the 5' identical sequence and the total length of the 3' identical sequence and the 3'-end sequence can be within a range of 25:75 to 75:25, for example, within a range of 30:70 to 70:30, within a range of 35:65 to 65:35, within a range of 40:60 to 60:40, or within a range of 45:55 to 55:45, more preferably within a range of 49:51 to 51:49, and most preferably 50:50.

The third single-stranded polynucleotide (herein, also referred to as an A' strand) is capable of giving enhanced editing efficiency through being combined with the first single-stranded polynucleotide as compared with base sequence editing only with the first single-stranded polynucleotide. Here, "third single-stranded polynucleotide" is a name for convenience, and this term does not necessarily mean that a second single-stranded polynucleotide (A strand) is a constituent component of composition I of the present invention. TDs containing an E strand and an A strand have been already known, and we first specify an E strand and an A strand as a first single-stranded polynucleotide and a second single-stranded polynucleotide, respectively. To distinguish them from an A' strand, we use the word "third". Therefore, the term "third single-stranded polynucleotide" itself does not mean that a second single-stranded polynucleotide is contained in composition I of the present invention. In addition, the first single-stranded polynucleotide (E strand) and the third single-stranded polynucleotide (A' strand) are not necessarily needed to be used in combination with a second single-stranded polynucleotide (A strand).

The base sequence of the third single-stranded polynucleotide has a sequence identity of 90% or more with a base sequence in the editing target strand 5' or 3' to a position corresponding to the mutation for editing in the first single-stranded polynucleotide, wherein, in the editing target strand, the base sequence having sequence identity with the first single-stranded polynucleotide and the base sequence having sequence identity with the third single-stranded polynucleotide are overlapping, adjacent, or separated by one to nine bases. Herein, the sequence having sequence identity with the third single-stranded polynucleotide in the editing target strand is also referred to as an "A'-strand-correspondent sequence".

In the present invention, being "adjacent" means that the A'-strand-correspondent sequence is consecutively disposed without any intervening base 5' or 3' to the E-strand-correspondent sequence in the editing target strand. Fig. 1 shows an arrangement in the editing target strand in which the A'-strand-correspondent sequence is disposed 5' to the E-strand-correspondent sequence and the E-strand-correspondent sequence and the A'-strand-correspondent sequence are adjacent or separated by one to nine bases.

Even in the case that the E-strand-correspondent sequence and the A'-strand-correspondent sequence are overlapping or separated by one to nine bases in the editing target strand, enhanced editing efficiency is obtained by combining the third single-stranded polynucleotide in such configuration with the first single-stranded polynucleotide, though it is preferable that the E-strand-correspondent sequence and the A'-strand-correspondent sequence be adjacent in the editing target strand.

In an embodiment, the third single-stranded polynucleotide can have a sequence identity of 90% or more with a base sequence in the editing target strand 5' to a position corresponding to the mutation for editing in the first single-stranded polynucleotide.

The base sequence of the third single-stranded polynucleotide does not need to completely match the corresponding base sequence in the editing target strand unless the effect of giving enhanced editing efficiency is lost, and the sequence identity can be 90% or more, for example, 95% or more, 96% or more, 97% or more, 98% or more, or 99% or more, and most preferably the base sequence of the third single-stranded polynucleotide can have a sequence identity of 100%.

The length of the third single-stranded polynucleotide is 15 to 200 nucleotides in length, and may be any length without limitation unless the effect of giving enhanced editing efficiency is lost; although depending on the length of the first single-stranded polynucleotide, the length of the third single-stranded polynucleotide can be, for example, 20 to 200 nucleotides in length, 25 to 200 nucleotides in length, 30 to 200 nucleotides in length, 35 to 200 nucleotides in length, 40 to 200 nucleotides in length, 45 to 200 nucleotides in length, 50 to 200 nucleotides in length, 55 to 200 nucleotides in length, 15 to 195 nucleotides in length, 20 to 195 nucleotides in length, 25 to 195 nucleotides in length, 30 to 195 nucleotides in length, 35 to 195 nucleotides in length, 40 to 195 nucleotides in length, 45 to 195 nucleotides in length, 50 to 195 nucleotides in length, 55 to 195 nucleotides in length, 15 to 190 nucleotides in length, 20 to 190 nucleotides in length, 25 to 190 nucleotides in length, 30 to 190 nucleotides in length, 35 to 190 nucleotides in length, 40 to 190 nucleotides in length, 45 to 190 nucleotides in length, 50 to 190 nucleotides in length, 55 to 190 nucleotides in length, 15 to 185 nucleotides in length, 20 to 185 nucleotides in length, 25 to 185 nucleotides in length, 30 to 185 nucleotides in length, 35 to 185 nucleotides in length, 40 to 185 nucleotides in length, 45 to 185 nucleotides in length, 50 to 185 nucleotides in length, 55 to 185 nucleotides in length, 15 to 180 nucleotides in length, 20 to 180 nucleotides in length, 25 to 180 nucleotides in length, 30 to 180 nucleotides in length, 35 to 180 nucleotides in length, 40 to 180 nucleotides in length, 45 to 180 nucleotides in length, 50 to 180 nucleotides in length, 55 to 180 nucleotides in length, 15 to 175 nucleotides in length, 20 to 175 nucleotides in length, 25 to 175 nucleotides in length, 30 to 175 nucleotides in length, 35 to 175 nucleotides in length, 40 to 175 nucleotides in length, 45 to 175 nucleotides in length, 50 to 175 nucleotides in length, 55 to 175 nucleotides in length, 15 to 170 nucleotides in length, 20 to 170 nucleotides in length, 25 to 170 nucleotides in length, 30 to 170 nucleotides in length, 35 to 170 nucleotides in length, 40 to 170 nucleotides in length, 45 to 170 nucleotides in length, 50 to 170 nucleotides in length, 55 to 170 nucleotides in length, 15 to 165 nucleotides in length, 20 to 165 nucleotides in length, 25 to 165 nucleotides in length, 30 to 165 nucleotides in length, 35 to 165 nucleotides in length, 40 to 165 nucleotides in length, 45 to 165 nucleotides in length, 50 to 165 nucleotides in length, 55 to 165 nucleotides in length, 15 to 160 nucleotides in length, 20 to 160 nucleotides in length, 25 to 160 nucleotides in length, 30 to 160 nucleotides in length, 35 to 160 nucleotides in length, 40 to 160 nucleotides in length, 45 to 160 nucleotides in length, 50 to 160 nucleotides in length, 55 to 160 nucleotides in length, 15 to 155 nucleotides in length, 20 to 155 nucleotides in length, 25 to 155 nucleotides in length, 30 to 155 nucleotides in length, 35 to 155 nucleotides in length, 40 to 155 nucleotides in length, 45 to 155 nucleotides in length, 50 to 155 nucleotides in length, 55 to 155 nucleotides in length, 15 to 150 nucleotides in length, 20 to 150 nucleotides in length, 25 to 150 nucleotides in length, 30 to 150 nucleotides in length, 35 to 150 nucleotides in length, 40 to 150 nucleotides in length, 45 to 150 nucleotides in length, 50 to 150 nucleotides in length, 55 to 150 nucleotides in length, 15 to 145 nucleotides in length, 20 to 145 nucleotides in length, 25 to 145 nucleotides in length, 30 to 145 nucleotides in length, 35 to 145 nucleotides in length, 40 to 145 nucleotides in length, 45 to 145 nucleotides in length, 50 to 145 nucleotides in length, 55 to 145 nucleotides in length, 15 to 140 nucleotides in length, 20 to 140 nucleotides in length, 25 to 140 nucleotides in length, 30 to 140 nucleotides in length, 35 to 140 nucleotides in length, 40 to 140 nucleotides in length, 45 to 140 nucleotides in length, 50 to 140 nucleotides in length, 55 to 140 nucleotides in length, 15 to 135 nucleotides in length, 20 to 135 nucleotides in length, 25 to 135 nucleotides in length, 30 to 135 nucleotides in length, 35 to 135 nucleotides in length, 40 to 135 nucleotides in length, 45 to 135 nucleotides in length, 50 to 135 nucleotides in length, 55 to 135 nucleotides in length, 15 to 130 nucleotides in length, 20 to 130 nucleotides in length, 25 to 130 nucleotides in length, 30 to 130 nucleotides in length, 35 to 130 nucleotides in length, 40 to 130 nucleotides in length, 45 to 130 nucleotides in length, 50 to 130 nucleotides in length, 55 to 130 nucleotides in length, 15 to 125 nucleotides in length, 20 to 125 nucleotides in length, 25 to 125 nucleotides in length, 30 to 125 nucleotides in length, 35 to 125 nucleotides in length, 40 to 125 nucleotides in length, 45 to 125 nucleotides in length, 50 to 125 nucleotides in length, 55 to 125 nucleotides in length, 15 to 120 nucleotides in length, 20 to 120 nucleotides in length, 25 to 120 nucleotides in length, 30 to 120 nucleotides in length, 35 to 120 nucleotides in length, 40 to 120 nucleotides in length, 45 to 120 nucleotides in length, 50 to 120 nucleotides in length, 55 to 120 nucleotides in length, 15 to 115 nucleotides in length, 20 to 115 nucleotides in length, 25 to 115 nucleotides in length, 30 to 115 nucleotides in length, 35 to 115 nucleotides in length, 40 to 115 nucleotides in length, 45 to 115 nucleotides in length, 50 to 115 nucleotides in length, 55 to 115 nucleotides in length, 15 to 110 nucleotides in length, 20 to 110 nucleotides in length, 25 to 110 nucleotides in length, 30 to 110 nucleotides in length, 35 to 110 nucleotides in length, 40 to 110 nucleotides in length, 45 to 110 nucleotides in length, 50 to 110 nucleotides in length, 55 to 110 nucleotides in length, 15 to 105 nucleotides in length, 20 to 105 nucleotides in length, 25 to 105 nucleotides in length, 30 to 105 nucleotides in length, 35 to 105 nucleotides in length, 40 to 105 nucleotides in length, 45 to 105 nucleotides in length, 50 to 105 nucleotides in length, 55 to 105 nucleotides in length, 15 to 100 nucleotides in length, 20 to 100 nucleotides in length, 25 to 100 nucleotides in length, 30 to 100 nucleotides in length, 35 to 100 nucleotides in length, 40 to 100 nucleotides in length, 45 to 100 nucleotides in length, 50 to 100 nucleotides in length, 55 to 100 nucleotides in length, 15 to 95 nucleotides in length, 20 to 95 nucleotides in length, 25 to 95 nucleotides in length, 30 to 95 nucleotides in length, 35 to 95 nucleotides in length, 40 to 95 nucleotides in length, 45 to 95 nucleotides in length, 50 to 95 nucleotides in length, 55 to 95 nucleotides in length, 15 to 90 nucleotides in length, 20 to 90 nucleotides in length, 25 to 90 nucleotides in length, 30 to 90 nucleotides in length, 35 to 90 nucleotides in length, 40 to 90 nucleotides in length, 45 to 90 nucleotides in length, 50 to 90 nucleotides in length, 55 to 90 nucleotides in length, 15 to 85 nucleotides in length, 20 to 85 nucleotides in length, 25 to 85 nucleotides in length, 30 to 85 nucleotides in length, 35 to 85 nucleotides in length, 40 to 85 nucleotides in length, 45 to 85 nucleotides in length, 50 to 85 nucleotides in length, 55 to 85 nucleotides in length, 15 to 80 nucleotides in length, 20 to 80 nucleotides in length, 25 to 80 nucleotides in length, 30 to 80 nucleotides in length, 35 to 80 nucleotides in length, 40 to 80 nucleotides in length, 45 to 80 nucleotides in length, 50 to 80 nucleotides in length, 55 to 80 nucleotides in length, 15 to 75 nucleotides in length, 20 to 75 nucleotides in length, 25 to 75 nucleotides in length, 30 to 75 nucleotides in length, 35 to 75 nucleotides in length, 40 to 75 nucleotides in length, 45 to 75 nucleotides in length, 50 to 75 nucleotides in length, or 55 to 75 nucleotides in length, and preferably 15 to 75 nucleotides in length or 55 to 75 nucleotides in length. The length of the third single-stranded polynucleotide can be, for example, 15 nucleotides or more in length, 20 nucleotides or more in length, 25 nucleotides or more in length, 30 nucleotides or more in length, 35 nucleotides or more in length, 40 nucleotides or more in length, 45 nucleotides or more in length, 50 nucleotides or more in length, or 55 nucleotides or more in length, and, 200 nucleotides or less in length, 195 nucleotides or less in length, 190 nucleotides or less in length, 185 nucleotides or less in length, 180 nucleotides or less in length, 175 nucleotides or less in length, 170 nucleotides or less in length, 165 nucleotides or less in length, 160 nucleotides or less in length, 155 nucleotides or less in length, 150 nucleotides or less in length, 145 nucleotides or less in length, 140 nucleotides or less in length, 135 nucleotides or less in length, 130 nucleotides or less in length, 125 nucleotides or less in length, 120 nucleotides or less in length, 115 nucleotides or less in length, 110 nucleotides or less in length, 105 nucleotides or less in length, 100 nucleotides or less in length, 95 nucleotides or less in length, 90 nucleotides or less in length, 85 nucleotides or less in length, 80 nucleotides or less in length, or 75 nucleotides or less in length.

In an embodiment, the third single-stranded polynucleotide can have a length of, for example, 15% or more, 20% or more, 25% or more, 30% or more, 35% or more, 40% or more, 45% or more, 50% or more, 55% or more, 60% or more, 65% or more, or 70% or more, and, 200% or less, 150% or less, 100% or less, or 95% or less of the length of the first single-stranded polynucleotide, and preferably a length of 70 to 95% of the length of the first single-stranded polynucleotide.

In a preferred embodiment,
the position of the mutation for editing in the first single-stranded polynucleotide is present within a range of 49 to 51% from the 5' end, with the full length from the 5' end to the 3' end defined as 100%, the base sequence of the first single-stranded polynucleotide has a sequence identity of 98% or more with the E-strand-correspondent sequence, and the length of the first single-stranded polynucleotide is 70 to 100 nucleotides in length, and
the third single-stranded polynucleotide has a sequence identity of 90% or more with a base sequence 5' adjacent to the position of the mutation for editing in the first single-stranded polynucleotide in the editing target strand, the E-strand-correspondent sequence and the A'-strand-correspondent sequence are adjacent, and the length of the third single-stranded polynucleotide is 55 to 75 nucleotides in length.

In a mode, composition I of the present invention can further contain a second single-stranded polynucleotide (herein, also referred to as an A strand (Assistant strand)). The second single-stranded polynucleotide is 20 to 200 nucleotides in length, contains a base sequence complementary to a partial base sequence of a region not containing the mutation for editing in the first single-stranded polynucleotide, and is capable of forming a double strand with the first single-stranded polynucleotide. If the second single-stranded polynucleotide contains a base sequence complementary to a region containing the mutation for editing (mutated part or mutated sequence) in the first single-stranded polynucleotide, lowered editing efficiency can result. Accordingly, the second single-stranded polynucleotide does not contain a base sequence complementary to a region containing the mutation for editing in the first single-stranded polynucleotide. The second single-stranded polynucleotide may contain a base sequence complementary to the base sequence of any part within a region 5' or 3' to the mutation for editing in the first single-stranded polynucleotide, as long as the second single-stranded polynucleotide is capable of forming a double strand with the first single-stranded polynucleotide. In a preferred embodiment, the second single-stranded polynucleotide can contain a base sequence complementary to a part within a region 3' to the position of the mutation for editing in the base sequence of the first single-stranded polynucleotide (see Fig. 1).

The length of the base sequence complementary to the first single-stranded polynucleotide in the second single-stranded polynucleotide is any length that allows base sequence editing without limitation, and can be, for example, 20 to 100 nucleotides in length (e.g., 25 to 100 nucleotides in length, 30 to 100 nucleotides in length, 20 to 95 nucleotides in length, 25 to 95 nucleotides in length, 30 to 95 nucleotides in length, 20 to 90 nucleotides in length, 25 to 90 nucleotides in length, 30 to 90 nucleotides in length, 20 to 85 nucleotides in length, 25 to 85 nucleotides in length, 30 to 85 nucleotides in length, 20 to 80 nucleotides in length, 25 to 80 nucleotides in length, 30 to 80 nucleotides in length, 20 to 75 nucleotides in length, 25 to 75 nucleotides in length, 30 to 75 nucleotides in length, 20 to 70 nucleotides in length, 25 to 70 nucleotides in length, 30 to 70 nucleotides in length, 20 to 65 nucleotides in length, 25 to 65 nucleotides in length, 30 to 65 nucleotides in length, 20 to 60 nucleotides in length, 25 to 60 nucleotides in length, 30 to 60 nucleotides in length, 20 to 55 nucleotides in length, 25 to 55 nucleotides in length, 30 to 55 nucleotides in length, 20 to 50 nucleotides in length, 25 to 50 nucleotides in length, 30 to 50 nucleotides in length, 20 to 45 nucleotides in length, 25 to 45 nucleotides in length, 30 to 45 nucleotides in length, 20 to 40 nucleotides in length, 25 to 40 nucleotides in length, 30 to 40 nucleotides in length, 20 to 35 nucleotides in length, 25 to 35 nucleotides in length, or 30 to 35 nucleotides in length), preferably 25 to 50 nucleotides in length, 30 to 50 nucleotides in length, 35 to 50 nucleotides in length, or 30 to 45 nucleotides in length, and more preferably 30 to 35 nucleotides in length.

The length of the second single-stranded polynucleotide is 20 to 200 nucleotides in length, and may be any length that allows base sequence editing without limitation; although depending on the length of the first single-stranded polynucleotide and the position of the mutation therein, the length of the second single-stranded polynucleotide can be, for example, 25 to 200 nucleotides in length, 30 to 200 nucleotides in length, 20 to 195 nucleotides in length, 25 to 195 nucleotides in length, 30 to 195 nucleotides in length, 20 to 190 nucleotides in length, 25 to 190 nucleotides in length, 30 to 190 nucleotides in length, 20 to 185 nucleotides in length, 25 to 185 nucleotides in length, 30 to 185 nucleotides in length, 20 to 180 nucleotides in length, 25 to 180 nucleotides in length, 30 to 180 nucleotides in length, 20 to 175 nucleotides in length, 25 to 175 nucleotides in length, 30 to 175 nucleotides in length, 20 to 170 nucleotides in length, 25 to 170 nucleotides in length, 30 to 170 nucleotides in length, 20 to 165 nucleotides in length, 25 to 165 nucleotides in length, 30 to 165 nucleotides in length, 20 to 160 nucleotides in length, 25 to 160 nucleotides in length, 30 to 160 nucleotides in length, 20 to 155 nucleotides in length, 25 to 155 nucleotides in length, 30 to 155 nucleotides in length, 20 to 150 nucleotides in length, 25 to 150 nucleotides in length, 30 to 150 nucleotides in length, 20 to 145 nucleotides in length, 25 to 145 nucleotides in length, 30 to 145 nucleotides in length, 20 to 140 nucleotides in length, 25 to 140 nucleotides in length, 30 to 140 nucleotides in length, 20 to 135 nucleotides in length, 25 to 135 nucleotides in length, 30 to 135 nucleotides in length, 20 to 130 nucleotides in length, 25 to 130 nucleotides in length, 30 to 130 nucleotides in length, 20 to 125 nucleotides in length, 25 to 125 nucleotides in length, 30 to 125 nucleotides in length, 20 to 120 nucleotides in length, 25 to 120 nucleotides in length, 30 to 120 nucleotides in length, 20 to 115 nucleotides in length, 25 to 115 nucleotides in length, 30 to 115 nucleotides in length, 20 to 110 nucleotides in length, 25 to 110 nucleotides in length, 30 to 110 nucleotides in length, 20 to 105 nucleotides in length, 25 to 105 nucleotides in length, 30 to 105 nucleotides in length, 20 to 100 nucleotides in length, 25 to 100 nucleotides in length, 30 to 100 nucleotides in length, 20 to 95 nucleotides in length, 25 to 95 nucleotides in length, 30 to 95 nucleotides in length, 20 to 90 nucleotides in length, 25 to 90 nucleotides in length, 30 to 90 nucleotides in length, 20 to 85 nucleotides in length, 25 to 85 nucleotides in length, 30 to 85 nucleotides in length, 20 to 80 nucleotides in length, 25 to 80 nucleotides in length, 30 to 80 nucleotides in length, 20 to 75 nucleotides in length, 25 to 75 nucleotides in length, 30 to 75 nucleotides in length, 20 to 70 nucleotides in length, 25 to 70 nucleotides in length, 30 to 70 nucleotides in length, 20 to 65 nucleotides in length, 25 to 65 nucleotides in length, 30 to 65 nucleotides in length, 20 to 60 nucleotides in length, 25 to 60 nucleotides in length, 30 to 60 nucleotides in length, 20 to 55 nucleotides in length, 25 to 55 nucleotides in length, 30 to 55 nucleotides in length, 20 to 50 nucleotides in length, 25 to 50 nucleotides in length, 30 to 50 nucleotides in length, 20 to 45 nucleotides in length, 25 to 45 nucleotides in length, 30 to 45 nucleotides in length, 20 to 40 nucleotides in length, 25 to 40 nucleotides in length, 30 to 40 nucleotides in length, 20 to 35 nucleotides in length, 25 to 35 nucleotides in length, or 30 to 35 nucleotides in length, preferably 25 to 100 nucleotides in length, 30 to 100 nucleotides in length, 35 to 100 nucleotides in length, 30 to 90 nucleotides in length, 25 to 50 nucleotides in length, 30 to 50 nucleotides in length, 35 to 50 nucleotides in length, or 30 to 45 nucleotides in length, more preferably 30 to 35 nucleotides in length (e.g., 33 nucleotides in length, 34 nucleotides in length, or 35 nucleotides in length), and even more preferably 34 nucleotides in length or 35 nucleotides in length.

In an embodiment, the second single-stranded polynucleotide can be a polynucleotide consisting of a base sequence complementary to a base sequence in the editing target strand 5' or 3' to the site to be edited (referred to as an "A-strand-correspondent sequence"). The A-strand-correspondent sequence may be within the range of the E-strand-correspondent sequence, or a sequence extending over either end of the E-strand-correspondent sequence. In the case that the A-strand-correspondent sequence extends over the range of the E-strand-correspondent sequence, the length of the exceeding part can be a length almost the same as the length from the site to be edited to the end of the E-strand-correspondent sequence or a length equal to or smaller than that length, and preferably a length of 90% or less, 80% or less, 70% or less, 60% or less, 50% or less, 40% or less, 30% or less, 20% or less, or 10% or less of the length from the site to be edited to the end of the E-strand-correspondent sequence.

Any base sequence may be inserted to the 5' end or 3' end of the second single-stranded polynucleotide unless lowered editing efficiency is caused. Examples of base sequences therefor include a base sequence that forms a hairpin structure similar to that of a single-stranded DNA in a TD disclosed in International Publication No. WO 2006/064813.

In the case that composition I of the present invention contains three single-stranded polynucleotides of the first to the third, the molar ratio among the first, second, and third single-stranded polynucleotides in the composition may be any value that allows base sequence editing without limitation; however, as the molar ratio of the second single-stranded polynucleotide is lower, or as the ratio of the molar concentration or dose (moles) of the second single-stranded polynucleotide to the total of the molar concentrations or doses of the first and third single-stranded polynucleotides is lower, higher editing efficiency may result. Accordingly, in an embodiment, the molar concentration or dose of the second single-stranded polynucleotide can be 0.2 to 2, preferably 0.2 to 1, and more preferably 0.2 to 0.5, as the total of the molar concentrations or doses of the first and third single-stranded polynucleotides is taken as 1. Otherwise, it is preferable in some cases that the molar concentration or dose of the second single-stranded polynucleotide be equal to or lower than the total of the molar concentrations or doses of the first and third single-stranded polynucleotides. Herein, the term dose means molar concentration in administering to a cell or subject to be subjected to base sequence editing.

In the case that composition I of the present invention contains three single-stranded polynucleotides of the first to the third, or contains two single-stranded polynucleotides of the first and the third, higher editing efficiency may result as the ratio of the molar concentration or dose (moles) of the third single-stranded polynucleotide to the molar concentration or dose of the first single-stranded polynucleotide is higher. Accordingly, in an embodiment, the molar concentration or dose of the third single-stranded polynucleotide can be 0.25 to 4, and preferably 1 to 4, as the molar concentration or dose of the first single-stranded polynucleotide is taken as 1. Otherwise, it is preferable in some cases that the molar concentration or dose of the third single-stranded polynucleotide be equal to or higher than the molar concentration or dose of the first single-stranded polynucleotide.

Higher editing efficiency may result as the ratio of the molar concentration or dose (moles) of the third single-stranded polynucleotide to the molar concentration or dose of the second single-stranded polynucleotide is higher. Accordingly, in an embodiment, in the case that composition I of the present invention contains three single-stranded polynucleotides of the first to the third, the molar concentration or dose of the third single-stranded polynucleotide can be 0.25 to 4, and preferably 1 to 4, as the molar concentration or dose of the second single-stranded polynucleotide is taken as 1. Otherwise, it is preferable in some cases that the molar concentration or dose of the third single-stranded polynucleotide be equal to or higher than the molar concentration or dose of the second single-stranded polynucleotide.

In a preferred embodiment,
the position of the mutation for editing in the first single-stranded polynucleotide is present within a range of 49 to 51%, with the full length from the 5' end to the 3' end defined as 100%, the base sequence of the first single-stranded polynucleotide has a sequence identity of 98% or more with the E-strand-correspondent sequence, and the length of the first single-stranded polynucleotide is 70 to 100 nucleotides in length,
the second single-stranded polynucleotide contains a base sequence complementary to a part within a region 3' to the position of the mutation for editing in the base sequence of the first single-stranded polynucleotide, and the length of the second single-stranded polynucleotide is 30 to 100 nucleotides in length, and
the third single-stranded polynucleotide has a sequence identity of 90% or more with a base sequence in the editing target strand 5' to the position of the mutation for editing in the first single-stranded polynucleotide, the E-strand-correspondent sequence and the A'-strand-correspondent sequence are adjacent, and the length of the third single-stranded polynucleotide is 55 to 75 nucleotides in length.

Each of the first to third single-stranded polynucleotides may be any of a DNA, an RNA, and a DNA/RNA chimera as long as base sequence editing is allowed, but is preferably a DNA. Each of the first to third single-stranded polynucleotides may contain a modified nucleotide or a modified internucleotide bond as long as base sequence editing is allowed.

In an embodiment, the first single-stranded polynucleotide can have at least one of the following features:
(a) at least one nucleotide in a region consisting of the position of the mutation for editing and a 10-base sequence 5' adjacent to the position of the mutation for editing and a 10-base sequence 3' adjacent to the mutation for editing in the first single-stranded polynucleotide is a sugar-modified nucleotide; and
(b) the first single-stranded polynucleotide contains at least one modified internucleotide bond, and the modified internucleotide bond is absent in a range from a third base 5' to the position of the mutation for editing to a third base 3' to the position of the mutation for editing.

In specifying the position of a sugar-modified nucleotide for feature (a), the position of the nearest nucleotide to the mutation for editing among nucleotides identical to those of the editing target strand 5' and 3' to the position of the mutation for editing in the first single-stranded polynucleotide is designated as position 1. For example, "position N located 5' to the position of the mutation for editing" refers to the Nth nearest nucleotide to the mutation for editing among nucleotides identical to those of the editing target strand 5' to the position of the mutation for editing in the first single-stranded polynucleotide. Herein, "position N located 5' to the position of the mutation for editing" is occasionally referred to as "position - N", and "position N located 3' to the position of the mutation for editing" is occasionally referred to as "position +N". "Position N located 3' to the position of the mutation for editing" refers to the Nth nearest nucleotide to the mutation for editing among nucleotides identical to those of the editing target strand 3' to the position of the mutation for editing in the first single-stranded polynucleotide. In the case that the first single-stranded polynucleotide contains multiple mutations for editing, the position of a sugar-modified nucleotide located closer to the 5' end is specified with reference to the position of the mutation for editing positioned closest to the 5' end among the mutations for editing, and the position of a sugar-modified nucleotide located closer to the 3' end is specified with reference to the position of the mutation for editing positioned closest to the 3' end among the mutations for editing. The "a 10-base sequence 5' adjacent to the position of the mutation for editing" are bases at position 1 to position 10 located 5' to the position of the mutation for editing. The "a 10-base sequence 3' adjacent to the position of the mutation for editing" are bases at position 1 to position 10 located 3' to the position of the mutation for editing.

For the number and positions of sugar-modified nucleotides to be introduced into the first single-stranded polynucleotide, appropriate nucleotides can be selected from the nucleotides contained in the region consisting of the position of the mutation for editing and a 10-base sequence 5' adjacent to the position of the mutation for editing and a 10-base sequence 3' adjacent to the position of the mutation for editing, as long as the selected ones allow base sequence editing. Likewise, the number of sugar-modified nucleotides is not limited, and can be, for example, 15 or less, and is preferably 10, 9, 8, 7, 6, 5, 4, 3, 2, or 1.

In an embodiment, in the first single-stranded polynucleotide, at least one selected from the position of the mutation for editing and the nucleotides at position 1, position 3, position 5, position 7, and position 9 located 5' to the position of the mutation for editing and at least one selected from the nucleotides at position 1, position 3, position 5, position 7, and position 9 located 3' to the position of the mutation for editing can be each a sugar-modified nucleotide; for example, the nucleotide at position 1 located 5' to the position of the mutation for editing and the nucleotide at position 1 located 3' to the position of the mutation for editing, the nucleotides at position 1 and position 3 located 5' to the position of the mutation for editing and the nucleotides at position 1 and position 3 located 3' to the position of the mutation for editing, the nucleotides at position 1, position 3, and position 5 located 5' to the position of the mutation for editing and the nucleotides at position 1, position 3, and position 5 located 3' to the position of the mutation for editing, the nucleotides at position 1, position 3, position 5, and position 7 located 5' to the position of the mutation for editing and the nucleotides at position 1, position 3, position 5, and position 7 located 3' to the position of the mutation for editing, or the nucleotides at position 1, position 3, position 5, position 7, and position 9 located 5' to the position of the mutation for editing and the nucleotides at position 1, position 3, position 5, position 7, and position 9 located 3' to the position of the mutation for editing can be each a sugar-modified nucleotide.

In a particular embodiment, in the first single-stranded polynucleotide,
the nucleotide at the position of the mutation for editing,
the nucleotide at position 1 located 5' to the position of the mutation for editing, the nucleotide at the position of the mutation for editing, and the nucleotide at position 1 located 3' to the position of the mutation for editing,
the nucleotide at position 1 located 5' to the position of the mutation for editing and the nucleotide at position 1 located 3' to the position of the mutation for editing,
the nucleotide at position 1 located 5' to the position of the mutation for editing, the nucleotide at the position of the mutation for editing, and the nucleotide at position 1 located 3' to the position of the mutation for editing,
the nucleotides at position 3 and position 1 located 5' to the position of the mutation for editing and the nucleotides at position 1 and position 3 located 3' to the position of the mutation for editing,
the nucleotides at position 5, position 3, and position 1 located 5' to the position of the mutation for editing and the nucleotides at position 1, position 3, and position 5 located 3' to the position of the mutation for editing,
the nucleotide at position 2 located 5' to the position of the mutation for editing and the nucleotide at position 2 located 3' to the position of the mutation for editing,
the nucleotides at position 2 and position 1 located 5' to the position of the mutation for editing and the nucleotides at position 1 and position 2 located 3' to the position of the mutation for editing,
the nucleotides at position 3 and position 2 located 5' to the position of the mutation for editing and the nucleotides at position 2 and position 3 located 3' to the position of the mutation for editing,
the nucleotides at position 3, position 2, and position 1 located 5' to the position of the mutation for editing and the nucleotides at position 1, position 2, and position 3 located 3' to the position of the mutation for editing,
the nucleotides at position 7, position 5, position 3, and position 1 located 5' to the position of the mutation for editing and the nucleotides at position 1, position 3, position 5, and position 7 located 3' to the position of the mutation for editing,
the nucleotides at position 9, position 7, position 5, position 3, and position 1 located 5' to the position of the mutation for editing and the nucleotides at position 1, position 3, position 5, position 7, and position 9 located 3' to the position of the mutation for editing,
any one of the nucleotides at position 1 to position 6 located 5' to the position of the mutation for editing,
any one of the nucleotides at position 1 to position 6 located 3' to the position of the mutation for editing,
the nucleotide at position 2 located 5' to the position of the mutation for editing and the nucleotide at position 2 located 3' to the position of the mutation for editing,
the nucleotide at position 3 located 5' to the position of the mutation for editing and the nucleotide at position 3 located 3' to the position of the mutation for editing,
the nucleotide at position 4 located 5' to the position of the mutation for editing and the nucleotide at position 4 located 3' to the position of the mutation for editing,
the nucleotide at position 5 located 5' to the position of the mutation for editing and the nucleotide at position 5 located 3' to the position of the mutation for editing,
the nucleotide at position 6 located 5' to the position of the mutation for editing and the nucleotide at position 6 located 3' to the position of the mutation for editing,
the nucleotides at position 5 and position 3 located 5' to the position of the mutation for editing,
the nucleotides at position 4 and position 2 located 5' to the position of the mutation for editing,
the nucleotides at position 3 and position 1 located 5' to the position of the mutation for editing,
the nucleotides at position 1 and position 3 located 3' to the position of the mutation for editing,
the nucleotides at position 2 and position 4 located 3' to the position of the mutation for editing,
the nucleotides at position 3 and position 5 located 3' to the position of the mutation for editing,
the nucleotide at position 7 located 5' to the position of the mutation for editing and the nucleotide at the position of the mutation for editing,
the nucleotide at position 6 located 5' to the position of the mutation for editing and the nucleotide at the position of the mutation for editing,
the nucleotide at position 5 located 5' to the position of the mutation for editing and the nucleotide at the position of the mutation for editing,
the nucleotide at position 4 located 5' to the position of the mutation for editing and the nucleotide at the position of the mutation for editing,
the nucleotide at position 3 located 5' to the position of the mutation for editing and the nucleotide at the position of the mutation for editing,
the nucleotide at position 2 located 5' to the position of the mutation for editing and the nucleotide at the position of the mutation for editing,
the nucleotide at position 1 located 5' to the position of the mutation for editing and the nucleotide at the position of the mutation for editing,
the nucleotide at the position of the mutation for editing and the nucleotide at position 1 located 3' to the position of the mutation for editing,
the nucleotide at the position of the mutation for editing and the nucleotide at position 2 located 3' to the position of the mutation for editing,
the nucleotide at the position of the mutation for editing and the nucleotide at position 3 located 3' to the position of the mutation for editing,
the nucleotide at the position of the mutation for editing and the nucleotide at position 4 located 3' to the position of the mutation for editing,
the nucleotide at the position of the mutation for editing and the nucleotide at position 5 located 3' to the position of the mutation for editing,
the nucleotide at the position of the mutation for editing and the nucleotide at position 6 located 3' to the position of the mutation for editing,
the nucleotide at the position of the mutation for editing and the nucleotide at position 7 located 3' to the position of the mutation for editing,
the nucleotide at position 1 located 5' to the position of the mutation for editing, the nucleotide at the position of the mutation for editing, and the nucleotide at position 1 located 3' to the position of the mutation for editing,
the nucleotide at position 2 located 5' to the position of the mutation for editing, the nucleotide at the position of the mutation for editing, and the nucleotide at position 2 located 3' to the position of the mutation for editing,
the nucleotide at position 3 located 5' to the position of the mutation for editing, the nucleotide at the position of the mutation for editing, and the nucleotide at position 3 located 3' to the position of the mutation for editing,
the nucleotide at position 4 located 5' to the position of the mutation for editing, the nucleotide at the position of the mutation for editing, and the nucleotide at position 4 located 3' to the position of the mutation for editing,
the nucleotide at position 5 located 5' to the position of the mutation for editing, the nucleotide at the position of the mutation for editing, and the nucleotide at position 5 located 3' to the position of the mutation for editing,
the nucleotide at position 6 located 5' to the position of the mutation for editing, the nucleotide at the position of the mutation for editing, and the nucleotide at position 6 located 3' to the position of the mutation for editing,
the nucleotide at position 7 located 5' to the position of the mutation for editing, the nucleotide at the position of the mutation for editing, and the nucleotide at position 7 located 3' to the position of the mutation for editing,
the nucleotides at position 2 and position 1 located 5' to the position of the mutation for editing and the nucleotides at position 1 and position 2 located 3' to the position of the mutation for editing,
the nucleotides at position 3 and position 2 located 5' to the position of the mutation for editing and the nucleotides at position 2 and position 3 located 3' to the position of the mutation for editing,
the nucleotides at position 3, position 2, and position 1 located 5' to the position of the mutation for editing and the nucleotides at position 1, position 2, and position 3 located 3' to the position of the mutation for editing,
the nucleotide at position 1 located 5' to the position of the mutation for editing and the nucleotides at position 1 and position 4 located 3' to the position of the mutation for editing,
the nucleotides at position 3 and position 1 located 5' to the position of the mutation for editing and the nucleotides at position 1 and position 3 located 3' to the position of the mutation for editing,
the nucleotides at position 3 and position 1 located 5' to the position of the mutation for editing and the nucleotides at position 1, position 3, and position 4 located 3' to the position of the mutation for editing,
the nucleotide at the position of the mutation for editing and the nucleotides at position 1 and position 4 located 3' to the position of the mutation for editing,
the nucleotide at position 1 located 5' to the position of the mutation for editing, the nucleotide at the position of the mutation for editing, and the nucleotide at position 4 located 3' to the position of the mutation for editing,
the nucleotide at the position of the mutation for editing and the nucleotides at position 2 and position 4 located 3' to the position of the mutation for editing, or
the nucleotide at position 2 located 5' to the position of the mutation for editing, the nucleotide at the position of the mutation for editing, and the nucleotide at position 4 located 3' to the position of the mutation for editing
can be each a sugar-modified nucleotide.

In a preferred embodiment, in the first single-stranded polynucleotide,
the nucleotide at the position of the mutation for editing,
the nucleotide at position 1 located 5' to the position of the mutation for editing, the nucleotide at the position of the mutation for editing, and the nucleotide at position 1 located 3' to the position of the mutation for editing,
the nucleotide at position 1 located 5' to the position of the mutation for editing and the nucleotide at position 1 located 3' to the position of the mutation for editing,
the nucleotide at position 1 located 5' to the position of the mutation for editing, the nucleotide at the position of the mutation for editing, and the nucleotide at position 1 located 3' to the position of the mutation for editing,
the nucleotides at position 3 and position 1 located 5' to the position of the mutation for editing and the nucleotides at position 1 and position 3 located 3' to the position of the mutation for editing,
the nucleotides at position 5, position 3, and position 1 located 5' to the position of the mutation for editing and the nucleotides at position 1, position 3, and position 5 located 3' to the position of the mutation for editing,
the nucleotide at position 3 located 5' to the position of the mutation for editing and the nucleotide at position 3 located 3' to the position of the mutation for editing,
the nucleotides at position 1 and position 3 located 3' to the position of the mutation for editing,
the nucleotide at position 2 located 5' to the position of the mutation for editing and the nucleotide at the position of the mutation for editing,
the nucleotide at position 1 located 5' to the position of the mutation for editing and the nucleotide at the position of the mutation for editing,
the nucleotide at the position of the mutation for editing and the nucleotide at position 1 located 3' to the position of the mutation for editing,
the nucleotide at the position of the mutation for editing and the nucleotide at position 2 located 3' to the position of the mutation for editing,
the nucleotide at the position of the mutation for editing and the nucleotide at position 3 located 3' to the position of the mutation for editing,
the nucleotide at the position of the mutation for editing and the nucleotide at position 4 located 3' to the position of the mutation for editing,
the nucleotide at the position of the mutation for editing and the nucleotide at position 5 located 3' to the position of the mutation for editing,
the nucleotide at the position of the mutation for editing and the nucleotide at position 6 located 3' to the position of the mutation for editing,
the nucleotide at the position of the mutation for editing and the nucleotide at position 7 located 3' to the position of the mutation for editing,
the nucleotide at position 2 located 5' to the position of the mutation for editing, the nucleotide at the position of the mutation for editing, and the nucleotide at position 2 located 3' to the position of the mutation for editing,
the nucleotide at position 3 located 5' to the position of the mutation for editing, the nucleotide at the position of the mutation for editing, and the nucleotide at position 3 located 3' to the position of the mutation for editing,
the nucleotide at position 4 located 5' to the position of the mutation for editing, the nucleotide at the position of the mutation for editing, and the nucleotide at position 4 located 3' to the position of the mutation for editing,
the nucleotide at position 5 located 5' to the position of the mutation for editing, the nucleotide at the position of the mutation for editing, and the nucleotide at position 5 located 3' to the position of the mutation for editing,
the nucleotide at position 1 located 5' to the position of the mutation for editing and the nucleotides at position 1 and position 4 located 3' to the position of the mutation for editing, the nucleotides at position 3 and position 1 located 5' to the position of the mutation for editing and the nucleotides at position 1, position 3, and position 4 located 3' to the position of the mutation for editing,
the nucleotide at the position of the mutation for editing and the nucleotides at position 1 and position 4 located 3' to the position of the mutation for editing,
the nucleotide at position 1 located 5' to the position of the mutation for editing, the nucleotide at the position of the mutation for editing, and the nucleotide at position 4 located 3' to the position of the mutation for editing,
the nucleotide at the position of the mutation for editing and the nucleotides at position 2 and position 4 located 3' to the position of the mutation for editing, or
the nucleotide at position 2 located 5' to the position of the mutation for editing, the nucleotide at the position of the mutation for editing, and the nucleotide at position 4 located 3' to the position of the mutation for editing
can be each a sugar-modified nucleotide.

In a preferred embodiment, each sugar-modified nucleotide can be a nucleotide containing a 4'-lower linear alkylene-O-2' cross-linkage in the ribose ring, more preferably a nucleotide containing a 2'-O,4'-C-methylene cross-linkage or a 2'-O,4'-C-ethylene cross-linkage in the ribose ring, and further preferably an LNA or an ENA.

The position of a modified internucleotide bond for feature (b) is represented as the position of a base of a nucleotide having a modification at the phosphate moiety at the 3' side. The position of a modified internucleotide bond in the vicinity of the mutation for editing is specified with reference to the position of the nearest base to the mutation for editing (position 1) among bases identical to those of the editing target strand 5' and 3' to the mutation for editing in the first single-stranded polynucleotide. In the case that the first single-stranded polynucleotide contains multiple mutations for editing, the position of a modified internucleotide bond at the 5' side is specified with reference to the position of the mutation for editing positioned closest to the 5' end among the mutations for editing, and the position of a modified internucleotide bond at the 3' side is specified with reference to the position of the mutation for editing positioned closest to the 3' end among the mutations for editing.

If a modified internucleotide bond is present within a range from the third base 5' to the position of the mutation for editing to the third base 3' to the position of the mutation for editing in the first single-stranded polynucleotide (i.e., a range from the base at position 3 located 5' to the mutation for editing to the base at position 3 located 3' to the mutation for editing), lowered editing efficiency may result. Accordingly, no modified internucleotide bond is disposed within that range.

In an embodiment, the first single-stranded polynucleotide can contain one to four (e.g., one, two, three, or four) modified internucleotide bonds near the 5' end (e.g., within 20 bases, within 15 bases, within 13 bases, within 10 bases, within 8 bases, within 6 bases, within 5 bases, within 4 bases, within 3 bases, or within 2 bases from the 5' end) and/or near the 3' end (e.g., within 20 bases, within 15 bases, within 13 bases, within 10 bases, within 8 bases, within 6 bases, within 5 bases, within 4 bases, within 3 bases, or within 2 bases from the 3' end). Each modified internucleotide bond in this case is not limited, and can be preferably a phosphorothioate bond (also expressed as a PS modification), an alkylphosphonate bond, or a phosphotriester bond, and is more preferably a phosphorothioate bond. In a preferred embodiment, the first single-stranded polynucleotide can contain one to four modified internucleotide bonds within 13 bases (more preferably, within five bases) from the 3' end. In a more preferred embodiment, the first single-stranded polynucleotide can contain two modified internucleotide bonds within three bases from the 3' end or one modified internucleotide bond within two bases from the 3' end. In an even more preferred embodiment, the first single-stranded polynucleotide contains one to four modified internucleotide bonds within 13 bases (preferably two modified internucleotide bonds within three bases or one modified internucleotide bond within two bases) from the 3' end, and each modified internucleotide bond can be a phosphorothioate bond.

In a preferred embodiment,
the position of the mutation for editing in the first single-stranded polynucleotide is present within a range of 49 to 51% from the 5' end, with the full length from the 5' end to the 3' end defined as 100%, the base sequence of the first single-stranded polynucleotide has a sequence identity of 98% or more with the E-strand-correspondent sequence, and the length of the first single-stranded polynucleotide is 70 to 100 nucleotides in length,
in the first single-stranded polynucleotide,

the nucleotide at the position of the mutation for editing,
the nucleotide at position 1 located 5' to the position of the mutation for editing, the nucleotide at the position of the mutation for editing, and the nucleotide at position 1 located 3' to the position of the mutation for editing,
the nucleotide at position 1 located 5' to the position of the mutation for editing, and the nucleotide at position 1 located 3' to the position of the mutation for editing,
the nucleotide at position 1 located 5' to the position of the mutation for editing, the nucleotide at the position of the mutation for editing, and the nucleotide at position 1 located 3' to the position of the mutation for editing,
the nucleotides at position 3 and position 1 located 5' to the position of the mutation for editing and the nucleotides at position 1 and position 3 located 3' to the position of the mutation for editing,
the nucleotides at position 5, position 3, and position 1 located 5' to the position of the mutation for editing and the nucleotides at position 1, position 3, and position 5 located 3' to the position of the mutation for editing,
the nucleotide at position 3 located 5' to the position of the mutation for editing and the nucleotide at position 3 located 3' to the position of the mutation for editing,
the nucleotides at position 1 and position 3 located 3' to the position of the mutation for editing,
the nucleotide at position 2 located 5' to the position of the mutation for editing and the nucleotide at the position of the mutation for editing,
the nucleotide at position 1 located 5' to the position of the mutation for editing and the nucleotide at the position of the mutation for editing,
the nucleotide at the position of the mutation for editing and the nucleotide at position 1 located 3' to the position of the mutation for editing,
the nucleotide at the position of the mutation for editing and the nucleotide at position 2 located 3' to the position of the mutation for editing,
the nucleotide at the position of the mutation for editing and the nucleotide at position 3 located 3' to the position of the mutation for editing,
the nucleotide at the position of the mutation for editing and the nucleotide at position 4 located 3' to the position of the mutation for editing,
the nucleotide at the position of the mutation for editing and the nucleotide at position 5 located 3' to the position of the mutation for editing,
the nucleotide at the position of the mutation for editing and the nucleotide at position 6 located 3' to the position of the mutation for editing,
the nucleotide at the position of the mutation for editing and the nucleotide at position 7 located 3' to the position of the mutation for editing,
the nucleotide at position 2 located 5' to the position of the mutation for editing, the nucleotide at the position of the mutation for editing, and the nucleotide at position 2 located 3' to the position of the mutation for editing,
the nucleotide at position 3 located 5' to the position of the mutation for editing, the nucleotide at the position of the mutation for editing, and the nucleotide at position 3 located 3' to the position of the mutation for editing,
the nucleotide at position 4 located 5' to the position of the mutation for editing, the nucleotide at the position of the mutation for editing, and the nucleotide at position 4 located 3' to the position of the mutation for editing,
the nucleotide at position 5 located 5' to the position of the mutation for editing, the nucleotide at the position of the mutation for editing, and the nucleotide at position 5 located 3' to the position of the mutation for editing,
the nucleotide at position 1 located 5' to the position of the mutation for editing and the nucleotides at position 1 and position 4 located 3' to the position of the mutation for editing, the nucleotides at position 3 and position 1 located 5' to the position of the mutation for editing and the nucleotides at position 1, position 3, and position 4 located 3' to the position of the mutation for editing,
the nucleotide at the position of the mutation for editing and the nucleotides at position 1 and position 4 located 3' to the position of the mutation for editing,
the nucleotide at position 1 located 5' to the position of the mutation for editing, the nucleotide at the position of the mutation for editing, and the nucleotide at position 4 located 3' to the position of the mutation for editing,
the nucleotide at the position of the mutation for editing and the nucleotides at position 2 and position 4 located 3' to the position of the mutation for editing, or
the nucleotide at position 2 located 5' to the position of the mutation for editing, the nucleotide at the position of the mutation for editing, and the nucleotide at position 4 located 3' to the position of the mutation for editing
are each a sugar-modified nucleotide,
   the sugar-modified nucleotide is a nucleotide containing a 4'-lower linear alkylene-O-2' cross-linkage in the ribose ring, more preferably a nucleotide containing a 2'-O,4'-C-methylene cross-linkage or a 2'-O,4'-C-ethylene cross-linkage in the ribose ring, and further preferably an LNA or an ENA,
   the first single-stranded polynucleotide contains one to four modified internucleotide bonds within five bases from the 3' end, and more preferably contains two modified internucleotide bonds within three bases from the 3' end or one modified internucleotide bond within two bases from the 3' end,
   each modified internucleotide bond is a phosphorothioate bond,
   the second single-stranded polynucleotide contains a base sequence complementary to a part 3' to the position of the mutation for editing in the base sequence of the first single-stranded polynucleotide, and the length of the second single-stranded polynucleotide is 30 to 100 nucleotides in length, and
   the third single-stranded polynucleotide has a sequence identity of 90% or more with a base sequence in the editing target strand 5' to the position of the mutation for editing in the first single-stranded polynucleotide, the E-strand-correspondent sequence and the A'-strand-correspondent sequence are adjacent, and the length of the third single-stranded polynucleotide is 55 to 75 nucleotides in length.

In an embodiment, the second single-stranded polynucleotide can contain at least one modified internucleotide bond. For the number and positions of modified internucleotide bonds, appropriate ones can be selected, as long as the selected ones allow base sequence editing. In a preferred embodiment, the second single-stranded polynucleotide contains one or two modified internucleotide bonds within three bases from the 5' end and/or 3' end or one modified internucleotide bond within two bases from the 5' end and/or 3' end, and can more preferably contain one or two modified internucleotide bonds within three bases from the 3' end or one modified internucleotide bond within two bases from the 3' end. In a preferred embodiment, each modified internucleotide bond can be a phosphorothioate bond, an alkylphosphonate bond, or a phosphotriester bond, more preferably an alkylphosphonate bond or a phosphotriester bond, and even more preferably a methylphosphonate bond or an ethylphosphotriester bond.

In an embodiment, each of the first to third single-stranded polynucleotides can have a chemical modification at the 5' end or the 3' end. Examples of chemical modifications include a linker such as an amino linker. The amino linker is a linker represented by formula (AL) in the following: 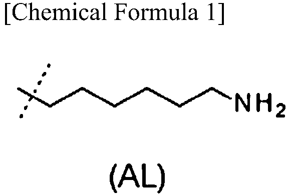 wherein the dashed line indicates bonding, representing that the methylene group is bound to the 5'-phosphate group or 3'-phosphate group of the nucleotide at the 5'-end of a single-stranded polynucleotide via a phosphodiester bond (hereinafter, also referred to as an AL linker). In bonding the AL linker to the 5' end of a single-stranded polynucleotide, 5'-Amino-Modifier C6 (product number: 0-1906-02, manufactured by Glen Research) or the like can be used, and in bonding to the 3' end, Phthalamido Amino C6 lcaa CPG (product number: N-8217-10, manufactured by ChemGenes Corporation) or the like can be used.

Examples of linkers having an amino group at an end other than the aforementioned AL linker having six methylene groups include a linker having an amino acid group at an end of 3 to 12 methylene groups. In bonding the present liner to the 5' end of a single-stranded polynucleotide, a linker having an amino acid group at an end of 4, 5, or 12 methylene groups can be obtained, for example, by correspondingly using TFA-Amino C-4 CED phosphoramidite (product number: CLP-1453, manufactured by ChemGenes Corporation), TFA amino C-5 CED phosphoramidite (product number: CLP-1357, manufactured by ChemGenes Corporation), or MMT-Amino C-12 CED phosphoramidite (product number: CLP-1453, manufactured by ChemGenes Corporation). In bonding the present linker to the 3' end of a single-stranded polynucleotide, a linker having an amino acid group at an end of three or six methylene groups can be obtained, for example, by correspondingly using 3'-Amino Modifier C-3 lcaa CPG (product number: N-9750-10, manufactured by ChemGenes Corporation) or 3'-Amino Modifier TFA Amino C-6 lcaa CPG (product number: N-1004-10, manufactured by ChemGenes Corporation).

Examples of other chemical modifications include a linker having a hydroxy group at an end. In bonding the present linker to the 5' end of a single-stranded polynucleotide, a linker having a hydroxy group at an end of 2, 3, 4, 6, 9, or 12 methylene groups can be obtained, for example, by correspondingly using DMT-ethane-Diol phosphoramidite (product number: CLP-2250, manufactured by ChemGenes Corporation), DMT-propane-Diol phosphoramidite (product number: CLP-1368, manufactured by ChemGenes Corporation), DMT-butane-Diol phosphoramidite (product number: CLP-9775, manufactured by ChemGenes Corporation), DMT-hexane-Diol phosphoramidite (product number: CLP-9765, manufactured by ChemGenes Corporation), DMT-nonane-Diol phosphoramidite (product number: CLP-9009, manufactured by ChemGenes Corporation), or DMT-dodecane-Diol phosphoramidite (product number: CLP-1114, manufactured by ChemGenes Corporation). A further example is a linker having a hydroxy group at an end of linked ethyloxy groups.

A linker having a hydroxy group at an end of three, four, or six linked ethyloxy groups can be obtained, for example, by correspondingly using DMT-triethyloxy-Glycol phosphoramidite (product number: CLP-1113, manufactured by ChemGenes Corporation), DMT-tetraethyloxy-Glycol CED phosphoramidite (product number: CLP-1368, manufactured by ChemGenes Corporation), or DMT Hexaethylene Glycol phosphoramidite (product number: CLP-9765, manufactured by ChemGenes Corporation). In bonding the present linker to the 3' end of a single-stranded polynucleotide, the linker can be obtained by bonding any of the phosphoramidite reagents to Universal UnyLinker Support (product number: N-4000-20, N-4000-10, N-4000-05, N-4000-03, manufactured by ChemGenes Corporation) or the like.

In an embodiment, any two or three of the first to third single-stranded polynucleotides may be linked via a linker. With a linker, an end of one single-stranded polynucleotide and an end of another single-stranded polynucleotide can be linked. For example, the 3' end of the first single-stranded polynucleotide and the 5' end of the second or third single-stranded polynucleotide, the 3' end of the second single-stranded polynucleotide and the 5' end of the first or third single-stranded polynucleotide, or the 3' end of the third single-stranded polynucleotide and the 5' end of the first or second single-stranded polynucleotide can be linked via a linker. In the present invention, pharmaceutically acceptable linkers known in the art can be used, and examples thereof include that described in WO 2012/074038. In a particular embodiment, the linker can be a linker represented by formula (Z) in the following: 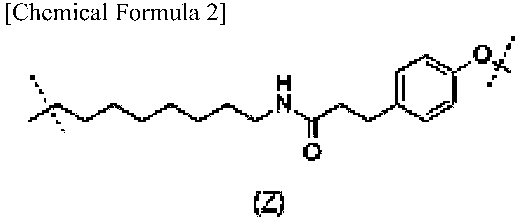 wherein the dashed lines each indicate bonding, representing that the oxygen bound to the phenyl group is bound to the 5'-phosphate group of the nucleotide at the 5' end of one single-stranded polynucleotide via a phosphodiester bond, and that the methylene group at the other end is bound to the 3'-phosphate group of the nucleotide at the 3' end of another single-stranded polynucleotide via a phosphodiester bond (hereinafter, also referred to as a Z linker). The Z linker can be appropriately prepared according to the description in WO 2012/074038.

Examples of other linkers include a linker having a disulfide bond (SS linker), which is cleaved under intracellular reductive conditions. The SS linker can be obtained, for example, by using 5'-Thiol C-6 Disulfide Modifier CED phosphoramidite (product number: CLP-8506, manufactured by ChemGenes Corporation).

The first, second, and third single-stranded polynucleotides can be produced by using a known chemical synthesis method. Alternatively, a single-stranded polynucleotide of intended length can be produced by synthesizing multiple short strands (e.g., about 100 nucleotides in length) and linking them by a known ligation method. Moreover, the first single-stranded polynucleotide can be produced by preparing a phage or phagemid DNA by using mutation-introducing PCR with a double-stranded DNA having a target region as a template, a commercially available mutation introduction kit, or the like and treating the phage or phagemid DNA with restriction enzymes.

In an embodiment, an abnormal base sequence causative of a disease is contained in the target region, and the base sequence of the first single-stranded polynucleotide can contain the mutation for editing so that the abnormal base sequence is repaired and thereby the disease is treated or prevented (e.g., so as to recover the normal base sequence, or give a base sequence differing from the normal base sequence but removed of the cause of the disease). The abnormal base sequence is a base sequence formed by causing the normal base sequence to have at least one mutation selected from the group consisting of deletion, substitution, and insertion of one or more nucleotides. Herein, repair of the abnormal base sequence is not limited to returning to the normal base sequence (or the wild-type base sequence), and the meaning can include introduction of a mutation that gives a synonymous codon encoding the same amino acid as the corresponding codon in the normal base sequence encodes, and introduction of a mutation that gives a codon encoding an amino acid differing from that encoded by the corresponding codon in the normal base sequence. Examples of mutations causative of a disease include single nucleotide substitutions in factor XI gene for hemophilia, ASS1 gene for citrullinemia type I, SLC25A13 gene for citrullinemia type II, Factor V Leiden gene for hemophilia, SERPINA1 gene for α1-antitrypsin deficiency, CBS gene for homocystinuria, PAH gene for phenylketonuria, HFE gene for hemochromatosis, TTR gene for transthyretin amyloidosis, PCSK9 gene, LDLR gene, apo (a) gene, and ANGPTL3 gene for familial hypercholesterolemia, LIPA gene for cholesteryl ester storage disease, ATP7B gene for Wilson's disease, ALAS1 gene for acute intermittent porphyria, GHR gene for acromegaly, UGT1A1 gene for Gilbert's syndrome, AGXT gene for primary hyperoxaluria, G6PC gene for glycogen storage disease type Ia, SLC26A1 gene for Hurler's syndrome:mucopolysaccharidosis (IDUA), GFAP gene for Alexander's disease, GRIA2 gene for amyotrophic lateral sclerosis, MECP2 gene for Rett syndrome, SCN1A gene for Dravet syndrome, SNCA gene and LRRK2 gene for Parkinson's disease, APP gene and MAPT gene for Alzheimer's disease, UBE3A gene for Angelman syndrome, PANK2 gene for pantothenic acid-related neurodegenerative disease, SMN1 gene for spinal muscular atrophy, EYS gene and RP1 gene for retinitis pigmentosa, ABCA4 gene for Stargardt's disease, USH2A gene for Usher's syndrome, CEP290 gene for Leber's congenital amaurosis, GNE gene for distal myopathy, GAA gene for Pompe's disease, CFTR gene and ENAC gene for cystic fibrosis, NPHS2 gene for focal segmental glomerulosclerosis, COL4A5 gene for Alport's syndrome, and SLCO2A1 gene for pachydermoperiostosis.

In a preferred embodiment, the base sequence of the first single-stranded polynucleotide can contain the mutation for editing so that the abnormal base sequence can recover the normal base sequence.

In an aspect, the present invention provides a pharmaceutical composition for treating or preventing a disease due to an abnormal base sequence contained in a target region present in an intracellular double-stranded DNA, the pharmaceutical composition containing composition I of the present invention (hereinafter, also referred to as pharmaceutical composition I of the present invention). Here, the base sequence of the first single-stranded polynucleotide in composition I of the present invention contains the mutation for editing so that the abnormal base sequence is repaired and thereby the disease is treated or prevented (e.g., so as to recover the normal base sequence, or give a base sequence differing from the normal base sequence but removed of the cause of the disease). With this configuration, the abnormal base sequence is repaired (e.g., recovers the normal base sequence) on the occurrence of base sequence editing with pharmaceutical composition I of the present invention, and the disease due to the abnormal base sequence is successfully treated or prevented.

Preferably, pharmaceutical composition I of the present invention contains the single-stranded polynucleotide(s) in a form that allows introduction into cells. The form that allows introduction into cells can be any form known in the art, and examples thereof include a dispersed form in an appropriate solvent (e.g., water), an encapsulated form in a hollow nanoparticle, a liposome, a lipid nanoparticle (LNP), or the like, and a complex with a cationic polymer.

Pharmaceutical composition I of the present invention is administered to a subject of, for example, a mammal, preferably of a human.

Any of oral administration and parenteral administration may be employed as a route of administration of pharmaceutical composition I of the present invention, and an appropriate route of administration for a target symptom or the like can be selected. Any of systemic administration and local administration may be employed as a route of administration. Examples of parenteral administration can include intravenous administration, intraarterial administration, intrathecal administration, intramuscular administration, intradermal administration, subcutaneous administration, intraperitoneal administration, transdermal administration, intraosseous administration, and intraarticular administration.

Pharmaceutical composition I of the present invention is administered in a therapeutically or prophylactically effective amount to a subject. The term "therapeutically or prophylactically effective amount" means an amount that allows a therapeutic or preventive effect to be exerted for a specific disease with a specific dosage form through a specific route of administration, and an appropriate therapeutically or prophylactically effective amount is determined in view of the species of a subject, the type of a disease, symptoms, sex, age, pre-existing diseases, and other factors.

An appropriate dose of pharmaceutical composition I of the present invention is determined in view of the species of a subject, the type of a disease, symptoms, sex, age, pre-existing diseases, and other factors.

Pharmaceutical composition I of the present invention can be used for treating or preventing any disease due to a mutation in a base sequence. Examples of such diseases include citrullinemia type I (ASS1), citrullinemia type II (SLC25A13), hemophilia (Factor V Leiden), α1-antitrypsin deficiency (SERPINA1), homocystinuria (CBS), phenylketonuria (PAH), hemochromatosis (HFE), transthyretin amyloidosis (TTR), familial hypercholesterolemia (PCSK9, LDLR, apo (a), ANGPTL3), cholesteryl ester storage disease (LIPA), Wilson's disease (ATP7B), acute intermittent porphyria (ALAS1), acromegaly (GHR), Gilbert's syndrome (UGT1A1), primary hyperoxaluria (AGXT), glycogen storage disease type Ia (G6PC), Hurler's syndrome:mucopolysaccharidosis (IDUA (SLC26A1)), Alexander's disease (GFAP), amyotrophic lateral sclerosis (GRIA2), Rett syndrome (MECP2), Dravet syndrome (SCN1A), Parkinson's disease (SNCA, LRRK2), Alzheimer's disease (APP, MAPT), Angelman syndrome (UBE3A), pantothenic acid-related neurodegenerative disease (PANK2), spinal muscular atrophy (SMN1), retinitis pigmentosa (EYS, RP1), Stargardt's disease (ABCA4), Usher's syndrome (USH2A), Leber's congenital amaurosis (CEP290), distal myopathy (GNE), Pompe's disease (GAA), cystic fibrosis (CFTR, ENAC), focal segmental glomerulosclerosis (NPHS2), Alport's syndrome (COL4A5), and pachydermoperiostosis (SLCO2A1). Shown in each pair of parentheses is the name of the corresponding target gene.

In an aspect, the present invention provides a method for treating or preventing a disease due to an abnormal base sequence contained in a target region present in an intracellular double-stranded DNA, the method including administering a therapeutically or prophylactically effective amount of composition I of the present invention to a subject. Here, the base sequence of the first single-stranded polynucleotide in composition I of the present invention contains the mutation for editing so that the abnormal base sequence is repaired and thereby the disease is treated or prevented (e.g., so as to recover the normal base sequence, or give a base sequence differing from the normal base sequence but removed of the cause of the disease). To the present invention, the same specific embodiments for administering pharmaceutical composition I of the present invention are applied.

In an aspect, the present invention provides a method for producing an isolated cell having a mutation introduced thereinto or an organism possessing a cell having a mutation introduced thereinto, the method including:
introducing composition I of the present invention into an isolated cell or a cell constituting a living body of an organism, thereby introducing a mutation into a base sequence in a target region present in an intracellular double-stranded DNA (hereinafter, also referred to as production method I of the present invention).

The cell in production method I of the present invention may be any of cells not constituting a living body (i.e., isolated cells) and cells being present in a living body and constituting the living body. In an embodiment, human cells can be excluded from the cells being present in a living body and constituting the living body. In another embodiment, introducing composition I of the present invention into cells by administering composition I of the present invention to a human for the purpose of treating a disease in the human can be included in production method I of the present invention. In the case that an isolated cell is targeted, production method I of the present invention can be performed *in vitro*. The cell may be any of eukaryotic cells and prokaryotic cells, and the origin of the cell is not limited to any particular biological species. The biological species as the origin of the cell may be any of an animal such as a mammal, a bird, and an insect, a plant, and a microorganism and the like, but is preferably an animal, and more preferably a mammal. Examples of the mammal include a human, a monkey, a bovine, a sheep, a goat, a horse, a pig, a rabbit, a dog, a cat, a rat, a mouse, and a guinea pig. The microorganism may be any of a prokaryotic microorganism and a eukaryotic microorganism; examples of the prokaryotic microorganism include a bacterium such as a Gram-positive bacterium and a Gram-negative bacterium, and examples of the eukaryotic microorganism include a fungus such as a filamentous fungus (mold) and a yeast.

The cell is not limited to any type, and examples include a somatic cell, a germ cell, a stem cell, and a cultured cell of any of them. Specific examples of somatic cells include living cells or cultured cells of them, the living cells separated from any of tissues and organs including: neural tissues such as the brain and spinal cord; sense organs such as retinal cells and olfactory cells; digestive organs such as the esophagus, stomach, small intestine and large intestine; respiratory organs such as the lungs and bronchi; reproductive organs such as the testes, ovaries, uterus, and placenta; urinary organs such as the kidneys and bladder; hematopoietic organs such as myelocytes and blood cells; muscle tissues such as skeletal muscles, smooth muscles, and cardiac muscles; bone tissues such as osteoblasts and osteoclasts; and skin tissues such as the skin and hair follicle cells. Specific examples of germ cells include ova, sperms, and cultured cells of them. Specific examples of stem cells include embryonic stem cells (ES cells), embryonic germ cells (EG cells), trophoblastic stem cells (TS cells), bone marrow stem cells, and neural stem cells.

A method known in the art can be used for introducing composition I of the present invention into a cell; applicable are, for example, an electroporation method, a calcium phosphate method, a lipofection method, and a microinjection method. As described for pharmaceutical composition I of the present invention, the first, second, and third single-stranded polynucleotides that have been made in a form that allows introduction into cells may be administered to a human or a non-human organism.

Once composition I of the present invention has been introduced into a cell, the same mutation that would have been obtained by homologous replacement of an editing target strand in a target region in an intracellular double-stranded DNA with the first single-stranded polynucleotide contained in composition I of the present invention is introduced into the editing target strand, the other strand is corrected to become complementary to the resultant, and as a result a mutation is introduced into each of the strands constituting the intracellular double-stranded DNA.

If an abnormal base sequence causative of a disease is contained in the target region, the disease due to the abnormal base sequence is successfully treated or prevented by introducing a mutation into the abnormal base sequence to repair (e.g., converting into the normal base sequence or converting into a base sequence differing from the normal base sequence but removed of the cause of the disease).

A cell having a mutation introduced into a target region and an organism individual possessing the cell are useful, for example, as a screening system or disease model whose sensitivity to a specific compound or the like is different from normal one.

3. Base sequence editing with composition containing single-stranded polynucleotide containing chemical modification

In an aspect, the present invention provides a composition containing a first single-stranded polynucleotide, wherein
the first single-stranded polynucleotide is 50 to 200 nucleotides in length, and has a base sequence having a sequence identity of 80% or more with a partial base sequence of an editing target strand in a target region present in an intracellular double-stranded DNA and containing at least one mutation for editing selected from the group consisting of deletion, substitution, and insertion of one or more nucleotides as compared with a base sequence of the editing target strand, and
the composition has at least one of the following features:
   (a) at least one nucleotide in a region consisting of the position of the mutation for editing and a 10-base sequence 5' adjacent to the position of the mutation for editing and a 10-base sequence 3' adjacent to the mutation for editing in the first single-stranded polynucleotide is a sugar-modified nucleotide; and
   (b) the first single-stranded polynucleotide contains at least one modified internucleotide bond, and the modified internucleotide bond is absent in a range from a third base 5' to the position of the mutation for editing to a third base 3' to the position of the mutation for editing (hereinafter, also referred to as composition II of the present invention).

Composition II of the present invention, introduced into a cell, allows base sequence editing corresponding to a mutation for editing contained in the first single-stranded polynucleotide for a target region present in an intracellular double-stranded DNA. That is, composition II of the present invention can be used for introducing a mutation into a target region present in an intracellular double-stranded DNA. In addition, base sequence editing with composition II of the present invention does not need any exogenous nuclease. Accordingly, in a preferred embodiment, composition II of the present invention contains neither an exogenous nuclease nor a polynucleotide capable of expressing it, and is not used in combination with any of them.

Here, "first single-stranded polynucleotide" is a name for convenience, and this term does not necessarily mean that a second single-stranded polynucleotide (A strand) or a third single-stranded polynucleotide (A' strand) is a constituent component of composition II of the present invention. Therefore, the term "first single-stranded polynucleotide" itself does not mean that a second single-stranded polynucleotide (A strand) or a third single-stranded polynucleotide (A' strand) is contained in composition II of the present invention. In using composition II of the present invention, base sequence editing may be performed only with the first single-stranded polynucleotide, or in combination with at least one of a second single-stranded polynucleotide (A strand) and a third single-stranded polynucleotide (A' strand). As described later, composition II of the present invention may contain at least one of a second single-stranded polynucleotide (A strand) and a third single-stranded polynucleotide (A' strand).

The first single-stranded polynucleotide in composition II of the present invention can have the same features as the first single-stranded polynucleotide in composition I of the present invention, except that having at least one of features (a) and (b) shown above is an essential feature. Features (a) and (b) are the same as features (a) and (b) for composition I of the present invention.

In the case of having feature (a), in the first single-stranded polynucleotide in an embodiment, at least one selected from the nucleotide at the position of the mutation for editing and the nucleotides at position 1, position 3, position 5, position 7, and position 9 located 5' to the mutation for editing and at least one selected from the nucleotides at position 1, position 3, position 5, position 7, and position 9 located 3' to the mutation for editing in the first single-stranded polynucleotide can be each a sugar-modified nucleotide; for example, the nucleotide at position 1 located 5' to the mutation for editing and the nucleotide at position 1 located 3' to the mutation for editing, the nucleotides at position 1 and position 3 located 5' to the mutation for editing and the nucleotides at position 1 and position 3 located 3' to the mutation for editing, the nucleotides at position 1, position 3, and position 5 located 5' to the mutation for editing and the nucleotides at position 1, position 3, and position 5 located 3' to the mutation for editing, the nucleotides at position 1, position 3, position 5, and position 7 located 5' to the mutation for editing and the nucleotides at position 1, position 3, position 5, and position 7 located 3' to the mutation for editing, or the nucleotides at position 1, position 3, position 5, position 7, and position 9 located 5' to the mutation for editing and the nucleotides at position 1, position 3, position 5, position 7, and position 9 located 3' to the mutation for editing can be each a sugar-modified nucleotide.

In a particular embodiment, in the first single-stranded polynucleotide,
the nucleotide at the position of the mutation for editing,
the nucleotide at position 1 located 5' to the position of the mutation for editing, the nucleotide at the position of the mutation for editing, and the nucleotide at position 1 located 3' to the position of the mutation for editing,
the nucleotide at position 1 located 5' to the position of the mutation for editing and the nucleotide at position 1 located 3' to the position of the mutation for editing,
the nucleotide at position 1 located 5' to the position of the mutation for editing, the nucleotide at the position of the mutation for editing, and the nucleotide at position 1 located 3' to the position of the mutation for editing,
the nucleotides at position 3 and position 1 located 5' to the position of the mutation for editing and the nucleotides at position 1 and position 3 located 3' to the position of the mutation for editing,
the nucleotides at position 5, position 3, and position 1 located 5' to the position of the mutation for editing and the nucleotides at position 1, position 3, and position 5 located 3' to the position of the mutation for editing,
the nucleotide at position 2 located 5' to the position of the mutation for editing and the nucleotide at position 2 located 3' to the position of the mutation for editing,
the nucleotides at position 2 and position 1 located 5' to the position of the mutation for editing and the nucleotides at position 1 and position 2 located 3' to the position of the mutation for editing,
the nucleotides at position 3 and position 2 located 5' to the position of the mutation for editing and the nucleotides at position 2 and position 3 located 3' to the position of the mutation for editing,
the nucleotides at position 3, position 2, and position 1 located 5' to the position of the mutation for editing and the nucleotides at position 1, position 2, and position 3 located 3' to the position of the mutation for editing,
the nucleotides at position 7, position 5, position 3, and position 1 located 5' to the position of the mutation for editing and the nucleotides at position 1, position 3, position 5, and position 7 located 3' to the position of the mutation for editing,
the nucleotides at position 9, position 7, position 5, position 3, and position 1 located 5' to the position of the mutation for editing and the nucleotides at position 1, position 3, position 5, position 7, and position 9 located 3' to the position of the mutation for editing,
any one of the nucleotides at position 1 to position 6 located 5' to the position of the mutation for editing,
any one of the nucleotides at position 1 to position 6 located 3' to the position of the mutation for editing,
the nucleotide at position 2 located 5' to the position of the mutation for editing and the nucleotide at position 2 located 3' to the position of the mutation for editing,
the nucleotide at position 3 located 5' to the position of the mutation for editing and the nucleotide at position 3 located 3' to the position of the mutation for editing,
the nucleotide at position 4 located 5' to the position of the mutation for editing and the nucleotide at position 4 located 3' to the position of the mutation for editing,
the nucleotide at position 5 located 5' to the position of the mutation for editing and the nucleotide at position 5 located 3' to the position of the mutation for editing,
the nucleotide at position 6 located 5' to the position of the mutation for editing and the nucleotide at position 6 located 3' to the position of the mutation for editing,
the nucleotides at position 5 and position 3 located 5' to the position of the mutation for editing,
the nucleotides at position 4 and position 2 located 5' to the position of the mutation for editing,
the nucleotides at position 3 and position 1 located 5' to the position of the mutation for editing,
the nucleotides at position 1 and position 3 located 3' to the position of the mutation for editing,
the nucleotides at position 2 and position 4 located 3' to the position of the mutation for editing,
the nucleotides at position 3 and position 5 located 3' to the position of the mutation for editing,
the nucleotide at position 7 located 5' to the position of the mutation for editing and the nucleotide at the position of the mutation for editing,
the nucleotide at position 6 located 5' to the position of the mutation for editing and the nucleotide at the position of the mutation for editing,
the nucleotide at position 5 located 5' to the position of the mutation for editing and the nucleotide at the position of the mutation for editing,
the nucleotide at position 4 located 5' to the position of the mutation for editing and the nucleotide at the position of the mutation for editing,
the nucleotide at position 3 located 5' to the position of the mutation for editing and the nucleotide at the position of the mutation for editing,
the nucleotide at position 2 located 5' to the position of the mutation for editing and the nucleotide at the position of the mutation for editing,
the nucleotide at position 1 located 5' to the position of the mutation for editing and the nucleotide at the position of the mutation for editing,
the nucleotide at the position of the mutation for editing and the nucleotide at position 1 located 3' to the position of the mutation for editing,
the nucleotide at the position of the mutation for editing and the nucleotide at position 2 located 3' to the position of the mutation for editing,
the nucleotide at the position of the mutation for editing and the nucleotide at position 3 located 3' to the position of the mutation for editing,
the nucleotide at the position of the mutation for editing and the nucleotide at position 4 located 3' to the position of the mutation for editing,
the nucleotide at the position of the mutation for editing and the nucleotide at position 5 located 3' to the position of the mutation for editing,
the nucleotide at the position of the mutation for editing and the nucleotide at position 6 located 3' to the position of the mutation for editing,
the nucleotide at the position of the mutation for editing and the nucleotide at position 7 located 3' to the position of the mutation for editing,
the nucleotide at position 1 located 5' to the position of the mutation for editing, the nucleotide at the position of the mutation for editing, and the nucleotide at position 1 located 3' to the position of the mutation for editing,
the nucleotide at position 2 located 5' to the position of the mutation for editing, the nucleotide at the position of the mutation for editing, and the nucleotide at position 2 located 3' to the position of the mutation for editing,
the nucleotide at position 3 located 5' to the position of the mutation for editing, the nucleotide at the position of the mutation for editing, and the nucleotide at position 3 located 3' to the position of the mutation for editing,
the nucleotide at position 4 located 5' to the position of the mutation for editing, the nucleotide at the position of the mutation for editing, and the nucleotide at position 4 located 3' to the position of the mutation for editing,
the nucleotide at position 5 located 5' to the position of the mutation for editing, the nucleotide at the position of the mutation for editing, and the nucleotide at position 5 located 3' to the position of the mutation for editing,
the nucleotide at position 6 located 5' to the position of the mutation for editing, the nucleotide at the position of the mutation for editing, and the nucleotide at position 6 located 3' to the position of the mutation for editing,
the nucleotide at position 7 located 5' to the position of the mutation for editing, the nucleotide at the position of the mutation for editing, and the nucleotide at position 7 located 3' to the position of the mutation for editing,
the nucleotides at position 2 and position 1 located 5' to the position of the mutation for editing and the nucleotides at position 1 and position 2 located 3' to the position of the mutation for editing,
the nucleotides at position 3 and position 2 located 5' to the position of the mutation for editing and the nucleotides at position 2 and position 3 located 3' to the position of the mutation for editing,
the nucleotides at position 3, position 2, and position 1 located 5' to the position of the mutation for editing and the nucleotides at position 1, position 2, and position 3 located 3' to the position of the mutation for editing,
the nucleotide at position 1 located 5' to the position of the mutation for editing and the nucleotides at position 1 and position 4 located 3' to the position of the mutation for editing,
the nucleotides at position 3 and position 1 located 5' to the position of the mutation for editing and the nucleotides at position 1 and position 3 located 3' to the position of the mutation for editing,
the nucleotides at position 3 and position 1 located 5' to the position of the mutation for editing and the nucleotides at position 1, position 3, and position 4 located 3' to the position of the mutation for editing,
the nucleotide at the position of the mutation for editing and the nucleotides at position 1 and position 4 located 3' to the position of the mutation for editing,
the nucleotide at position 1 located 5' to the position of the mutation for editing, the nucleotide at the position of the mutation for editing, and the nucleotide at position 4 located 3' to the position of the mutation for editing,
the nucleotide at the position of the mutation for editing and the nucleotides at position 2 and position 4 located 3' to the position of the mutation for editing, or
the nucleotide at position 2 located 5' to the position of the mutation for editing, the nucleotide at the position of the mutation for editing, and the nucleotide at position 4 located 3' to the position of the mutation for editing
can be each a sugar-modified nucleotide.

In a preferred embodiment, in the first single-stranded polynucleotide,
the nucleotide at the position of the mutation for editing,
the nucleotide at position 1 located 5' to the position of the mutation for editing, the nucleotide at the position of the mutation for editing, and the nucleotide at position 1 located 3' to the position of the mutation for editing,
the nucleotide at position 1 located 5' to the position of the mutation for editing and the nucleotide at position 1 located 3' to the position of the mutation for editing,
the nucleotide at position 1 located 5' to the position of the mutation for editing, the nucleotide at the position of the mutation for editing, and the nucleotide at position 1 located 3' to the position of the mutation for editing,
the nucleotides at position 3 and position 1 located 5' to the position of the mutation for editing and the nucleotides at position 1 and position 3 located 3' to the position of the mutation for editing,
the nucleotides at position 5, position 3, and position 1 located 5' to the position of the mutation for editing and the nucleotides at position 1, position 3, and position 5 located 3' to the position of the mutation for editing,
the nucleotide at position 3 located 5' to the position of the mutation for editing and the nucleotide at position 3 located 3' to the position of the mutation for editing,
the nucleotides at position 1 and position 3 located 3' to the position of the mutation for editing,
the nucleotide at position 2 located 5' to the position of the mutation for editing and the nucleotide at the position of the mutation for editing,
the nucleotide at position 1 located 5' to the position of the mutation for editing and the nucleotide at the position of the mutation for editing,
the nucleotide at the position of the mutation for editing and the nucleotide at position 1 located 3' to the position of the mutation for editing,
the nucleotide at the position of the mutation for editing and the nucleotide at position 2 located 3' to the position of the mutation for editing,
the nucleotide at the position of the mutation for editing and the nucleotide at position 3 located 3' to the position of the mutation for editing,
the nucleotide at the position of the mutation for editing and the nucleotide at position 4 located 3' to the position of the mutation for editing,
the nucleotide at the position of the mutation for editing and the nucleotide at position 5 located 3' to the position of the mutation for editing,
the nucleotide at the position of the mutation for editing and the nucleotide at position 6 located 3' to the position of the mutation for editing,
the nucleotide at the position of the mutation for editing and the nucleotide at position 7 located 3' to the position of the mutation for editing,
the nucleotide at position 2 located 5' to the position of the mutation for editing, the nucleotide at the position of the mutation for editing, and the nucleotide at position 2 located 3' to the position of the mutation for editing,
the nucleotide at position 3 located 5' to the position of the mutation for editing, the nucleotide at the position of the mutation for editing, and the nucleotide at position 3 located 3' to the position of the mutation for editing,
the nucleotide at position 4 located 5' to the position of the mutation for editing, the nucleotide at the position of the mutation for editing, and the nucleotide at position 4 located 3' to the position of the mutation for editing,
the nucleotide at position 5 located 5' to the position of the mutation for editing, the nucleotide at the position of the mutation for editing, and the nucleotide at position 5 located 3' to the position of the mutation for editing,
the nucleotide at position 1 located 5' to the position of the mutation for editing and the nucleotides at position 1 and position 4 located 3' to the position of the mutation for editing,
the nucleotides at position 3 and position 1 located 5' to the position of the mutation for editing and the nucleotides at position 1, position 3, and position 4 located 3' to the position of the mutation for editing,
the nucleotide at the position of the mutation for editing and the nucleotides at position 1 and position 4 located 3' to the position of the mutation for editing,
the nucleotide at position 1 located 5' to the position of the mutation for editing, the nucleotide at the position of the mutation for editing, and the nucleotide at position 4 located 3' to the position of the mutation for editing,
the nucleotide at the position of the mutation for editing and the nucleotides at position 2 and position 4 located 3' to the position of the mutation for editing, or
the nucleotide at position 2 located 5' to the position of the mutation for editing, the nucleotide at the position of the mutation for editing, and the nucleotide at position 4 located 3' to the position of the mutation for editing
can be each a sugar-modified nucleotide.

In a preferred embodiment, each sugar-modified nucleotide can be a nucleotide containing a 4'-lower linear alkylene-O-2' cross-linkage in the ribose ring, more preferably a nucleotide containing a 2'-O,4'-C-methylene cross-linkage or a 2'-O,4'-C-ethylene cross-linkage in the ribose ring, and further preferably an LNA or an ENA.

In the case of having feature (b), in an embodiment, the first single-stranded polynucleotide can contain one to four (e.g., one, two, three, or four) modified internucleotide bonds near the 5' end (e.g., within 20 bases, within 15 bases, within 13 bases, within 10 bases, within 8 bases, within 6 bases, within 5 bases, within 4 bases, within 3 bases, or within 2 bases from the 5' end) and/or near the 3' end (e.g., within 20 bases, within 15 bases, within 13 bases, within 10 bases, within 8 bases, within 6 bases, within 5 bases, within 4 bases, within 3 bases, or within 2 bases from the 3' end). In a preferred embodiment, the first single-stranded polynucleotide can contain one to four modified internucleotide bonds within 13 bases (more preferably within five bases) from the 3' end. In a more preferred embodiment, the first single-stranded polynucleotide can contain two modified internucleotide bonds within three bases from the 3' end or one modified internucleotide bond within two bases from the 3' end. In a preferred embodiment, each modified internucleotide bond can be a phosphorothioate bond, an alkylphosphonate bond, or a phosphotriester bond. In a more preferred embodiment, the first single-stranded polynucleotide can contain one to four modified internucleotide bonds within 13 bases (preferably two modified internucleotide bonds within three bases or one modified internucleotide bond within two bases) from the 3' end, and each modified internucleotide bond can be a phosphorothioate bond.

In a preferred embodiment,
the position of the mutation for editing in the first single-stranded polynucleotide is present within a range of 49 to 51% from the 5' end, with the full length from the 5' end to the 3' end defined as 100%, the base sequence of the first single-stranded polynucleotide has a sequence identity of 98% or more with the E-strand-correspondent sequence, and the length of the first single-stranded polynucleotide is 70 to 100 nucleotides in length,
in the first single-stranded polynucleotide,

the nucleotide at the position of the mutation for editing,
the nucleotide at position 1 located 5' to the position of the mutation for editing, the nucleotide at the position of the mutation for editing, and the nucleotide at position 1 located 3' to the position of the mutation for editing,
the nucleotide at position 1 located 5' to the position of the mutation for editing and the nucleotide at position 1 located 3' to the position of the mutation for editing,
the nucleotide at position 1 located 5' to the position of the mutation for editing, the nucleotide at the position of the mutation for editing, and the nucleotide at position 1 located 3' to the position of the mutation for editing,
the nucleotides at position 3 and position 1 located 5' to the position of the mutation for editing and the nucleotides at position 1 and position 3 located 3' to the position of the mutation for editing,
the nucleotides at position 5, position 3, and position 1 located 5' to the position of the mutation for editing and the nucleotides at position 1, position 3, and position 5 located 3' to the position of the mutation for editing,
the nucleotide at position 3 located 5' to the position of the mutation for editing and the nucleotide at position 3 located 3' to the position of the mutation for editing,
the nucleotides at position 1 and position 3 located 3' to the position of the mutation for editing,
the nucleotide at position 2 located 5' to the position of the mutation for editing and the nucleotide at the position of the mutation for editing,
the nucleotide at position 1 located 5' to the position of the mutation for editing and the nucleotide at the position of the mutation for editing,
the nucleotide at the position of the mutation for editing and the nucleotide at position 1 located 3' to the position of the mutation for editing,
the nucleotide at the position of the mutation for editing and the nucleotide at position 2 located 3' to the position of the mutation for editing,
the nucleotide at the position of the mutation for editing and the nucleotide at position 3 located 3' to the position of the mutation for editing,
the nucleotide at the position of the mutation for editing and the nucleotide at position 4 located 3' to the position of the mutation for editing,
the nucleotide at the position of the mutation for editing and the nucleotide at position 5 located 3' to the position of the mutation for editing,
the nucleotide at the position of the mutation for editing and the nucleotide at position 6 located 3' to the position of the mutation for editing,
the nucleotide at the position of the mutation for editing and the nucleotide at position 7 located 3' to the position of the mutation for editing,
the nucleotide at position 2 located 5' to the position of the mutation for editing, the nucleotide at the position of the mutation for editing, and the nucleotide at position 2 located 3' to the position of the mutation for editing,
the nucleotide at position 3 located 5' to the position of the mutation for editing, the nucleotide at the position of the mutation for editing, and the nucleotide at position 3 located 3' to the position of the mutation for editing,
the nucleotide at position 4 located 5' to the position of the mutation for editing, the nucleotide at the position of the mutation for editing, and the nucleotide at position 4 located 3' to the position of the mutation for editing,
the nucleotide at position 5 located 5' to the position of the mutation for editing, the nucleotide at the position of the mutation for editing, and the nucleotide at position 5 located 3' to the position of the mutation for editing,
the nucleotide at position 1 located 5' to the position of the mutation for editing and the nucleotides at position 1 and position 4 located 3' to the position of the mutation for editing,
the nucleotides at position 3 and position 1 located 5' to the position of the mutation for editing and the nucleotides at position 1, position 3, and position 4 located 3' to the position of the mutation for editing,
the nucleotide at the position of the mutation for editing and the nucleotides at position 1 and position 4 located 3' to the position of the mutation for editing,
the nucleotide at position 1 located 5' to the position of the mutation for editing, the nucleotide at the position of the mutation for editing, and the nucleotide at position 4 located 3' to the position of the mutation for editing,
the nucleotide at the position of the mutation for editing and the nucleotides at position 2 and position 4 located 3' to the position of the mutation for editing, or
the nucleotide at position 2 located 5' to the position of the mutation for editing, the nucleotide at the position of the mutation for editing, and the nucleotide at position 4 located 3' to the position of the mutation for editing
are each a sugar-modified nucleotide,
   the sugar-modified nucleotide is a nucleotide containing a 4'-lower linear alkylene-O-2' cross-linkage in the ribose ring, more preferably a nucleotide containing a 2'-O,4'-C-methylene cross-linkage or a 2'-O,4'-C-ethylene cross-linkage in the ribose ring, and further preferably an LNA or an ENA,
   the first single-stranded polynucleotide contains one to four modified internucleotide bonds within five bases from the 3' end, and more preferably contains two modified internucleotide bonds within three bases from the 3' end or one modified internucleotide bond within two bases from the 3' end, and
   each modified internucleotide bond is a phosphorothioate bond, an alkylphosphonate bond, or a phosphotriester bond.

In an embodiment, composition II of the present invention can further contain a second single-stranded polynucleotide. The second single-stranded polynucleotide in composition II of the present invention can have the same features as the second single-stranded polynucleotide in composition I of the present invention has.

In the case that the second single-stranded polynucleotide contains at least one modified internucleotide bond, in a preferred embodiment, the second single-stranded polynucleotide contains one or two modified internucleotide bonds within three bases from the 5' end and/or 3' end or one modified internucleotide bond within two bases from the 5' end and/or 3' end, and can more preferably contain one or two modified internucleotide bonds within three bases from the 3' end or one modified internucleotide bond within two bases from the 3' end. In a preferred embodiment, each modified internucleotide bond can be a phosphorothioate bond, an alkylphosphonate bond, or a phosphotriester bond, more preferably an alkylphosphonate bond or a phosphotriester bond, and further preferably a methylphosphonate bond or an ethylphosphotriester bond.

In an embodiment, composition II of the present invention can further contain a third single-stranded polynucleotide. The third single-stranded polynucleotide in composition II of the present invention can have the same features as the third single-stranded polynucleotide in composition I of the present invention has.

In an embodiment, an abnormal base sequence causative of a disease is contained in the target region, and the base sequence of the first single-stranded polynucleotide can contain the mutation for editing so that the abnormal base sequence is repaired and thereby the disease is treated or prevented (e.g., so as to recover the normal base sequence, or give a base sequence differing from the normal base sequence but removed of the cause of the disease).

In an aspect, the present invention provides a pharmaceutical composition for treating or preventing a disease due to an abnormal base sequence contained in a target region present in an intracellular double-stranded DNA, the pharmaceutical composition containing composition II of the present invention (hereinafter, also referred to as pharmaceutical composition II of the present invention). Here, the base sequence of the first single-stranded polynucleotide in composition II of the present invention contains the mutation for editing so that the abnormal base sequence is repaired and thereby the disease is treated or prevented (e.g., so as to recover the normal base sequence, or give a base sequence differing from the normal base sequence but removed of the cause of the disease). With this configuration, the abnormal base sequence is repaired (e.g., recovers the normal base sequence or give a base sequence differing from the normal base sequence but removed of the cause of the disease) on the occurrence of base sequence editing with pharmaceutical composition II of the present invention, and the disease due to the abnormal base sequence is successfully treated or prevented. To pharmaceutical composition II of the present invention, the same specific embodiments for pharmaceutical composition I of the present invention are applied.

In an aspect, the present invention provides a method for treating or preventing a disease due to an abnormal base sequence contained in a target region present in an intracellular double-stranded DNA, the method including administering a therapeutically or prophylactically effective amount of composition II of the present invention to a subject. Here, the base sequence of the first single-stranded polynucleotide in composition II of the present invention contains the mutation for editing so that the abnormal base sequence is repaired and thereby the disease is treated or prevented (e.g., so as to recover the normal base sequence, or give a base sequence differing from the normal base sequence but removed of the cause of the disease). To the present invention, the same specific embodiments for administering pharmaceutical composition II of the present invention are applied.

In an aspect, the present invention provides a method for producing an isolated cell having a mutation introduced thereinto or an organism possessing a cell having a mutation introduced thereinto, the method including:
introducing composition II of the present invention into an isolated cell or a cell constituting a living body of an organism, thereby introducing a mutation into a base sequence in a target region present in an intracellular double-stranded DNA (hereinafter, also referred to as production method II of the present invention). To production method II of the present invention, the specific embodiments the same as those of production method I of the present invention are applied, except that composition II of the present invention is used in place of composition I of the present invention.

### 4. Pharmaceutical composition containing A' strand for use in combination with composition containing E strand or TD

In an aspect, the present invention provides a pharmaceutical composition containing a third single-stranded polynucleotide for use in combination with a composition containing a first single-stranded polynucleotide, wherein
the first single-stranded polynucleotide is 50 to 200 nucleotides in length, and has a base sequence having a sequence identity of 80% or more with a partial base sequence of an editing target strand in a target region present in an intracellular double-stranded DNA and containing at least one mutation for editing selected from the group consisting of deletion, substitution, and insertion of one or more nucleotides as compared with a base sequence of the editing target strand,
the third single-stranded polynucleotide is 15 to 200 nucleotides in length, and has a base sequence having a sequence identity of 90% or more with a base sequence in the editing target strand 5' or 3' to a position corresponding to the mutation for editing in the first single-stranded polynucleotide, wherein, in the editing target strand, the base sequence having sequence identity with the first single-stranded polynucleotide and the base sequence having sequence identity with the third single-stranded polynucleotide are overlapping, adjacent, or separated by one to nine bases, and
an abnormal base sequence causative of a disease is contained in the target region, and the base sequence of the first single-stranded polynucleotide contains the mutation for editing so that the abnormal base sequence is repaired and thereby the disease is treated or prevented (e.g., so as to recover the normal base sequence, or give a base sequence differing from the normal base sequence but removed of the cause of the disease) (hereinafter, also referred to as pharmaceutical composition III of the present invention).

Pharmaceutical composition III of the present invention, introduced into a cell with use of a composition containing a first single-stranded polynucleotide in combination, causes base sequence editing, and the abnormal base sequence is successfully repaired (e.g., converted into the normal base sequence or converted into a base sequence differing from the normal base sequence but removed of the cause of the disease). Accordingly, pharmaceutical composition III of the present invention can be a pharmaceutical composition for treating or preventing a disease due to an abnormal base sequence contained in a target region present in an intracellular double-stranded DNA. Examples of the disease include those shown for pharmaceutical composition I of the present invention.

Base sequence editing by using pharmaceutical composition III of the present invention and a composition containing a first single-stranded polynucleotide in combination does not need any exogenous nuclease. Accordingly, in a preferred embodiment, pharmaceutical composition III of the present invention contains neither an exogenous nuclease nor a polynucleotide capable of expressing it, and is not used in combination with any of them.

The third single-stranded polynucleotide in pharmaceutical composition III of the present invention can have the same features as the third single-stranded polynucleotide in composition I of the present invention has.

Here, "third single-stranded polynucleotide" is a name for convenience, and this term does not necessarily mean that a first single-stranded polynucleotide (E strand) or a second single-stranded polynucleotide (A strand) is a constituent component of pharmaceutical composition III of the present invention.

The first single-stranded polynucleotide in the composition to be used in combination with pharmaceutical composition III of the present invention can have the same features as the first single-stranded polynucleotide in composition I of the present invention has. The composition containing a single-stranded polynucleotide of the first single-stranded polynucleotide can correspond to the embodiment of pharmaceutical composition II of the present invention containing a first single-stranded polynucleotide but containing neither a second nor a third single-stranded polynucleotide.

In an embodiment, the composition containing the first single-stranded polynucleotide to be used in combination with pharmaceutical composition III of the present invention can further contain a second single-stranded polynucleotide. Here, the second single-stranded polynucleotide can have the same features as the second single-stranded polynucleotide in composition I of the present invention has. The composition containing single-stranded polynucleotides of the first and second single-stranded polynucleotides can correspond to the embodiment of pharmaceutical composition II of the present invention containing first and second single-stranded polynucleotides but containing no third single-stranded polynucleotide.

Herein, the expressions "use in combination" and "using in combination" mean administering two or more drugs or pharmaceutical compositions to the same individual. These drugs or pharmaceutical compositions may be administered simultaneously or almost simultaneously (e.g., within 1 hour), or separately at intervals (e.g., at intervals of several hours) or consecutively. Pharmaceutical composition III of the present invention may be administered together with the composition containing the first single-stranded polynucleotide simultaneously or almost simultaneously, or at an interval. Pharmaceutical composition III of the present invention and the composition containing the first single-stranded polynucleotide can be administered in any order. In a preferred embodiment, pharmaceutical composition III of the present invention and the composition containing the first single-stranded polynucleotide can be administered simultaneously.

Preferably, pharmaceutical composition III of the present invention contains the third single-stranded polynucleotide in a form that allows introduction into cells. The form that allows introduction into cells can be any form known in the art, and examples thereof include a dispersed form in an appropriate solvent (e.g., water), an encapsulated form in a hollow nanoparticle, a liposome, a lipid nanoparticle (LNP), or the like, and a complex with a cationic polymer.

Pharmaceutical composition III of the present invention is administered to a subject of, for example, a mammal, preferably of a human.

Any of oral administration and parenteral administration may be employed as a route of administration of pharmaceutical composition III of the present invention, and an appropriate route of administration for a target symptom or the like can be selected. Any of systemic administration and local administration may be employed as a route of administration. Examples of parenteral administration can include intravenous administration, intraarterial administration, intrathecal administration, intramuscular administration, intradermal administration, subcutaneous administration, intraperitoneal administration, transdermal administration, intraosseous administration, and intraarticular administration.

Pharmaceutical composition III of the present invention is administered to a subject in a therapeutically or prophylactically effective amount. The term "therapeutically or prophylactically effective amount" means an amount that allows a therapeutic or preventive effect to be exerted for a specific disease with a specific dosage form through a specific route of administration, and an appropriate therapeutically or prophylactically effective amount is determined in view of the species of a subject, the type of a disease, symptoms, sex, age, pre-existing diseases, and other factors.

An appropriate dose of pharmaceutical composition III of the present invention is determined in view of the species of a subject, the type of a disease, symptoms, sex, age, pre-existing diseases, and other factors.

The ratio between the doses of pharmaceutical composition III of the present invention and the composition containing the first single-stranded polynucleotide is not limited as long as enhanced editing efficiency is successfully obtained with the composition containing the first single-stranded polynucleotide.

In an embodiment, pharmaceutical composition III of the present invention can be used in such a manner that the dose (moles) of the second single-stranded polynucleotide is 0.2 to 2, preferably 0.2 to 1, and more preferably 0.2 to 0.5 as the total of the doses of the first and third single-stranded polynucleotides is taken as 1. Otherwise, pharmaceutical composition III of the present invention is preferably used in such a manner that the dose of the second single-stranded polynucleotide is equal to or lower than the total of the doses of the first and third single-stranded polynucleotides.

In an embodiment, pharmaceutical composition III of the present invention can be used in such a manner that the dose (moles) of the third single-stranded polynucleotide is 0.25 to 4, and preferably 1 to 4 as the dose of the first single-stranded polynucleotide is taken as 1. Otherwise, pharmaceutical composition III of the present invention is preferably used in such a manner that the dose of the third single-stranded polynucleotide is equal to or higher than the dose of the first single-stranded polynucleotide.

In an embodiment, pharmaceutical composition III of the present invention can be used in such a manner that the dose (moles) of the third single-stranded polynucleotide is 0.25 to 4, and preferably 1 to 4 as the dose of the second single-stranded polynucleotide is taken as 1. Otherwise, pharmaceutical composition III of the present invention is preferably used in such a manner that the dose of the third single-stranded polynucleotide is equal to or higher than the dose of the second single-stranded polynucleotide.

In an aspect, the present invention provides a method for treating or preventing a disease due to an abnormal base sequence contained in a target region present in an intracellular double-stranded DNA, the method including administering a therapeutically or prophylactically effective amount of composition III of the present invention to a subject together with the composition containing the first single-stranded polynucleotide simultaneously, separately, or consecutively. To the present invention, the same specific embodiments for administering pharmaceutical composition III of the present invention are applied.

In an aspect, the present invention provides a method for producing an isolated cell having a mutation introduced thereinto or an organism possessing a cell having a mutation introduced thereinto, the method including:
introducing pharmaceutical composition III of the present invention into an isolated cell or a cell constituting a living body of an organism together with the composition containing the first single-stranded polynucleotide simultaneously, separately, or consecutively, thereby introducing a mutation into a base sequence in a target region present in an intracellular double-stranded DNA (hereinafter, also referred to as production method III of the present invention). To production method III of the present invention, the specific embodiments the same as those of production method I of the present invention are applied, except that the third single-stranded polynucleotide and the first polynucleotide are contained separately in different compositions, and they are introduced into a cell simultaneously, separately, or consecutively.

Pharmaceutical composition III of the present invention and the composition containing the first single-stranded polynucleotide may be introduced into a cell separately or consecutively, but are preferably introduced simultaneously.

The following describes the present invention in more detail by showing examples and test examples, but the scope of the present invention is not limited by them. In Examples, "n-" is used to indicate that heat treatment and annealing treatment were not performed in preparing the nucleic acid for editing. In Examples, "n-E" indicates that an E strand was introduced into a cell, "n-TD" indicates that a TD (E strand and A strand) was introduced into a cell, and "n-TD'" indicates that a TD (E strand and A strand) and an A' strand were introduced into a cell.

### EXAMPLES

### Example 1: Enhancement of base sequence editing efficiency by addition of A' strand

Examination was performed by means of a green fluorescent protein on change in editing efficiency by addition of an A' strand in a base sequence editing method with a TD.

copGFP gene, which encodes copepod-derived green fluorescent protein, was used as a target gene. A plasmid DNA having a mutated copGFP gene (SEQ ID NO: 1) obtained by changing the base sequence TAC corresponding to Tyr in Gly-Tyr-Gly constituting the fluorophore to CAC (encoding His) and a TD were co-introduced into human U2OS cells. The TD had a sequence identical to a part of the antisense strand of the target gene, and consisted of: an E strand (SEQ ID NO: 5) having a length of 79 nucleotides with the sequence encoding the fluorophore being wild type; and an A strand (SEQ ID NO: 6) hybridizable with the region of 35 nucleotides from the 3' end of the E strand. Once base sequence editing has been caused by the TD, the mutated copGFP gene in the plasmid DNA becomes the wild-type copGFP gene, allowing green fluorescence to be observed. A' strands of various lengths were introduced together with the target plasmid and the TD, and the proportions of cells for which green fluorescence was observed through a fluorescence microscope were examined.

E strand
copGFP-153-79b-AS (AS_E strand): SEQ ID NO: 5

5'-
CGTAGCCGCTGGGGTAGGTGCCGAAGTGGTAGAAGCCGTAGCCCATCACGTGGC
TCAGCAGGTAGGGGCTGAAGGTCAG -3'

A strand
copGFP-153-79b-AS-35TD3'-ODN (A strand for AS_E strand): SEQ ID NO: 6 5'-CTGACCTTCAGCCCCTACCTGCTGAGCCACGTGAT -3'

A' strand
copGFP-153-79b-AS-65TDdown-ODN (alias: copGFP_A_prime65) (A' strand for AS_E strand): SEQ ID NO: 7

5'-
CTCGTACTTCTCGATGCGGGTGTTGGTGTAGCCGCCGTTGTTGATGGCGTGCAGG
AAGGGGTTCT -3'

The specific experimental method is as follows.

At an intended ratio, 1 µM aqueous solutions of the E strand, the A strand, and the A' strand were mixed together, and the resultant was left to stand at room temperature for 1 hour without heat treatment to prepare a nucleic acid for editing. U2OS cells (8 × 10³ cells; obtained from American Type Culture Collection (Manassas, VA, USA)) were seeded in each well of a 384-well plate and cultured overnight, and an mPlum-T2A-copGFP (Y/H) expression vector plasmid (30.0 ng; H. Kawai, R. Kamitsubo, H. Kamiya. Correction of monomeric enhanced green fluorescent protein (mEGFP) gene by short 5'-tailed duplexes. J. Biosci. Bioengng. 134, 175-181 (2022)) containing the mutated copGFP gene for editing efficiency analysis and the nucleic acid for editing (E strand alone, E strand + A strand, or E strand + A strand + A' strand) were co-introduced by using Lipofectamine 3000. After 4 hours, medium exchange was performed. After culturing for 48 hours, nuclear staining was performed with Hoechst 33342, and the resultant was fixed with 4% paraformaldehyde (100 µL/well), and observed through a fluorescence microscope.

Editing efficiency was calculated by the following method.

Fluorescence microscope images were acquired with an Opera Phenix (R) high-throughput/high-contents imaging system (PerkinElmer, Inc.). With use of the image analysis software Harmony (PerkinElmer, Inc.), fluorescence intensity data were acquired for Hoechst 33342, mPlum, and copGFP in the cells. For every experiment, average fluorescence intensity and only definitely shaped cell nuclei were extracted from fluorescence intensity data for Hoechst 33342 in the 384-well plate, and subjected to analysis on editing efficiency. For each of fluorescence brightnesses at fluorescence wavelengths of mPlum and copGFP in the cell nuclei, median/cumulative total of values at the pixels was calculated. A calibration curve was prepared from the vector abundance ratios and the fluorescence intensities of copGFP in samples produced by mixing an mPlum-T2A-copGFP (normal) expression vector plasmid and an mPlum-T2A-copGFP (Y/H) expression vector plasmid at specific ratios, and with use of the calibration curve the editing efficiency was calculated from the cumulative total of fluorescence intensities calculated for the wells.

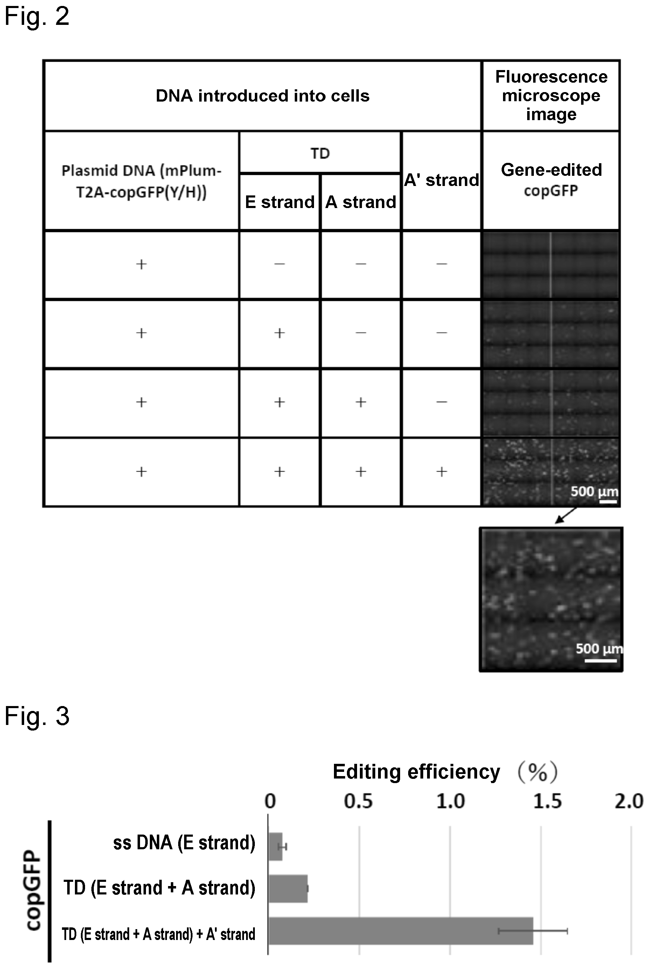

The results showed that more cells having the fluorescence of copGFP were observed for the case with introduction of the E strand, the A strand, and the A' strand than for the case with introduction only of the E strand and the A strand, indicating enhanced editing efficiency. In particular, the combination of the E strand and A strand used in the present example gave substantially enhanced editing efficiency when co-introduced with the A' strand of 65 nucleotides (SEQ ID NO: 7; in the antisense strand of the target gene, the base sequence having sequence identity with the E strand and the base sequence having sequence identity with the A' strand are adjacent (i.e., the interval between the two is 0 base)) (Figs. 2 and 3).

Likewise, use of the E strand alone instead of the TD was confirmed to give an effect of giving enhanced editing efficiency by addition of the A' strand.

### Example 2: Enhancement of base sequence editing efficiency by addition of A' strand

With the target gene changed to mEGFP gene, the effect of giving enhanced editing efficiency by addition of an A' strand was confirmed in the same manner as in Example 1.

mEGFP is a green fluorescent protein, and the amino acid of mEGFP has a homology only of 27% to that of copGFP, and the DNA sequence encoding mEGFP has almost no homology to that encoding copGFP. A TD (E strand and A strand) or a TD + A' strand were co-introduced into U2OS cells together with a plasmid having a mutated gene (SEQ ID NO: 3) obtained by changing the base sequence TAC corresponding to Tyr in Thr-Tyr-Gly in the fluorophore of mEGFP to CAC (mPlum-T2A-mEGFP (Y/H) expression vector plasmid; H. Kawai, R. Kamitsubo, H. Kamiya. Correction of monomeric enhanced green fluorescent protein (mEGFP) gene by short 5'-tailed duplexes. J. Biosci. Bioengng. 134, 175-181 (2022)). Fluorescence microscopy was performed in the same manner as in Example 1.

The nucleic acids for editing that were used are shown in the following.

E strand
mEGFP-79_AS_E: SEQ ID NO: 8

5'-
TCATGTGGTCGGGGTAGCGGCTGAAGCACTGCACGCCGTAGGTCAGGGTGGTCA
CGAGGGTGGGCCAGGGCACGGGCAG -3'

A strand
mEGFP-35_S_A: SEQ ID NO: 9
5'-CTGCCCGTGCCCTGGCCCACCCTCGTGACCACCCT -3'

A' strand
mEGFP-65_AS_Ap: SEQ ID NO: 10

5'-
GAAGAAGATGGTGCGCTCCTGGACGTAGCCTTCGGGCATGGCGGACTTGAAGAA
GTCGTGCTGCT -3'

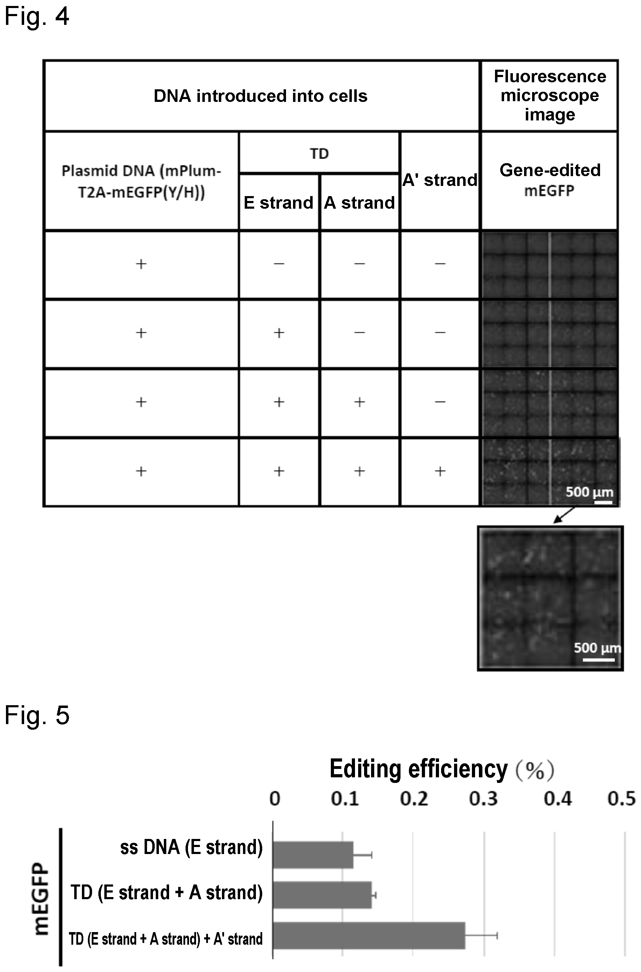

The results showed that, also in the experiment with mEGFP gene, more cells having green fluorescence were observed for the case with addition of the A' strand than for the case only with E strand + A strand, indicating remarkably enhanced editing efficiency (Figs. 4 and 5).

### Example 3: Enhancement of base sequence editing efficiency by chemical modification in E strand or A strand

The influence of a chemical modification in an E strand and an A strand on editing efficiency was analyzed with use of the analysis method for copGFP gene as a target gene (Example 1). In this example, the A' strand in an n-TD' is copGFP_A_prime65 as in Example 1, unless otherwise specified.

### (3-1) Influence of phosphorothioate (PS) modification on base sequence editing efficiency - Part 1

For each of E strands, A strands, and A' strands, a PS modification was introduced at one or two positions in the 5'-end portion or the 3'-end portion, and examination was performed on base sequence editing efficiency in the same manner as in Example 1. The nucleic acids for editing that were used (only those with a modification) are shown in the following (having a PS modification in the 3' phosphate moiety of each underlined part).

E strand
copGFP-79_AS_E 5'3'S: SEQ ID NO: 11 
5'-

CGTAGCCGCTGGGGTAGGTGCCGAAGTGGTAGAAGCCGTAGCCCATCACGTGGC

TCAGCAGGTAGGGGCTGAAGGTCAG -3'
copGFP-79_AS_E 5'S: SEQ ID NO: 12 5'- 
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E 3'S: SEQ ID NO: 13 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E 5'3'SS: SEQ ID NO: 14 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E 5'SS: SEQ ID NO: 15 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP -79_AS_E 3'SS: SEQ ID NO: 16

5'-
CGTAGCCGCTGGGGTAGGTGCCGAAGTGGTAGAAGCCGTAGCCCATCACGTGGC
TCAGCAGGTAGGGGCTGAAGGTCAG -3'

A strand
copGFP-35_S_A 5'3'S: SEQ ID NO: 17
   5'-CTGACCTTCAGCCCCTACCTGCTGAGCCACGTGAT -3'
copGFP-35_S_A 5'S: SEQ ID NO: 18
   5'-CTGACCTTCAGCCCCTACCTGCTGAGCCACGTGAT -3'
copGFP-35_S_A 3'S: SEQ ID NO: 19
   5'- CTGACCTTCAGCCCCTACCTGCTGAGCCACGTGAT -3'
copGFP-35_S_A 5'3'SS: SEQ ID NO: 20
   5'- CTGACCTTCAGCCCCTACCTGCTGAGCCACGTGAT -3'
copGFP-35_S_A 5'SS: SEQ ID NO: 21
   5'- CTGACCTTCAGCCCCTACCTGCTGAGCCACGTGAT -3'
copGFP-35_S_A 3'SS: SEQ ID NO: 22
   5'- CTGACCTTCAGCCCCTACCTGCTGAGCCACGTGAT -3'

A' strand
copGFP-79_AS_Ap 5'3'S: SEQ ID NO: 23 
5'-

CTCGTACTTCTCGATGCGGGTGTTGGTGTAGCCGCCGTTGTTGATGGCGTGCAGG

AAGGGGTTCT -3'
copGFP-79_AS_Ap 5'S: SEQ ID NO: 24 
5'-
CTCGTACTTCTCGATGCGGGTGTTGGTGTAGCCGCCGTTGTTGATGGCGTGCAGG
AAGGGGTTCT -3'
copGFP-79_AS_Ap 3'S: SEQ ID NO: 25 
5'-
CTCGTACTTCTCGATGCGGGTGTTGGTGTAGCCGCCGTTGTTGATGGCGTGCAGG
AAGGGGTTCT -3'
copGFP-79_AS_Ap 5'3'SS: SEQ ID NO: 26 
5'-
CTCGTACTTCTCGATGCGGGTGTTGGTGTAGCCGCCGTTGTTGATGGCGTGCAGG
AAGGGGTTCT -3'
copGFP-79_AS_Ap 5'SS: SEQ ID NO: 27 
5'-
CTCGTACTTCTCGATGCGGGTGTTGGTGTAGCCGCCGTTGTTGATGGCGTGCAGG
AAGGGGTTCT -3'
copGFP-79_AS_Ap 3'SS: SEQ ID NO: 28

5'-
CTCGTACTTCTCGATGCGGGTGTTGGTGTAGCCGCCGTTGTTGATGGCGTGCAGG
AAGGGGTTCT -3'

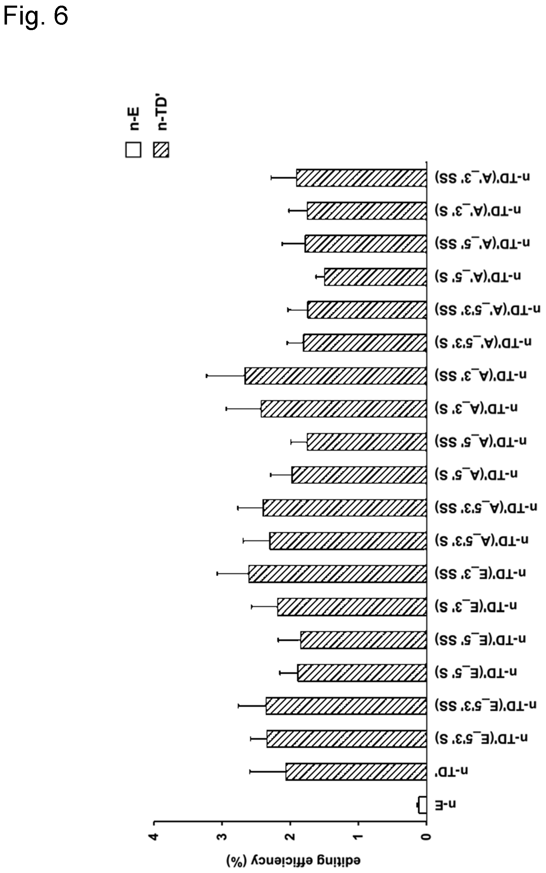

Fig. 6 shows the results. For any of the E strands and the A strands, introduction of a PS modification in the 3'-end portion of the strand gave enhanced editing efficiency. Although the A' strands having a PS modification introduced in any of the 5'-end portion and the 3'-end portion were not found to give enhanced editing efficiency in the present experimental system, an internucleotide modification in an A' strand may be useful for imparting nuclease resistance in introduction into cells.

### (3-2) Influence of PS modification on base sequence editing efficiency - Part 2

For each of E strands and A strands, a PS modification was introduced at one to four positions in the 3'-end portion, and examination was performed on base sequence editing efficiency in the same manner as in Example 1. The nucleic acids for editing that were used (only those with a modification) are shown in the following (having a PS modification in the 3' phosphate moiety of each underlined part).

E strand
copGFP-79_AS_E 3'S: SEQ ID NO: 13
copGFP -79_AS_E 3'SS: SEQ ID NO: 16
copGFP-79_AS_E 3'SSS: SEQ ID NO: 29 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E 3'SSSS: SEQ ID NO: 30

5'-
CGTAGCCGCTGGGGTAGGTGCCGAAGTGGTAGAAGCCGTAGCCCATCACGTGGC
TCAGCAGGTAGGGGCTGAAGGTCAG -3'

A strand
copGFP-35_S_A 3'S: SEQ ID NO: 19
copGFP-35_S_A 3'SS: SEQ ID NO: 22
copGFP-35_S_A 3'SSS: SEQ ID NO: 31
   5'- CTGACCTTCAGCCCCTACCTGCTGAGCCACGTGAT -3'
copGFP-35_S_A 3'SSSS: SEQ ID NO: 32
   5'- CTGACCTTCAGCCCCTACCTGCTGAGCCACGTGAT -3'

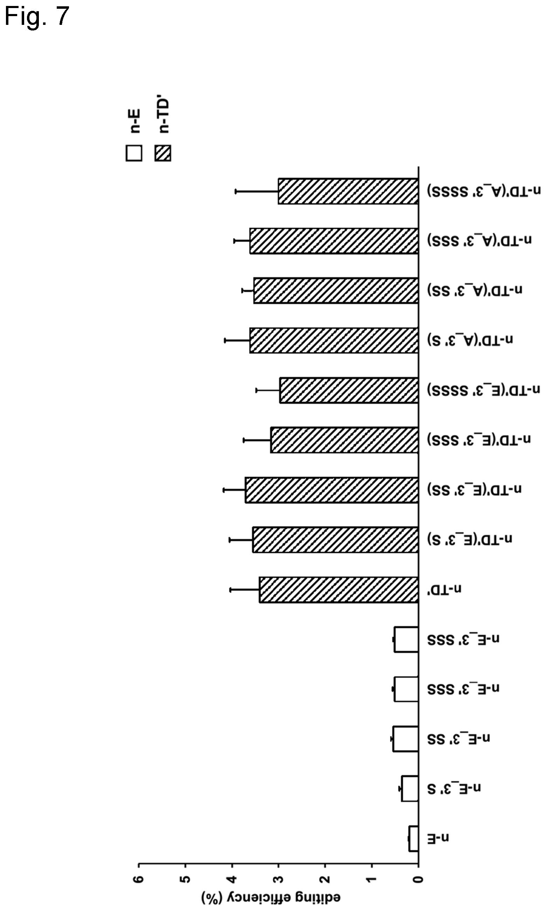

Fig. 7 shows the results. PS modifications at up to two positions from the 3' end for E strands and at up to three positions from the 3' end for A strands were found to tend to give enhanced editing efficiency.

### (3-3) Influence of PS modification on base sequence editing efficiency - Part 3

Using E strands having PS modifications at two different positions, examination was performed on base sequence editing efficiency in the same manner as in Example 1. The nucleic acids for editing that were used (only those with a modification) are shown in the following (having a PS modification in the 3' phosphate moiety of each underlined part).

E strand
copGFP-79_AS_E 3'SSp11: SEQ ID NO: 33 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E 3'SSp21: SEQ ID NO: 34 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E 3'SSp31: SEQ ID NO: 35 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E 3'SSp41: SEQ ID NO: 36 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E 3'SSp51: SEQ ID NO: 37 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E 3'SSp61: SEQ ID NO: 38 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E 3'SSp71: SEQ ID NO: 39

5'-
CGTAGCCGCTGGGGTAGGTGCCGAAGTGGTAGAAGCCGTAGCCCATCACGTGGC
TCAGCAGGTAGGGGCTGAAGGTCAG -3'

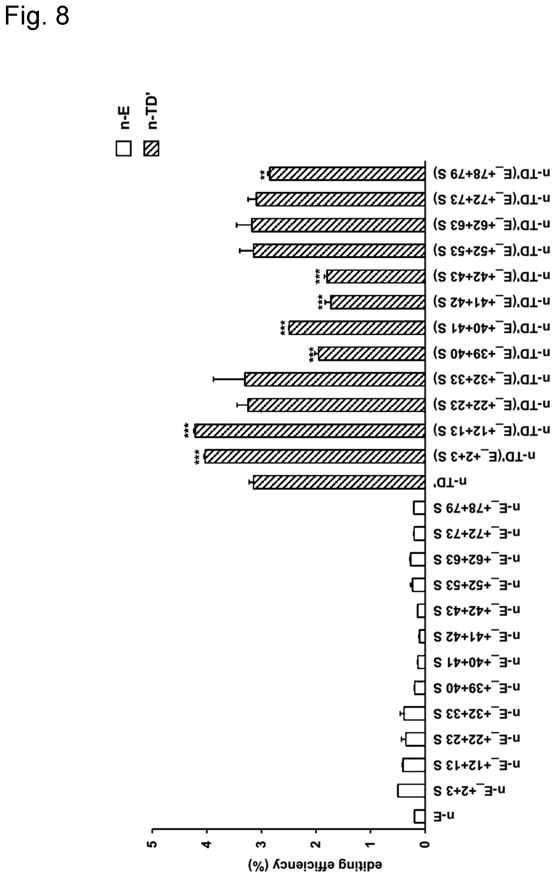

Fig. 8 shows the results. PS modifications at two positions in an E strand gave enhanced editing efficiency in the case that the PS modifications were near the 3' end of the E strand. On the other hand, inclusion of PS modifications at two positions within the range from the third base 5' to the mutation introduced for base sequence editing to the first base 3' to the mutation resulted in lowered editing efficiency.

### (3-4) Influence of PCH₃ (P-Me) modification and POCH₂CH₃ (P-OEt) modification in A strand on base sequence editing efficiency

For each of A strands, a PCH₃ (P-Me) modification (substitution of a phosphodiester bond with a methylphosphonate bond) or POCH₂CH₃ (P-OEt) modification (substitution of a phosphodiester bond with an ethylphosphotriester bond) was introduced at two positions in the 5'-end portion or the 3'-end portion, and examination was performed on base sequence editing efficiency in the same manner as in Example 1. The nucleic acids for editing that were used (only those with a modification) are shown in the following (having a P-Me modification or a P-OEt modification in the 3' phosphate moiety of each underlined part).

A strand
copGFP_forAS_E79 A35 5' p-met ×2: SEQ ID NO: 40
   5'-CTGACCTTCAGCCCCTACCTGCTGAGCCACGTGAT -3'
copGFP_forAS_E79 A35 3' p-met ×2: SEQ ID NO: 41
   5'-CTGACCTTCAGCCCCTACCTGCTGAGCCACGTGAT -3'
copGFP_forAS_E79 A35 5' p-etho ×2: SEQ ID NO: 42
   5'-CTGACCTTCAGCCCCTACCTGCTGAGCCACGTGAT -3'
copGFP_forAS_E79 A35 3' p-etho ×2: SEQ ID NO: 43
   5'-CTGACCTTCAGCCCCTACCTGCTGAGCCACGTGAT -3'

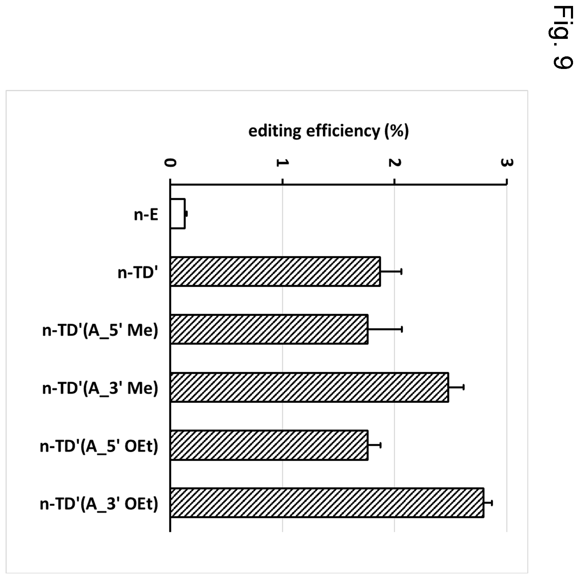

Fig. 9 shows the results. P-Me modifications or P-OEt modifications at two positions 3' to an A strand gave enhanced editing efficiency.

### (3-5) Influence of P-OEt modification in A strand on base sequence editing efficiency

For each of A strands, a P-OEt modification was introduced at one or two positions in the 3'-end portion, and examination was performed on base sequence editing efficiency in the same manner as in Example 1. The nucleic acids for editing that were used (only those with a P-OEt modification) are shown below (having a P-OEt modification in the 3' phosphate moiety of the nucleotide at each underlined part). In addition, A strands containing a PS modification(s) at one or two positions on the 3'-end portion (copGFP-35_S_A 3'S and copGFP-35_S_A 3'SS) were used.

A strand
copGFP-35_S_A 3'S: SEQ ID NO: 19
copGFP-35_S_A 3'SS: SEQ ID NO: 22
copGFP_forAS_E79 A35 3' p-etho ×1: SEQ ID NO: 44
   5'-CTGACCTTCAGCCCCTACCTGCTGAGCCACGTGAT -3'
copGFP_forAS_E79 A35 3' p-etho ×2: SEQ ID NO: 45
   5'-CTGACCTTCAGCCCCTACCTGCTGAGCCACGTGAT -3'

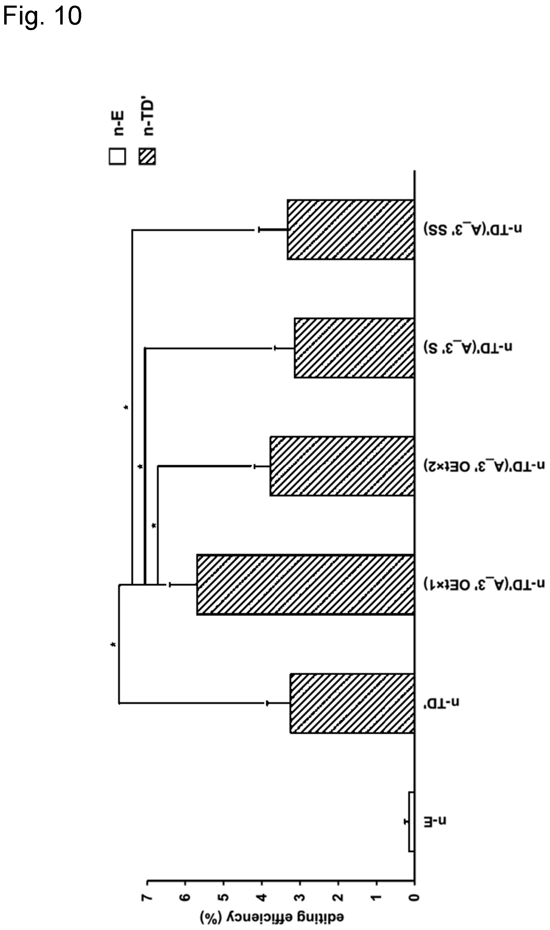

Fig. 10 shows the results. A P-OEt modification in the 3'-end portion of an A strand gave enhanced editing efficiency.

### (3-6) Influence of LNA modification in E strand on base sequence editing efficiency

For each of E strands, an LNA modification was introduced in the vicinity of a mutation introduced for base sequence editing, and examination was performed on base sequence editing efficiency in the same manner as in Example 1. The nucleic acids for editing that were used (only those with a modification) are shown below (having an LNA modification on the nucleotide at each underlined part). For the names of the sequences, the position of the mutation was defined as 0, and the position of a base 5' to position 0 was specified with a minus sign (-) and the position of a base 3' to position 0 was specified with a plus sign (+).

E strand
copGFP-79_AS_E LNA0: SEQ ID NO: 46 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-1+0+1: SEQ ID NO: 47 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-1+1: SEQ ID NO: 48 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-3-1+1+3: SEQ ID NO: 49 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-5-3-1+1+3+5: SEQ ID NO: 50 
5'-

CGTAGCCGCTGGGGTAGGTGCCGAAGTGGTAGAAGCCGTAGCCCATCACGTGGC

TCAGCAGGTAGGGGCTGAAGGTCAG -3'
copGFP-79_AS_E LNA-2+2: SEQ ID NO: 51 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-2-1+1+2: SEQ ID NO: 52 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-3-2+2+3: SEQ ID NO: 53 
5'-

CGTAGCCGCTGGGGTAGGTGCCGAAGTGGTAGAAGCCGTAGCCCATCACGTGGC

TCAGCAGGTAGGGGCTGAAGGTCAG -3'
copGFP-79_AS_E LNA-3-2-1+1+2+3: SEQ ID NO: 54

5'-
CGTAGCCGCTGGGGTAGGTGCCGAAGTGGTAGAAGCCGTAGCCCATCACGTGGC
TCAGCAGGTAGGGGCTGAAGGTCAG -3'

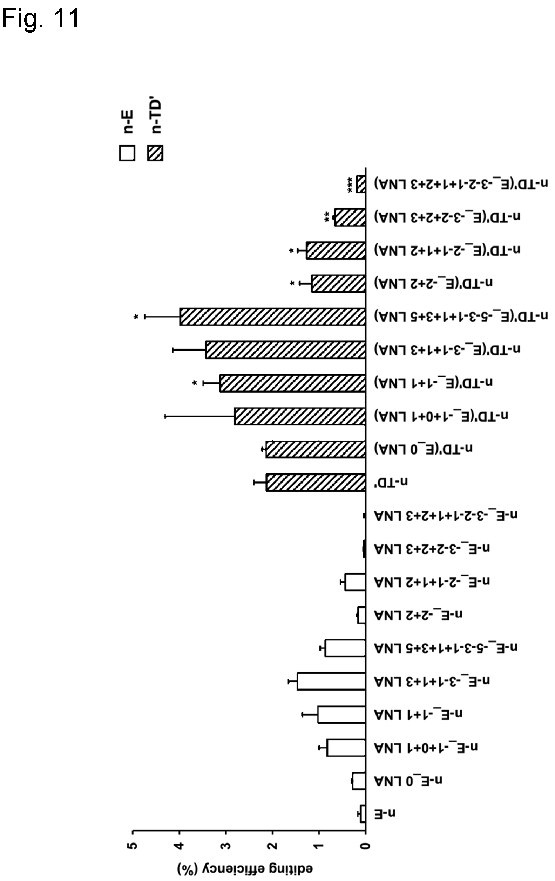

Fig. 11 shows the results. An LNA modification between specific positions sandwiching the mutation gave enhanced editing efficiency. In particular, introduction of LNA modifications at six positions of -5, -3, -1, +1, +3, and +5, with the position of the mutation defined as 0, gave the most enhanced editing efficiency. On the other hand, introduction of LNA modifications at positions of -2 and +2 resulted in lowered editing efficiency. The same results were obtained in editing an E strand alone.

### (3-7) Enhancement of base sequence editing efficiency by combination of LNA modification in E strand and P-OEt modification in A strand

More positions for modification were employed in introducing an LNA modification in the vicinity of a mutation in an E strand in combination with a P-OEt modification in an A strand, and examination was performed on base sequence editing efficiency in the same manner as in Example 1. The nucleic acids for editing that were used (only those with a modification) are shown below (having an LNA modification on the nucleotide at each underlined part). For the names of the sequences, the position of the mutation was defined as 0, and the position of a base 5' to position 0 was specified with a minus sign (-) and the position of a base 3' to position 0 was specified with a plus sign (+).

E strand
copGFP-79_AS_E LNA-1+1: SEQ ID NO: 48
copGFP-79_AS_E LNA-3-1+1+3: SEQ ID NO: 49
copGFP-79_AS_E LNA-5-3-1+1+3+5: SEQ ID NO: 50
copGFP-79_AS_E LNA-7-5-3-1+1+3+5+7: SEQ ID NO: 55 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-9-7-5-3-1+1+3+5+7+9: SEQ ID NO: 56

5'-
CGTAGCCGCTGGGGTAGGTGCCGAAGTGGTAGAAGCCGTAGCCCATCACGTGGC
TCAGCAGGTAGGGGCTGAAGGTCAG -3'

A strand
copGFP_forAS_E79 A35 3' p-etho ×1: SEQ ID NO: 44

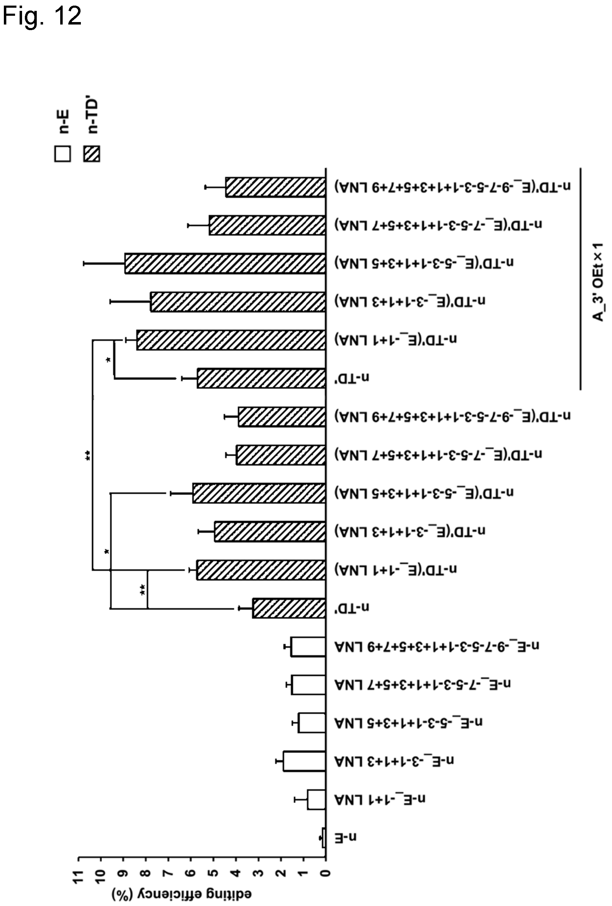

Fig. 12 shows the results. Introduction of LNA modifications at eight positions of - 7, -5, -3, -1, +1, +3, +5, and +7, or even at 10 positions of -9, -7, -5, -3, -1, +1, +3, +5, +7, and +9, with the position of the mutation defined as 0, gave enhanced editing efficiency. The same results were obtained in editing an E strand alone. It was found that combining with a P-OEt modification in an A strand tended to give enhanced editing efficiency in an additive manner; in particular, E strands having LNA modifications introduced at two to six positions gave high editing efficiency.

### Example 4: Enhancement of base sequence editing efficiency by LNA modification in E strand and P-OEt modification in A strand

With use of a mutated mEGFP gene as a target gene, examination was performed on enhancement of base sequence editing efficiency by an LNA modification in an E strand and a P-OEt modification in an A strand in the same manner as in Example 2. The nucleic acids for editing that were used (only those with a modification for E strands and A strands) are shown below. For an E strand containing a modification, the E strand has an LNA modification in the nucleotide at each underlined part. For an A strand having a modification, the A strand has a P-OEt modification in the 3' phosphate moiety of the nucleotide at the underlined part. As controls, an E strand (SEQ ID NO: 8) and an A strand (SEQ ID NO: 9) each containing no modification were used.

E strand
mEGFP-79_AS_E LNA-2,0: SEQ ID NO: 57

5'-

TCATGTGGTCGGGGTAGCGGCTGAAGCACTGCACGCCGTAGGTCAGGGTGGTCA

CGAGGGTGGGCCAGGGCACGGGCAG -3'

A strand
mEGFP-35_S_A 3' p-etho×1: SEQ ID NO: 58
5'- CTGCCCGTGCCCTGGCCCACCCTCGTGACCACCCT -3'

A' strand
mEGFP-65_AS_Ap: SEQ ID NO: 59

5'-
GAAGAAGATGGTGCGCTCCTGGACGTAGCCTTCGGGCATGGCGGACTTGAAGAA
GTCGTGCTGCT -3'

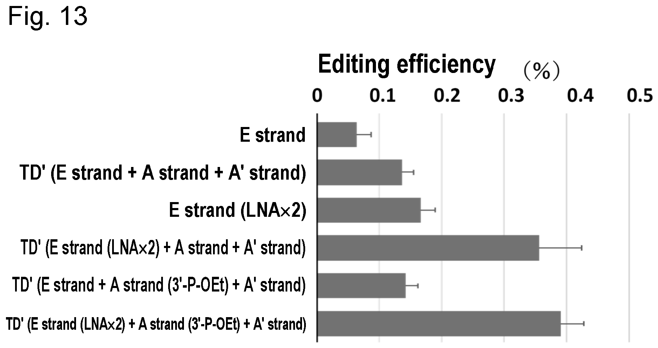

Fig. 13 shows the results. Introduction of LNA modifications at two positions of position -2 and position 0 in an E strand and a P-OEt modification in an A strand gave enhanced editing efficiency. In particular, an LNA modification in an E strand gave substantially enhanced editing efficiency. The same results were obtained in editing an E strand alone.

### Example 5: Examination on ratio among E strand, A strand, and A' strand

The molar ratio of nucleic acids for editing (total of E strand, A strand, and A' strand) to a plasmid DNA (pDNA) containing a target gene (pDNA:nucleic acids for editing) was set to 1:60 and the molar ratio of E strand:A strand:A' strand was varied, and examination was performed on base sequence editing efficiency in the same manner as in Example 1. The nucleic acids for editing that were used are shown in the following.

E strand
copGFP-153-79b-AS (AS_E strand): SEQ ID NO: 5

A strand
copGFP-153-79b-AS-35TD3'-ODN (A strand for AS_E strand): SEQ ID NO: 6

A' strand
copGFP-153-79b-AS-65TDdown-ODN (alias: copGFP_A_prime65) (A' strand for AS_E strand): SEQ ID NO: 7

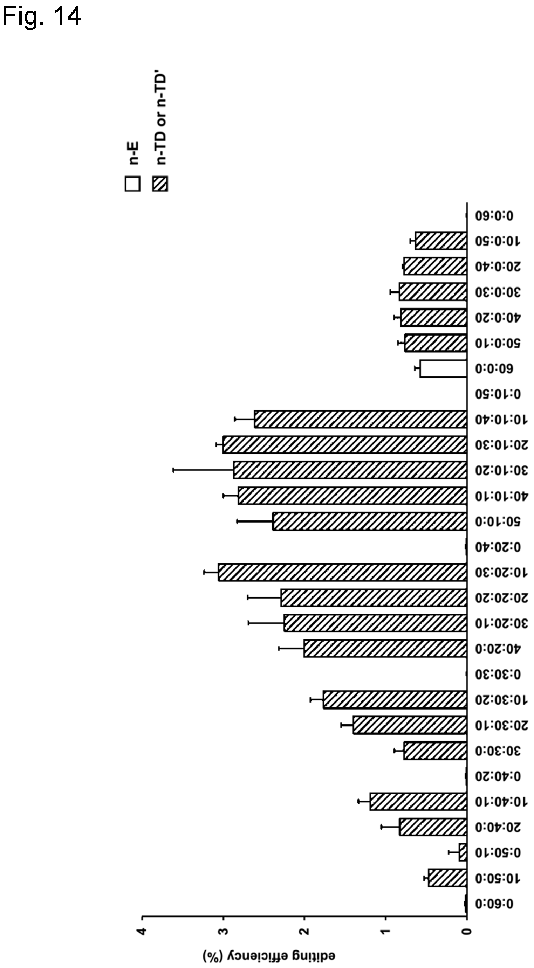

Fig. 14 shows the results. At a constant molar concentration of the E strand, a tendency of higher editing efficiency was found when the molar concentration of the A' strand was equal to or higher than the molar concentration of the A strand. At a constant molar concentration of the A strand, a tendency of higher editing efficiency was found when the molar concentration of the A' strand was equal to or higher than the molar concentration of the E strand. At a constant molar concentration of the A' strand, a tendency of higher editing efficiency was found when the molar concentration of the E strand was equal to or higher than the molar concentration of the A strand. A tendency of higher editing efficiency was found when the molar concentration of the A strand was lower than the total of the molar concentrations of the E strand and the A' strand.

### Example 6: Influence of positional relationship between A' strand and E strand on base sequence editing efficiency - Part 1

A' strands were designed to give an interval (i.e., a gap) of zero to nine bases between a base sequence having sequence identity with an E strand and a base sequence having sequence identity with an A' strand in the antisense strand of a target gene, and examination was performed on base sequence editing efficiency in the same manner as in Example 1. The nucleic acids for editing that were used are shown below. For the name of each A' strand, the numeral following "Gap" indicates the number of nucleotides of the gap. For copGFP_A_prime65 (SEQ ID NO: 7), the gap is of 0 base (this positional relationship is also referred to as "nick").

E strand
copGFP-153-79b-AS (AS_E strand): SEQ ID NO: 5

A strand
copGFP-153-79b-AS-35TD3'-ODN (A strand for AS_E strand): SEQ ID NO: 6 A' strand
copGFP-153-79b-AS-65TDdown-ODN (alias: copGFP_A_prime65) (A' strand for AS_E strand): SEQ ID NO: 7
65Ap_AS_Gap1: SEQ ID NO: 60 
5'-
CCTCGTACTTCTCGATGCGGGTGTTGGTGTAGCCGCCGTTGTTGATGGCGTGCAG
GAAGGGGTTC -3'
65Ap_AS_Gap2: SEQ ID NO: 61 
5'-
TCCTCGTACTTCTCGATGCGGGTGTTGGTGTAGCCGCCGTTGTTGATGGCGTGCA
GGAAGGGGTT -3'
65Ap_AS_Gap3: SEQ ID NO: 62 
5'-
GTCCTCGTACTTCTCGATGCGGGTGTTGGTGTAGCCGCCGTTGTTGATGGCGTGC
AGGAAGGGGT -3'
65Ap_AS_Gap6: SEQ ID NO: 63 
5'-
GCCGTCCTCGTACTTCTCGATGCGGGTGTTGGTGTAGCCGCCGTTGTTGATGGCG
TGCAGGAAGG -3'
65Ap_AS_Gap9: SEQ ID NO: 64

5'-

GCCGCCGTCCTCGTACTTCTCGATGCGGGTGTTGGTGTAGCCGCCGTTGTTGATG
GCGTGCAGGA -3'

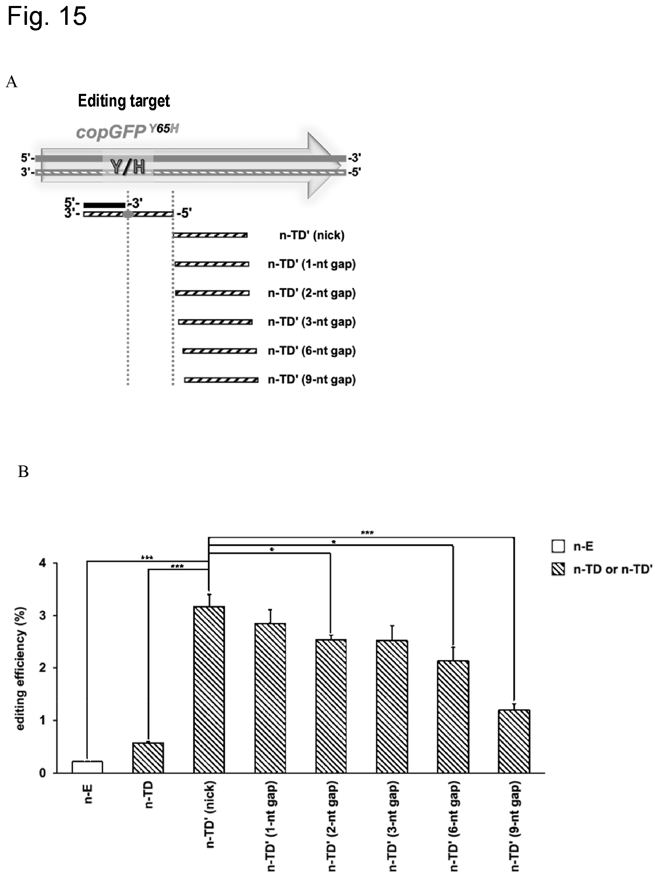

Fig. 15 shows the results. Although lower editing efficiency resulted as the gap was larger, enhanced editing efficiency by addition of an A' strand was found even with a gap of nine bases.

### Example 7: Influence of positional relationship between A' strand and E strand on base sequence editing efficiency - Part 2

A' strands were designed to give an overlapping of 3 to 39 bases between a base sequence having sequence identity with an E strand and a base sequence having sequence identity with an A' strand in the antisense strand of a target gene, and examination was performed on base sequence editing efficiency in the same manner as in Example 1. The nucleic acids for editing that were used are shown in the following.

E strand
copGFP-153-79b-AS (AS_E strand): SEQ ID NO: 5

A strand
copGFP-153-79b-AS-35TD3'-ODN (A strand for AS_E strand): SEQ ID NO: 6

A' strand
copGFP-153-79b-AS-65TDdown-ODN (alias: copGFP_A_prime65) (A' strand for AS_E strand): SEQ ID NO: 7
copGFP-79_AS_Ap 3-overlap: SEQ ID NO: 65 
5'-
GTACTTCTCGATGCGGGTGTTGGTGTAGCCGCCGTTGTTGATGGCGTGCAGGAAG
GGGTTCTCGT -3'
copGFP-79_AS_Ap 6-overlap: SEQ ID NO: 66 
5'-
CTTCTCGATGCGGGTGTTGGTGTAGCCGCCGTTGTTGATGGCGTGCAGGAAGGGG
TTCTCGTAGC -3'
copGFP-79_AS_Ap 9-overlap: SEQ ID NO: 67 
5'-
CTCGATGCGGGTGTTGGTGTAGCCGCCGTTGTTGATGGCGTGCAGGAAGGGGTTC
TCGTAGCCGC -3'
copGFP-79_AS_Ap 12-overlap: SEQ ID NO: 68 
5'-
GATGCGGGTGTTGGTGTAGCCGCCGTTGTTGATGGCGTGCAGGAAGGGGTTCTCG
TAGCCGCTGG -3'
copGFP-79_AS_Ap 15-overlap: SEQ ID NO: 69 
5'-
GCGGGTGTTGGTGTAGCCGCCGTTGTTGATGGCGTGCAGGAAGGGGTTCTCGTAG
CCGCTGGGGT -3'
copGFP-79_AS_Ap 19-overlap: SEQ ID NO: 70 
5'-
GTGTTGGTGTAGCCGCCGTTGTTGATGGCGTGCAGGAAGGGGTTCTCGTAGCCGC
TGGGGTAGGT -3'
copGFP-79_AS_Ap 29-overlap: SEQ ID NO: 71 
5'-
AGCCGCCGTTGTTGATGGCGTGCAGGAAGGGGTTCTCGTAGCCGCTGGGGTAGG
TGCCGAAGTGG -3'
copGFP-79_AS_Ap 39-overlap: SEQ ID NO: 72

5'-
GTTGATGGCGTGCAGGAAGGGGTTCTCGTAGCCGCTGGGGTAGGTGCCGAAGTG
GTAGAAGCCGT -3'

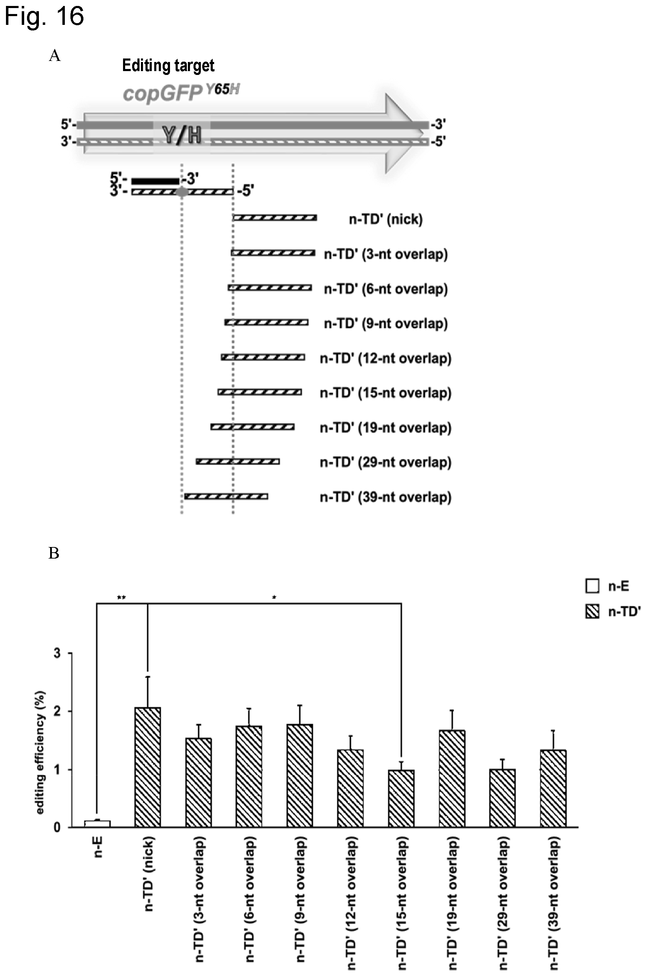

Fig. 16 shows the results. Although it was found that the presence of an overlap tended to give lower editing efficiency than the absence of an overlap (n-TD'(nick)), enhanced editing efficiency by addition of an A' strand was confirmed even with an overlap of 3 to 39 bases.

### Example 8: Influence of length of A strand on base sequence editing efficiency

A strands with varied lengths and positions to form a double strand with an E strand were designed, and examination was performed on base sequence editing efficiency in the same manner as in Example 1. The nucleic acids for editing that were used are shown below. An A strand consisting of 35 nucleotides (SEQ ID NO: 7) was used as a reference, and the length was extended to the 5' portion or the 3' portion. Considering that if the length of the A strand is extended in the 3' direction, the resulting strand contains a base sequence complementary to a part containing a mutation in an E strand, A strands each having the sequence of wild-type (WT) copGFP gene (i.e., completely complementary to the E strand; SEQ ID NOs: 71 to 75) and A strands each having the sequence of a mutated (Y/H) copGFP gene (i.e., not complementary to the E strand and the mutated part; SEQ ID NOs: 76 to 80) were both designed.

E strand
copGFP-153-79b-AS (AS_E strand): SEQ ID NO: 5

A strand
copGFP-153-79b-AS-35TD3'-ODN (A strand for AS_E strand): SEQ ID NO: 6
copGFP-153-79b-AS-45TD3'-ODN5': SEQ ID NO: 73
   5'- CAAAGGCGCCCTGACCTTCAGCCCCTACCTGCTGAGCCACGTGAT -3'
copGFP-153-79b-AS-55TD3'-ODN5': SEQ ID NO: 74 
5'-
TGAAGAGCACCAAAGGCGCCCTGACCTTCAGCCCCTACCTGCTGAGCCACGTGA
T -3'
copGFP-153-79b-AS-65TD3'-ODN5': SEQ ID NO: 75 
5'-
ACCAACAAGATGAAGAGCACCAAAGGCGCCCTGACCTTCAGCCCCTACCTGCTG
AGCCACGTGAT -3'
copGFP-153-79b-AS-75TD3'-ODN5': SEQ ID NO: 76 
5'-
GGGCCGCATGACCAACAAGATGAAGAGCACCAAAGGCGCCCTGACCTTCAGCCC
 CTACCTGCTGAGCCACGTGAT -3'
copGFP-153-79b-AS-45TD3'-ODN3': SEQ ID NO: 77
   5'- CTGACCTTCAGCCCCTACCTGCTGAGCCACGTGATGGGCTACGGC -3'
copGFP-153-79b-AS-55TD3'-ODN3': SEQ ID NO: 78 
5'-
CTGACCTTCAGCCCCTACCTGCTGAGCCACGTGATGGGCTACGGCTTCTACCACT
-3'
copGFP-153-79b-AS-65TD3'-ODN3': SEQ ID NO: 79 
5'-
CTGACCTTCAGCCCCTACCTGCTGAGCCACGTGATGGGCTACGGCTTCTACCACT
TCGGCACCTA -3'
copGFP-153-79b-AS-75TD3'-ODN3': SEQ ID NO: 80 
5'-
CTGACCTTCAGCCCCTACCTGCTGAGCCACGTGATGGGCTACGGCTTCTACCACT
TCGGCACCTACCCCAGCGGC -3'
copGFP-153-79b-AS-79TD3'-ODN3': SEQ ID NO: 81 
5'-
CTGACCTTCAGCCCCTACCTGCTGAGCCACGTGATGGGCTACGGCTTCTACCACT
TCGGCACCTACCCCAGCGGCTACG -3'
79E_AS_45TD3'-ODN3': SEQ ID NO: 82
   5'- CTGACCTTCAGCCCCTACCTGCTGAGCCACGTGATGGGCCACGGC -3'
79E_AS_55TD3'-ODN3': SEQ ID NO: 83 
5'-
CTGACCTTCAGCCCCTACCTGCTGAGCCACGTGATGGGCCACGGCTTCTACCACT
-3'
79E_AS_65TD3'-ODN3': SEQ ID NO: 84 
5'-
CTGACCTTCAGCCCCTACCTGCTGAGCCACGTGATGGGCCACGGCTTCTACCACT
 TCGGCACCTA -3'
79E_AS_75TD3'-ODN3': SEQ ID NO: 85 
5'-
CTGACCTTCAGCCCCTACCTGCTGAGCCACGTGATGGGCCACGGCTTCTACCACT
TCGGCACCTACCCCAGCGGC -3'
79E_AS_79TD3'-ODN3': SEQ ID NO: 86

5'-
CTGACCTTCAGCCCCTACCTGCTGAGCCACGTGATGGGCCACGGCTTCTACCACT
TCGGCACCTACCCCAGCGGCTACG -3'

A' strand
copGFP-153-79b-AS-65TDdown-ODN (alias: copGFP_A_prime65) (A' strand for AS_E strand): SEQ ID NO: 7

Fig. 17 shows the results. For A strands each having the 5' end extended over the 3' end of the E strand, a tendency of lower editing efficiency was found as the length was larger, whereas high editing efficiency by addition of an A' strand seemed to be maintained. By contrast, for A strands each having the 3'-end extended to contain a base sequence complementary to a part containing the mutation in the E strand, lowered editing efficiency resulted.

### Example 9: Influence of distance between double-stranded part of n-TD' and mutation on base sequence editing efficiency

E strands and A strands were designed to dispose a mutation in an E strand at a position of position +44 (the 5' end of the E strand), position +34, position +24, position +14, position +5, position +4, position +1, position -6, position -16, position -26, or position -34, with the position of the base in the E strand complementary to the base at the 3' end of an A strand defined as position -1 (+ indicates that the double strand part of a TD does not overlap with the mutated part, and - indicates that the double strand part of a TD overlaps with the mutated part), and examination was performed on base sequence editing efficiency in the same manner as in Example 1. The nucleic acids for editing that were used are shown in the following.
79GFP_AS_5'-1-mut(+44-WT): SEQ ID NO: 87 
5'-
AGCCCATCACGTGGCTCAGCAGGTAGGGGCTGAAGGTCAGGGCGCCTTTGGTGC
TCTTCATCTTGTTGGTCATGCGGCC -3'
35mer_for_79GFP_AS_5'-1-mut(+44-WT): SEQ ID NO: 88
   5'- GGCCGCATGACCAACAAGATGAAGAGCACCAAAGG -3'
65Ap_for_79E_AS_5'-1-mut(+44-WT): SEQ ID NO: 89 
5'-
GTTGATGGCGTGCAGGAAGGGGTTCTCGTAGCCGCTGGGGTAGGTGCCGAAGTG
GTAGAAGCCGT -3'
79GFP_AS_5'-11-mut(+34-WT): SEQ ID NO: 90 
5'-
TAGAAGCCGTAGCCCATCACGTGGCTCAGCAGGTAGGGGCTGAAGGTCAGGGCG
CCTTTGGTGCTCTTCATCTTGTTGG -3'
35mer_for_79GFP_AS_5'-11-mut(+34-WT): SEQ ID NO: 91
   5'-CCAACAAGATGAAGAGCACCAAAGGCGCCCTGACC -3'
65Ap_for_79E_AS_5'-11-mut(+34-WT): SEQ ID NO: 92 
5'-
AGCCGCCGTTGTTGATGGCGTGCAGGAAGGGGTTCTCGTAGCCGCTGGGGTAGG
TGCCGAAGTGG -3'
79GFP_AS_5'-21-mut(+24-WT): SEQ ID NO: 93 
5'-
GCCGAAGTGGTAGAAGCCGTAGCCCATCACGTGGCTCAGCAGGTAGGGGCTGAA
GGTCAGGGCGCCTTTGGTGCTCTTC -3'
35mer_for_79GFP_AS_5'-21-mut(+24-WT): SEQ ID NO: 94
   5'-GAAGAGCACCAAAGGCGCCCTGACCTTCAGCCCCT -3'
65Ap_for_79E_AS_5'-21-mut(+24-WT): SEQ ID NO: 95 
5'-
GTGTTGGTGTAGCCGCCGTTGTTGATGGCGTGCAGGAAGGGGTTCTCGTAGCCGC
TGGGGTAGGT -3'
79GFP_AS_5'-31-mut(+14-WT): SEQ ID NO: 96 
5'-
TGGGGTAGGTGCCGAAGTGGTAGAAGCCGTAGCCCATCACGTGGCTCAGCAGGT
AGGGGCTGAAGGTCAGGGCGCCTTT -3'
35mer_for_79GFP_AS_5'-31-mut(+14-WT): SEQ ID NO: 97
   5'-AAAGGCGCCCTGACCTTCAGCCCCTACCTGCTGAG -3'
65Ap_for_79E_AS_5'-31-mut(+14-WT): SEQ ID NO: 98 
5'-
CTCGATGCGGGTGTTGGTGTAGCCGCCGTTGTTGATGGCGTGCAGGAAGGGGTTC
TCGTAGCCGC -3'
79GFP_AS_5'-41-mut(+4-WT): SEQ ID NO: 99 
5'-
TCGTAGCCGCTGGGGTAGGTGCCGAAGTGGTAGAAGCCGTAGCCCATCACGTGG
CTCAGCAGGTAGGGGCTGAAGGTCA -3'
35mer_for_79GFP_AS_5'-41-mut(+4-WT): SEQ ID NO: 100
   5'-TGACCTTCAGCCCCTACCTGCTGAGCCACGTGATG -3'
65Ap_for_79E_AS_5'-41-mut(+4-WT): SEQ ID NO: 101 
5'-
CCTCGTACTTCTCGATGCGGGTGTTGGTGTAGCCGCCGTTGTTGATGGCGTGCAG
GAAGGGGTTC -3'
79GFP_AS_5'-44-mut(+1-WT): SEQ ID NO: 102 
5'-
TTCTCGTAGCCGCTGGGGTAGGTGCCGAAGTGGTAGAAGCCGTAGCCCATCACG
TGGCTCAGCAGGTAGGGGCTGAAGG -3'
35mer_for_79GFP_AS_5'-44-mut(+1-WT): SEQ ID NO: 103
   5'-CCTTCAGCCCCTACCTGCTGAGCCACGTGATGGGC -3'
65Ap_for_79E_AS_5'-44-mut(+1-WT): SEQ ID NO: 104 
5'-
CGTCCTCGTACTTCTCGATGCGGGTGTTGGTGTAGCCGCCGTTGTTGATGGCGTG
CAGGAAGGGG -3'
79GFP_AS_5'-51-mut(-6): SEQ ID NO: 105 
5'-
GAAGGGGTTCTCGTAGCCGCTGGGGTAGGTGCCGAAGTGGTAGAAGCCGTAGCC
CATCACGTGGCTCAGCAGGTAGGGG -3'
35mer_for_79GFP_AS_5'-51-mut(-6-WT): SEQ ID NO: 106
   5'-CCCCTACCTGCTGAGCCACGTGATGGGCTACGGCT -3'
35A_AS_5'-51-mut(-6-YH): SEQ ID NO: 107
   5'-CCCCTACCTGCTGAGCCACGTGATGGGCCACGGCT -3'
65Ap_for_79E_AS_5'-51-mut(-6): SEQ ID NO: 108 
5'-
ACGCCGCCGTCCTCGTACTTCTCGATGCGGGTGTTGGTGTAGCCGCCGTTGTTGA
TGGCGTGCAG -3'
79GFP_AS_5'-61-mut(-16): SEQ ID NO: 109 
5'-
TGGCGTGCAGGAAGGGGTTCTCGTAGCCGCTGGGGTAGGTGCCGAAGTGGTAGA
AGCCGTAGCCCATCACGTGGCTCAG -3'
35mer_for_79GFP_AS_5'-61-mut(-16-WT): SEQ ID NO: 110
   5'-CTGAGCCACGTGATGGGCTACGGCTTCTACCACTT -3'
35A_AS_5'-61-mut(-16-YH): SEQ ID NO: 111
   5'-CTGAGCCACGTGATGGGCCACGGCTTCTACCACTT -3'
65Ap_for_79E_AS_5'-61-mut(-16): SEQ ID NO: 112 
5'-
CACGTGCAGCACGCCGCCGTCCTCGTACTTCTCGATGCGGGTGTTGGTGTAGCCG
 CCGTTGTTGA -3'
79GFP_AS_5'-71-mut(-26): SEQ ID NO: 113 
5'-
CCGTTGTTGATGGCGTGCAGGAAGGGGTTCTCGTAGCCGCTGGGGTAGGTGCCG
AAGTGGTAGAAGCCGTAGCCCATCA -3'
35mer_for_79GFP_AS_5'-71-mut(-26-WT): SEQ ID NO: 114
   5'-TGATGGGCTACGGCTTCTACCACTTCGGCACCTAC -3'
35A_AS_5'-71-mut(-26-YH): SEQ ID NO: 115
   5'-TGATGGGCCACGGCTTCTACCACTTCGGCACCTAC -3'
65Ap_for_79E_AS_5'-71-mut(-26): SEQ ID NO: 116 
5'-
AGCTGAAGCTCACGTGCAGCACGCCGCCGTCCTCGTACTTCTCGATGCGGGTGTT
GGTGTAGCCG -3'
79GFP_AS_5'-79-mut(-34): SEQ ID NO: 117 
5'-
TGTAGCCGCCGTTGTTGATGGCGTGCAGGAAGGGGTTCTCGTAGCCGCTGGGGTA
GGTGCCGAAGTGGTAGAAGCCGTA -3'
35mer_for_79GFP_AS_5'-79-mut(-34-WT): SEQ ID NO: 118
   5'-TACGGCTTCTACCACTTCGGCACCTACCCCAGCGG -3'
35A_AS_5'-79-mut(-34-YH): SEQ ID NO: 119
   5'-CACGGCTTCTACCACTTCGGCACCTACCCCAGCGG -3'
65Ap_for_79E_AS_5'-79-mut(-34): SEQ ID NO: 120

5'-
GGTTGTGGGCGTAGCTCTTCATGCTCCTGCCGCCGCACGACGTGCACTCGAAGTC
GATGGCGATG -3'

Fig. 18 shows the results. Introduction of a mutation to the center base, the 35th base from each end, of an E strand of 79 nucleotides in length ("+5-WT") gave the highest editing efficiency. When the position of the mutation was moved by varying the base sequences while the lengths of the three strands and the relative positional relationship were kept constant, lowered editing efficiency resulted whether it was moved in the 5' direction or 3' direction. In particular, substantially lowered editing efficiency resulted when the mutation was away from position +14 or closer than position +4.

### Example 10: Examination on position of nick in n-TD'

If a sequence having sequence identity with an E strand and a sequence having sequence identity with an A' strand are adjacent in an editing target strand, the E strand and the A' strand can be regarded as two single-stranded polynucleotides obtained by introducing an unlinked part (nick) at one position of a single-stranded polynucleotide of a certain length. In view of this, E strands and A' strands corresponding to varied nick positions were designed, and examination was performed on base sequence editing efficiency in the same manner as in Example 1. Specifically, E strands as antisense strands of 109, 79, 65, and 35 nucleotides in length and A' strands of 35, 65, 79, and 109 nucleotides in length each being immediately 5' to the corresponding E strand were produced. In addition, an E strand of 144 nucleotides in length without being combined with any A' strand was prepared. A single-stranded polynucleotide consisting of a base sequence complementary to 35 nucleotides from the 3' end of each E strand was used as an A strand. The nucleic acids for editing that were used are shown in the following.
144E_AS_5'-105-mut: SEQ ID NO: 121 
5'-
CTCGTACTTCTCGATGCGGGTGTTGGTGTAGCCGCCGTTGTTGATGGCGTGCAGG
AAGGGGTTCTCGTAGCCGCTGGGGTAGGTGCCGAAGTGGTAGAAGCCGTAGCCC
ATCACGTGGCTCAGCAGGTAGGGGCTGAAGGTCAG -3'
109E_AS_5'-70-mut_for_35Ap: SEQ ID NO: 122 
5'-
CGTTGTTGATGGCGTGCAGGAAGGGGTTCTCGTAGCCGCTGGGGTAGGTGCCGA
AGTGGTAGAAGCCGTAGCCCATCACGTGGCTCAGCAGGTAGGGGCTGAAGGTCA
G -3'
35Ap_for_109E_AS_5'-70-mut: SEQ ID NO: 123
   5'-CTCGTACTTCTCGATGCGGGTGTTGGTGTAGCCGC -3'
65E_AS_5'-26-mut_for_79Ap: SEQ ID NO: 124 
5'-
TAGGTGCCGAAGTGGTAGAAGCCGTAGCCCATCACGTGGCTCAGCAGGTAGGGG
CTGAAGGTCAG -3'
79Ap_for_65E_AS_5'-26-mut: SEQ ID NO: 125 
5'-
CTCGTACTTCTCGATGCGGGTGTTGGTGTAGCCGCCGTTGTTGATGGCGTGCAGG
AAGGGGTTCTCGTAGCCGCTGGGG -3'
35E_AS_notmut_for_109Ap: SEQ ID NO: 126
   5'-ATCACGTGGCTCAGCAGGTAGGGGCTGAAGGTCAG -3'
109Ap_5'-105-mut_for_35E_AS_notmut: SEQ ID NO: 127

5'-
CTCGTACTTCTCGATGCGGGTGTTGGTGTAGCCGCCGTTGTTGATGGCGTGCAGG
AAGGGGTTCTCGTAGCCGCTGGGGTAGGTGCCGAAGTGGTAGAAGCCGTAGCCC
-3'

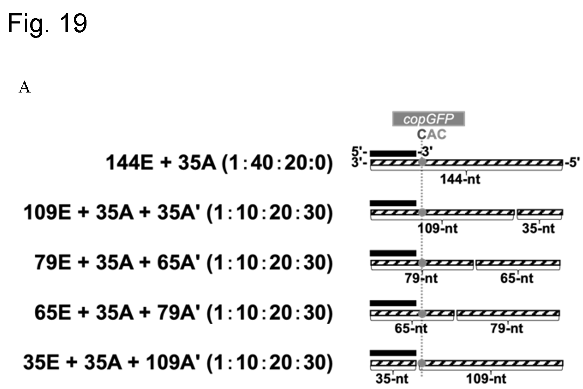

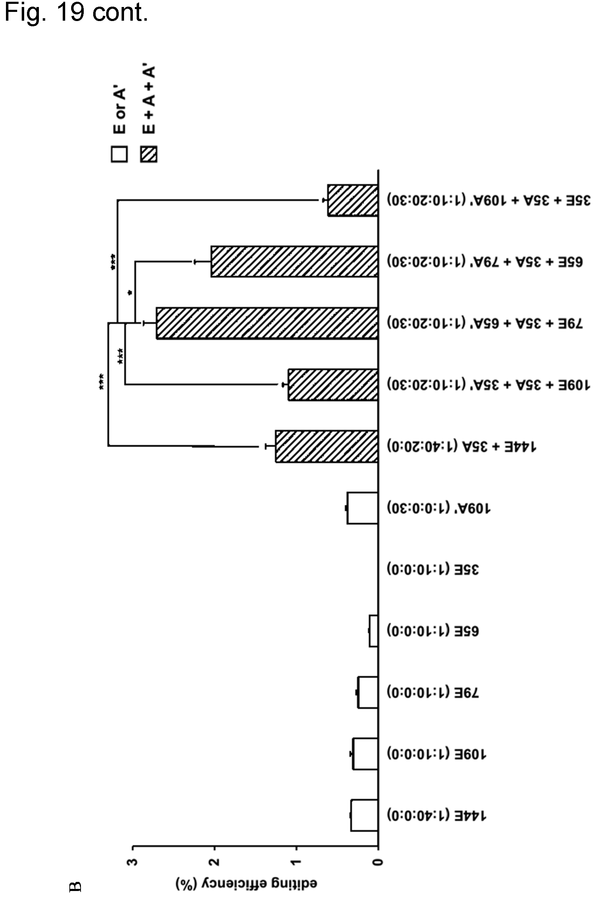

Fig. 19 shows the results. Use of a combination of an E strand of 79 nucleotides in length and an A' strand of 65 nucleotides in length, or a combination of an E strand of 65 nucleotides in length and an A' strand of 79 nucleotides in length gave high editing efficiency.

### Example 11: Genome editing using TD with addition of A' strand

With U2OS cells having a mutated copGFP gene, as a target gene, inserted into the genome, examination was performed on base sequence editing efficiency in the same manner as in Example 1.

The cells used in the present example had been produced by lipofection of U2OS cells with an mPlum-T2A-copGFP (Y/H) expression vector plasmid (H. Kawai, R. Kamitsubo, H. Kamiya. Correction of monomeric enhanced green fluorescent protein (mEGFP) gene by short 5'-tailed duplexes. J. Biosci. Bioengng. 134, 175-181 (2022)) and the subsequent cloning with drug (Zeocin) resistance and the fluorescence of mPlum as indicators.

For each of an E strand having LNA introduced at two positions of position -1 and position +1, an A strand, and an A' strand, a PS modification was introduced at one position in the 5'-end portion, and the resulting strands were used. The nucleic acids for editing that were used (with each modified part indicated with an underline; having a PS modification in the 3' phosphate moiety of each underlined part).
E strand
copGFP-79_AS_E LNA-1+1_5'PS: SEQ ID NO: 128

5'-
CGTAGCCGCTGGGGTAGGTGCCGAAGTGGTAGAAGCCGTAGCCCATCACGTGGC
TCAGCAGGTAGGGGCTGAAGGTCAG -3'

A strand
copGFP-35_S_A_5'PS (alias: copGFP-35_S_A 5'S): SEQ ID NO: 18
5'-CTGACCTTCAGCCCCTACCTGCTGAGCCACGTGAT -3'

A' strand
copGFP_A_prime65_5'PS: SEQ ID NO: 129

5'-
CTCGTACTTCTCGATGCGGGTGTTGGTGTAGCCGCCGTTGTTGATGGCGTGCAGG
AAGGGGTTCT -3'

Fluorescence microscopy was performed with an Opera Phenix (R) high-throughput/high-contents imaging system (PerkinElmer, Inc.).

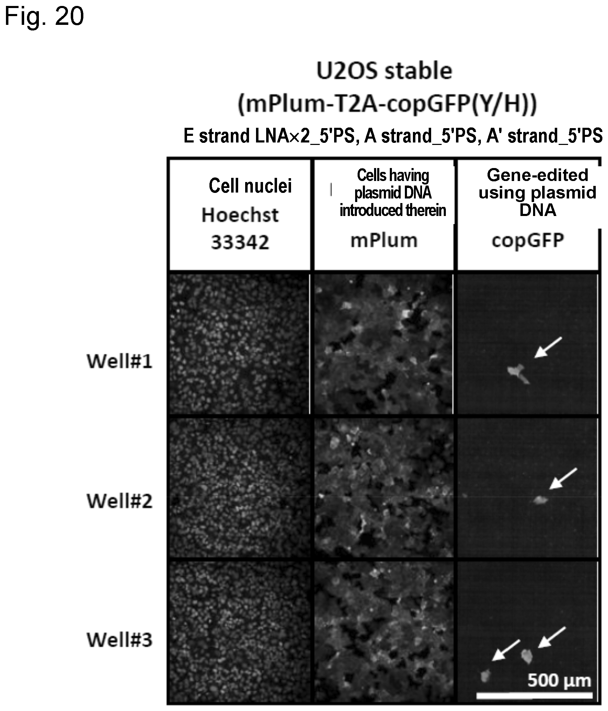

Fig. 20 shows the results. After the introduction of the TD and the A' strand, cells having the fluorescence of copGFP were observed. Accordingly, the method of the present invention was confirmed to allow genome editing.

### Example 12: Enhancement of base sequence editing efficiency by LNA modification in E strand and P-OEt modification in A strand

With use of a mutated mEGFP gene as a target gene, examination was performed on enhancement of base sequence editing efficiency by an LNA modification in an E strand and a P-OEt modification in an A strand in the same manner as in Example 2. The nucleic acids for editing that were used (only those with a modification for E strands and A strands) are shown below. For an E strand containing a modification, the E strand has an LNA modification on the nucleotide at each underlined part. For an A strand having a modification, the A strand has a P-OEt modification in the 3' phosphate moiety of the nucleotide at the underlined part. As controls, an E strand (SEQ ID NO: 8) and an A strand (SEQ ID NO: 9) each containing no modification were used.

### E strand

mEGFP-79_AS_E LNA-1+1: SEQ ID NO: 130

5'-
TCATGTGGTCGGGGTAGCGGCTGAAGCACTGCACGCCGTAGGTCAGGGTGGTCA
CGAGGGTGGGCCAGGGCACGGGCAG -3'

A strand
mEGFP-35_S_A 3' p-etho × 1: SEQ ID NO: 58
5'- CTGCCCGTGCCCTGGCCCACCCTCGTGACCACCCT -3'

A' strand
mEGFP-65_AS_Ap: SEQ ID NO: 59

5'-
GAAGAAGATGGTGCGCTCCTGGACGTAGCCTTCGGGCATGGCGGACTTGAAGAA
GTCGTGCTGCT -3'

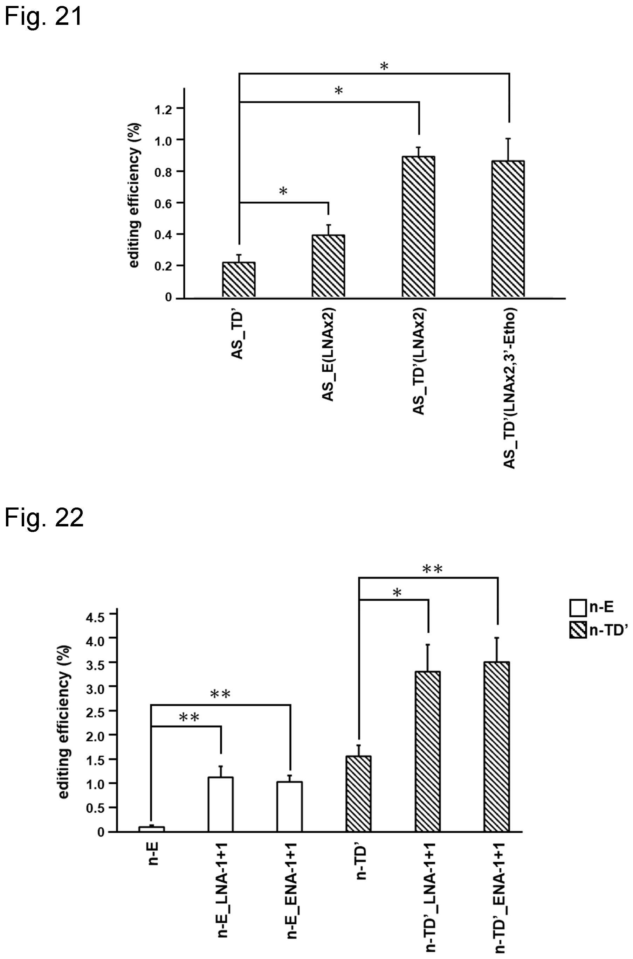

Fig. 21 shows the results. As with the case of using copGFP gene as a target gene, introduction of LNA modifications in the vicinity of a mutation in an E strand and a P-OEt modification in an A strand gave enhanced editing efficiency. In particular, LNA modifications in an E strand gave substantially enhanced editing efficiency. The same results were obtained in editing an E strand alone. In Fig. 21, "AS_TD'" shows the result for the TD' containing no modification, "AS_E(LNAx2)" shows the result for the E strand containing modifications, "AS_TD'(LNAx2)" shows the result for the TD' containing the E strand containing modifications, and "AS_TD'(LNAx2,3'-Etho)" shows the result for the TD' containing the E strand containing modifications and the A strand containing a modification.

### Example 13: Influence of ENA modification in E strand on base sequence editing efficiency

For an E strand, an ENA modification was introduced in the vicinity of a mutation introduced for base sequence editing (with each position of introduction of an LNA modification or an ENA modification indicated with an underline in the sequences), and examination was performed on base sequence editing efficiency in the same manner as in Example 1. The nucleic acids for editing that were used (only those with a modification) are shown below (having an LNA modification or an ENA modification on the nucleotide at each underlined part). For the names of the sequences, the position of a mutation was defined as 0, and the position of a base 5' to position 0 was specified with a minus sign (-) and the position of a base 3' to position 0 was specified with a plus sign (+).
copGFP-79_AS_E LNA-1+1: SEQ ID NO: 48 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E ENA-1+1: SEQ ID NO: 131

5'-
CGTAGCCGCTGGGGTAGGTGCCGAAGTGGTAGAAGCCGTAGCCCATCACGTGGC
TCAGCAGGTAGGGGCTGAAGGTCAG -3'

Fig. 22 shows the results. In editing with the n-E (only E strand) and the n-TD' (E strand + A strand + A strand'), the LNA modifications and the ENA modifications exhibited almost equivalent effects.

### Example 14: Influence of introduction of amino linker to 5' end or 3' end of different oligodeoxynucleotides (ODNs) on base sequence editing efficiency

An amino linker (AL linker) was introduced to the 5' end or 3' end of any of ODNs contained in an n-TD' (E strand + A strand + A strand'), and examination was performed on base sequence editing efficiency in the same manner as in Example 1. The nucleic acids for editing that were used (only those having an AL linker) are shown below. For introduction of an AL linker to the 5' ends and the 3' ends, 5'-Amino-Modifier C6 (product number: 0-1906-02, manufactured by Glen Research) and Phthalamido Amino C6 lcaa CPG (product number: N- 8217-10, manufactured by ChemGenes Corporation) were used, respectively.

E strand
copGFP-153-79b-AS(E)_5'NH2: an AL linker was introduced to the 5' end of SEQ ID NO: 5 
5'-AL-

CGTAGCCGCTGGGGTAGGTGCCGAAGTGGTAGAAGCCGTAGCCCATCACGTGGC

TCAGCAGGTAGGGGCTGAAGGTCAG-3'
copGFP-153-79b-AS(E)_3'NH2: an AL linker was introduced to the 3' end of SEQ ID NO: 5

5'-
CGTAGCCGCTGGGGTAGGTGCCGAAGTGGTAGAAGCCGTAGCCCATCACGTGGC
TCAGCAGGTAGGGGCTGAAGGTCAG-AL-3'

A strand
copGFP-153-79b-AS-35TD3'-ODN(A)_5'NH2: an AL linker was introduced to the 5' end of SEQ ID NO: 6
5'-AL-CTGACCTTCAGCCCCTACCTGCTGAGCCACGTGAT-3'
copGFP-153-79b-AS-35TD3'-ODN(A)_3'NH2: an AL linker was introduced to the 3' end of SEQ ID NO: 6
5'-CTGACCTTCAGCCCCTACCTGCTGAGCCACGTGAT-AL-3'

A' strand
copGFP-153-79b-AS-65TDdown-ODN(Aprime)_5'NH2: an AL linker was introduced to the 5' end of SEQ ID NO: 7 
5'-AL-
CTCGTACTTCTCGATGCGGGTGTTGGTGTAGCCGCCGTTGTTGATGGCGTGCAGG
AAGGGGTTCT-3'
copGFP-153-79b-AS-65TDdown-ODN(Aprime)_3'NH2: an AL linker was introduced to the 3' end of SEQ ID NO: 7

5'-
CTCGTACTTCTCGATGCGGGTGTTGGTGTAGCCGCCGTTGTTGATGGCGTGCAGG
AAGGGGTTCT-AL-3'

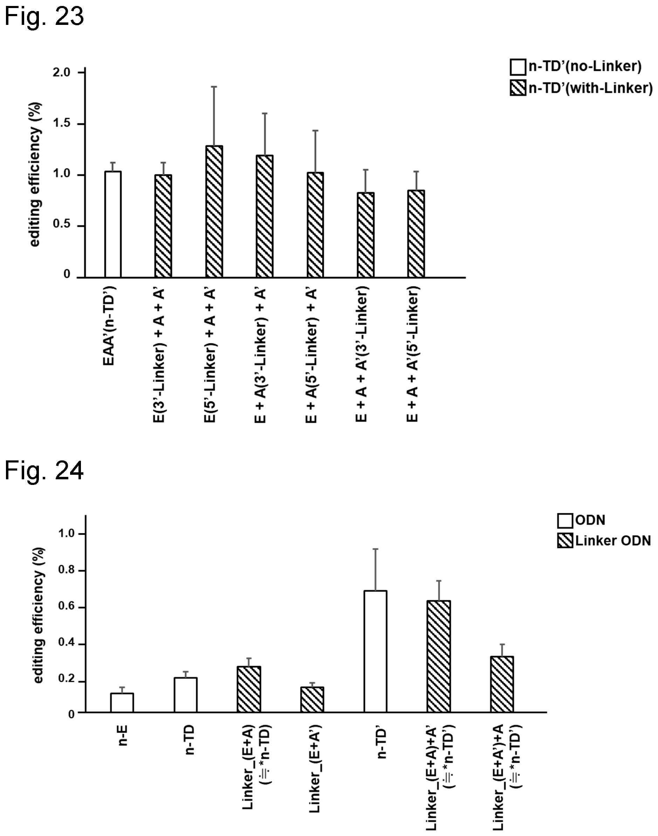

Fig. 23 shows the results. Introduction of an AL linker to the 5' end or 3' end of any of an E strand, A strand, and A' strand (in the graph, 5'-Linker or 3'-Linker) caused no influence on editing efficiency, as can be seen from comparison with the case without introduction of an AL linker (in the graph, EAA'(n-TD')). These results indicate that each end of each ODN can be modified and each two ODNs can be linked via a cleavable linker.

### Example 15: Comparison of base sequence editing efficiency with ODNs connected with linker

An E strand and an A strand, and an E strand and an A' strand were each linked via a Z linker, and examination was performed on base sequence editing efficiency in the same manner as in Example 1. An E strand having a length of 79-nt as an antisense strand (SEQ ID NO: 5) was used as a nucleic acid for editing, and linked to an A strand (SEQ ID NO: 6) or an A' strand (SEQ ID NO: 7) via a Z linker. Linking via a Z linker was performed according to description in WO 2012/074038. Irrespective of the presence or absence of a Z linker, ODNs were mixed to give identical molar concentrations. Specifically, a molar ratio of 1:1 as E strand:A strand was used for an n-TD and a Linker_(E+A) (a construct obtained by linking the 3' end of the E strand and the 5' end of the A strand together), a molar ratio of 1:1 as E strand:A' strand was used for a Linker_(E+A') (a construct obtained by linking the 3' end of the E strand and the 5' end of the A' strand together), and a molar ratio of 1:1:1 as E strand:A strand:A' strand was used for an n-TD', a Linker_(E+A)+A' (a construct obtained by adding the A' strand to the Linker_(E+A)), and a Linker_(E+A')+A (a construct obtained by adding the A strand to the Linker_(E+A')).

The nucleic acids for editing that were used (only those obtained by linking via a Z linker) are shown in the following.

Construct obtained by linking E strand and A strand together copGFP-79_AS_5'E3'_C8Ph_5'A3': obtained by linking the 3' end of SEQ ID NO: 5 and the 5' end of SEQ ID NO: 6 via a Z linker

5'-
CGTAGCCGCTGGGGTAGGTGCCGAAGTGGTAGAAGCCGTAGCCCATCACGTGGC
TCAGCAGGTAGGGGCTGAAGGTCAG-Z-
CTGACCTTCAGCCCCTACCTGCTGAGCCACGTGAT-3'

Construct obtained by linking E strand and A' strand together copGFP-79_AS_5'E3'_C8Ph_5'A'3': obtained by linking the 3' end of SEQ ID NO: 5 and the 5' end of SEQ ID NO: 7 via a Z linker

5'-
CGTAGCCGCTGGGGTAGGTGCCGAAGTGGTAGAAGCCGTAGCCCATCACGTGGC
TCAGCAGGTAGGGGCTGAAGGTCAG-Z-
CTCGTACTTCTCGATGCGGGTGTTGGTGTAGCCGCCGTTGTTGATGGCGTGCAGG
AAGGGGTTCT-3'

Fig. 24 shows the results. The Linker_(E+A) exhibited an effect similar to that in the case that the E strand and the A strand were mixed for preparation (n-TD). In contrast, the Linker_(E+A') gave editing efficiency almost equal to that with the E strand alone, and the A' strand linked via a Z linker did not have any influence.

### Example 16: Editing efficiency for nonsense mutations of causative genes reported for human diseases

Nonsense mutation sequences reported for eight human diseases were used as target sequences for gene editing (https://www.ncbi.nlm.nih.gov/snp/). Table 1 shows the disease names, the causative genes, and each sequence in the vicinity of the corresponding nonsense mutation.

**[Table 1]**

| Gene name | Disease name | ***target** |
|---|---|---|
| **DMD (C→T)** | Duchenne muscular dystrophy | AATATCATGGCTGGATTGCAACAAACCAACAGTGAAAAGATTCTCCTGAGCTGGGTC TGACAATCAACTCGTAATTATCCACAGGTTAATGTAATCAACTTCACCACCAGC |
| **WRN (C→T)** | Werner's syndrome | ACAATTGGCATGCACTTATCCCAAGCGGTGAAAGCTGGCTGCCCCCTTGATTTGGAG TGAGCAGGCCTGACTCCAGAGGTTCAGAAGATTATTGCTGATGTTATCCGAAAC |
| **HBB (G→T)** | Sickle-cell anemia | GAGAAGTCTGCCGTTACTGCCCTGTGGGGCAAGGTGAACGTGGATGAAGTTGGTGGT TAGGCCCTGGGCAGGCTGCTGGTGGTCTACCCTTGGACCCAGAGGTTCTTTGAG |
| **ADA (C→T)** | ADA deficiency | GACCTCACCCCAGACGAGGTGGTGGCCCTAGTGGCCAGGCCTGCAGGAGGGGAG TGAGACTTCGGGTCAAGGCCCGGTCCATCCTGTGCTGCATGCGCCACCAGCCC |
| **LCA (C→T)** | Leber's congenital amaurosis | TTTCCTTCCCAGGATCAGATGGCATCTCCTGAGGTTCCCATTGAAGCTGGCCAGTAT TGATCTAAGAGAAAACCTCCTCATGGGGGAGAAAGAAAGGAGAAGGAGCACCAG |
| **PRKN (T→G)** | Parkinson's syndrome | ACCTGCAGGCAGGCAACGCTCACCTTGACCCAGGGTCCATCTTGCTGGGATGATGTT TGAATTCCAAACCGGATGAGTGGTGAATGCCAATCCCCACACTGCCCTGGGACT |
| **BRAF (G→T)** | Malignant melanoma | TGGGAGATTCCTGATGGGCAGATTACAGTGGGACAAAGAATTGGATCTGGATCATTT TGAACAGTCTACAAGGGAAAGTGGCATGGTGATGTGGCAGTGAAAATGTTGAAT |
| **NRAS (C→T)** | Malignant melanoma | AAACAAGTGGTTATAGATGGTGAAACCTGTTTGTTGGACATACTGGATACAGCTGGA TAAGAAGAGTACAGTGCCATGAGAGACCAATACATGAGGACAGGCGAAGGCTTC |

In Table 1, each underline indicates a stop codon generated by a nonsense mutation, and the hatched character therein indicates the nonsense mutation. GC are represented with outlined characters. The pieces of gene information are registered in the website of the National Center for Biotechnology Information (https://www.ncbi.nlm.nih.gov/snp/) under the following numbers.
DMD: rs128626235
WRN: rs121908446
HBB: rs33950507
ADA: rs780014431
LCA: rs780667159
PRKN: rs1562485799
BRAF: rs121913357
NRAS: rs1562485799

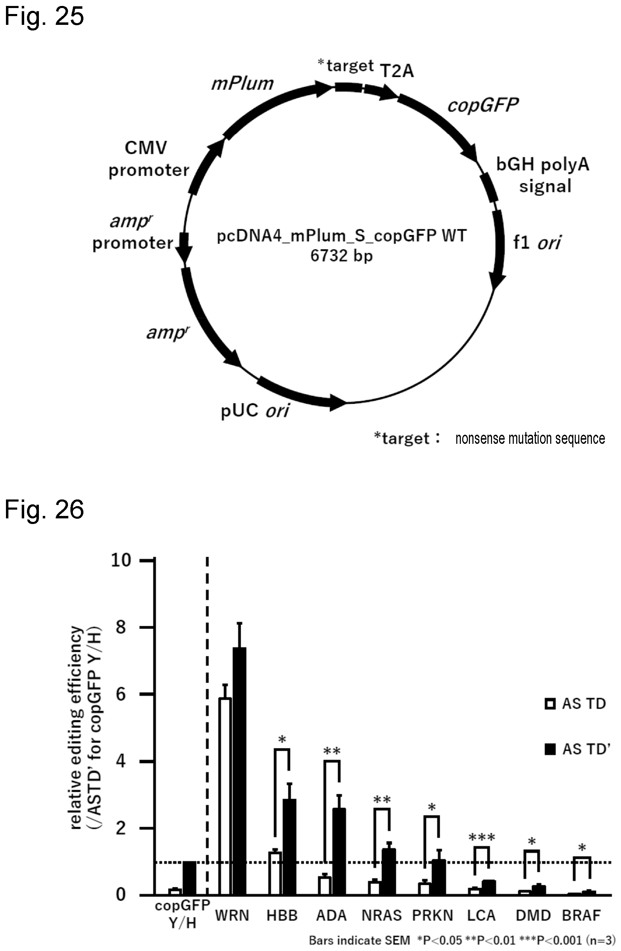

For each of these nonsense mutations, 55-nt before and after the nonsense mutation was inserted between mPlum gene for a red fluorescent protein and a T2A sequence in an expression vector to produce pcDNA4_mPlum_S_copGFP WT (Fig. 25) such that the nonsense mutation was to be edited by an n-TD or an n-TD' and as a result copGFP gene was to be translated.

The n-TD' was prepared with an antisense (AS) E strand having a length of 79-nt, an A strand of 35-nt having a sequence complementary to the 3'-end portion of the E strand, and an A' strand of 65-nt extending from the 5' end of the E strand to give a molar ratio of E:A:A' = 10:20:30 in introducing into cells as the molar ratio of the plasmid DNA was taken as 1.

The n-TD was prepared without use of the A' strand to give a molar ratio of E:A = 10:20 in introducing into cells as the molar ratio of the plasmid DNA was taken as 1.

As in Example 1, pcDNA4_mPlum_T2A_copGFP Y/H having fluorescence-deficient copGFP incorporated therein, an E strand (SEQ ID NO: 5), an A strand (SEQ ID NO: 6), and an A' strand (SEQ ID NO: 7) were used as controls to measure editing efficiency.

Fig. 26 and Table 2 show the results. For any of the sequences, the AS n-TD' gave higher gene editing efficiency than the AS n-TD (Fig. 26). For gene sequences for each of which the AS n-TD' gave higher gene editing efficiency, the GC contents of the E strand parts tended to be high, and this suggested that the GC content of an E strand had influence on editing efficiency.

**[Table 2]**

| gene | relative editing efficiency | GC% of E strand |
|---|---|---|
| copGFP Y/H | 1.00 | 63.3% |
| WRN | 35.3 | 55.7% |
| HBB | 7.24 | 64.6% |
| ADA | 3.16 | 69.6% |
| NRAS | 2.47 | 45.6% |
| PRKN | 2.09 | 49.4% |
| LCA | 1.13 | 49.4% |
| DMD | 0.76 | 40.5% |
| BRAF | 0.30 | 45.6% |

### Example 17-1: Influence of addition of oligodeoxynucleotide (ODN) having length of 35 nucleotides and disposed differently from E strand for target region on base sequence editing efficiency by E strand

Examination was performed on change in editing efficiency by addition of an ODN having a length of 35 nucleotides and disposed differently from an E strand for a target region in a base sequence editing method with an E strand by means of a green fluorescent protein.

copGFP gene, which encodes copepod-derived green fluorescent protein, was used as a target gene. A plasmid DNA having a mutated copGFP gene (SEQ ID NO: 1) obtained by changing the base sequence TAC corresponding to Tyr in Gly-Tyr-Gly constituting the fluorophore to CAC (encoding His), an E strand, and any of different ODNs were co-introduced into human U2OS cells, and the proportions of cells for which green fluorescence was observed through a fluorescence microscope were compared. We used, as an E strand, a single-stranded polynucleotide having a length of 79 nucleotides and having a sequence identical to a part of the antisense strand of the target gene with the sequence encoding the fluorophore being wild type (AS_E strand, SEQ ID NO: 5). We used ODNs each having a length of 35 nucleotides, having sequence identity with the antisense strand or sense strand, and positioned differently from the E strand for the target region. The sequences of the ODNs used are shown in the following.
copGFP-153-79b-S-35TDup-ODN(AS_Up_O): SEQ ID NO: 140
5'-GGCGCCTTTGGTGCTCTTCATCTTGTTGGTCATGC -3'
copGFP-153-79b-S-35TD5'-ODN(AS_Up_T): SEQ ID NO: 141
5'-ATCACGTGGCTCAGCAGGTAGGGGCTGAAGGTCAG -3'
copGFP-153-79b-S-35TDcn-ODN(AS_Cnt_Y): SEQ ID NO: 142
5'-GAAGTGGTAGAAGCCGTAGCCCATCACGTGGCTCA -3'
copGFP-153-79b-S-35TDcnYH-ODN(AS_Cnt_H): SEQ ID NO: 143
5'-GAAGTGGTAGAAGCCGTGGCCCATCACGTGGCTCA -3'
copGFP-153-79b-S-35TD3'-ODN(AS_Dwn_T): SEQ ID NO: 144
5'-CGTAGCCGCTGGGGTAGGTGCCGAAGTGGTAGAAG -3'
copGFP-153-79b-S-35TDdown-ODN(AS_Dwn_O): SEQ ID NO: 145
5'-GCCGCCGTTGTTGATGGCGTGCAGGAAGGGGTTCT -3'
copGFP-153-79b-AS-35TDup-ODN(S_Up_O): SEQ ID NO: 146
5'-GCATGACCAACAAGATGAAGAGCACCAAAGGCGCC -3'
copGFP-153-79b-AS-35TD3'-ODN (S_Up_T, A strand for AS_E strand): SEQ ID NO: 6
5'-CTGACCTTCAGCCCCTACCTGCTGAGCCACGTGAT -3'
copGFP-153-79b-AS-35TDcn-ODN(S_Cnt_Y): SEQ ID NO: 147
5'-TGAGCCACGTGATGGGCTACGGCTTCTACCACTTC -3'
copGFP-153-79b-AS-35TDcnYH-ODN(S_Cnt_H): SEQ ID NO: 148
5'-TGAGCCACGTGATGGGCCACGGCTTCTACCACTTC -3'
copGFP-153-79b-AS-35TD5'-ODN(S_Dwn_T): SEQ ID NO: 149
5'-CTTCTACCACTTCGGCACCTACCCCAGCGGCTACG -3'
copGFP-153-79b-AS-35TDdown-ODN(S_Dwn_O): SEQ ID NO: 150
5'-AGAACCCCTTCCTGCACGCCATCAACAACGGCGGC -3'

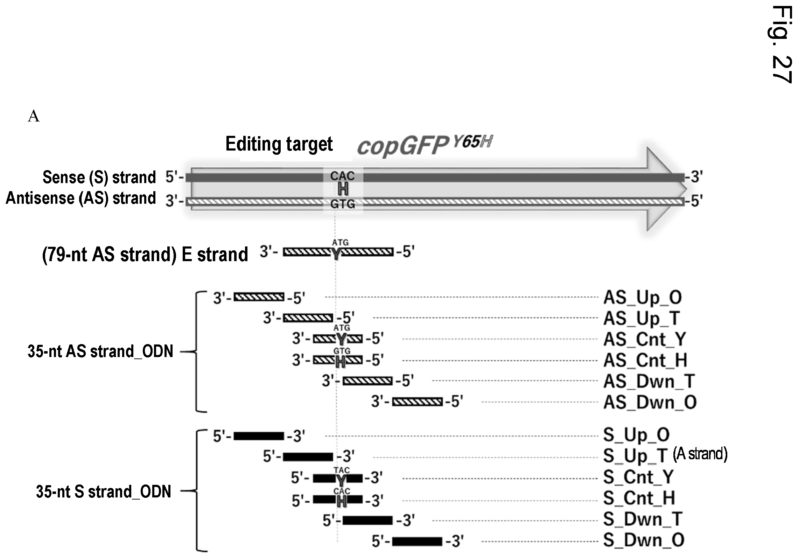

Fig. 27A shows the arrangement of the E strand and the ODNs each having a length of 35 nucleotides relative to the target region. The names of ODNs each having a length of 35 nucleotides and having sequence identity with the antisense strand of copGFP gene contain AS, and the names of those having sequence identity with the sense strand of copGFP gene contain S. The names of ODNs each having a length of 35 nucleotides and positioned upstream of copGFP gene from the base for editing (the mutation for editing in the E strand) contain Up (upstream), the names of those positioned downstream of copGFP gene contain Dwn (downstream), and the names of those positioned at the base for editing contain Cnt (center). The names of ODNs each having a length of 35 nucleotides with the base sequences each being adjacent to the base sequence of the E strand without overlapping contain O (outside), and the names of those with the base sequences each being overlapping with an end of the E strand contain T (terminal). The names of ODNs each positioned at the base for editing and containing the sequence 5'-TAC-3' (sense strand), which encodes Tyr in the fluorophore of fluorescent copGFP, or the complementary strand 5'-GTA-3' (antisense strand) contain Y, and the names of those containing the sequence 5'-CAC-3' (sense strand), which encodes His in a mutated copGFP, and the complementary strand 5'-GTG-3' (antisense strand) contain H. S_Up_T is an ODN identical to an A strand of a TD (SEQ ID NO: 6).

The specific experimental method is as follows.

For each of the ODNs having a length of 35 nucleotides and disposed differently from the E strand for the target region, solutions of the E strand and the ODN were mixed together at a ratio of 1:10, and the resultant was left to stand at room temperature for 1 hour to prepare a nucleic acid for editing. An mPlum-T2A-copGFP (Y/H) expression vector plasmid (pDNA; 30.0 ng, 7.33 fmol) containing a mutated copGFP gene for editing efficiency analysis and the nucleic acid for editing prepared were mixed together in such a manner that the molar ratio between the pDNA and the E strand reached 1:10, and the resultant was co-introduced with Lipofectamine 3000 into U2OS cells seeded in each well of a 384-well plate (8 × 10³ cells) and then cultured overnight. After 4 hours, medium exchange was performed, and culture was performed for 48 hours. Nuclear staining was performed with Hoechst 33342, the resultant was fixed with 4% paraformaldehyde, and a fluorescence microscope image was acquired.

Editing efficiency was calculated by the following method.

Fluorescence microscope images were acquired by photographing the wells of a 384-well plate with an Opera Phenix. From data on the fluorescence intensity of Hoechst 33342 in all wells, cell nuclei were identified by using the image analysis software Harmony, and median/cumulative total of values at the pixels was calculated for each of fluorescence intensities at fluorescence wavelengths of Hoechst 33342, mPlum, and copGFP in the cell nuclei. From the fluorescence intensity data for Hoechst 33342 in all wells, cell nuclei having average fluorescence intensity and form were selected as analysis targets for editing efficiency. From fluorescence intensity data for mPlum and copGFP in cell nuclei in the well having only the mPlum-T2A-copGFP (Y/H) expression vector plasmid introduced therein, a threshold line between cells negative for the fluorescence of copGFP (cells not containing the pDNA having the edited fluorescent copGFP gene) and cells positive for the fluorescence of copGFP (cells containing the pDNA having the fluorescent copGFP gene) was determined. From the fluorescence intensities of mPlum in cell nuclei as analysis targets in all wells, a threshold between cells positive for mPlum (cells containing the pDNA) and cells negative for mPlum (cells not containing the pDNA) was determined. For each well, the copGFP-positive cell count was divided by the mPlum-positive cell count, and the resulting value was determined as the editing efficiency.

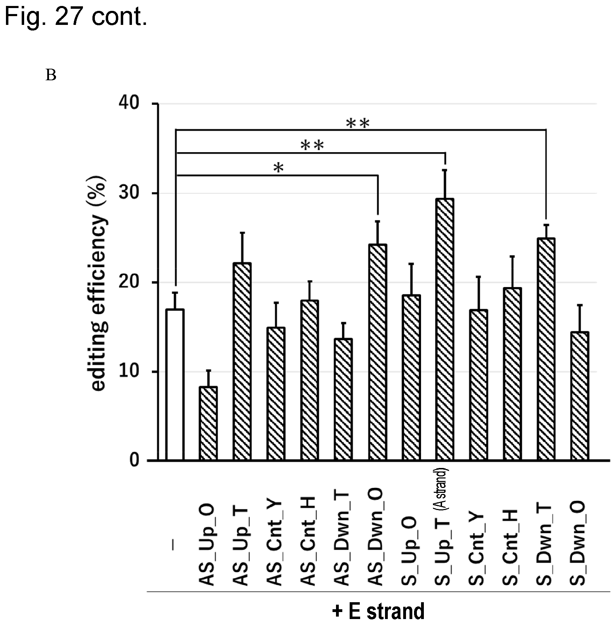

Fig. 27B shows the results. As compared with the editing efficiency for only the E strand (-), addition of S_Up_T (A strand), which is the ODN of a strand complementary to the 3'-end portion of the E strand, gave the most enhanced editing efficiency in the 5'-tailed duplex (TD). Addition of the ODN of a strand complementary to the 5'-end portion of the E strand (S_Dwn_T) also gave significantly enhanced editing efficiency. Moreover, addition of an ODN adjacent to the 5' end of the E strand (AS_Dwn_O) also gave significantly enhanced editing efficiency.

Example 17-2: Influence of difference in amounts of introduction of E strand, A strand, and ODN adjacent to 5' end of E strand (AS_Dwn_O, A' strand having length of 35 nucleotides) on base sequence editing efficiency

Examination was performed on the influence of change in the total amount of introduction of an E strand, an A strand, and an ODN adjacent to the 5' end of the E strand (AS_Dwn_O, an A' strand having a length of 35 nucleotides) for a target plasmid on editing efficiency in a base sequence editing method with an E strand by means of a green fluorescent protein.

For a target plasmid DNA, an E strand, an A strand, and an A' strand having a length of 35 nucleotides were introduced into cells in different combinations at different doses, and the proportions of cells for which green fluorescence was observed through a fluorescence microscope were compared.

The specific experimental method is as follows.

Solutions of an E strand (SEQ ID NO: 5), an A strand (SEQ ID NO: 6), and an A' strand having a length of 35 nucleotides (AS_Dwn_O, SEQ ID NO: 145) were mixed together at a molar ratio of 1:0:0, 1:10:0, 1:0:10, or 1:5:5, and the resultant was left to stand at room temperature for 1 hour to prepare a nucleic acid for editing. An mPlum-T2A-copGFP (Y/H) expression vector plasmid (pDNA; 30.0 ng, 7.33 fmol) containing a mutated copGFP gene for editing efficiency analysis and the nucleic acid for editing prepared were mixed together in such a manner that the molar ratio between the pDNA and the E strand reached 1:10, 1:1, or 1:0.1, and the resultant was co-introduced with Lipofectamine 3000 into U2OS cells seeded in each well of a 384-well plate (8 × 10³ cells) and then cultured overnight. After 4 hours, medium exchange was performed, and culture was performed for 48 hours. Nuclear staining was performed with Hoechst 33342, the resultant was fixed with 4% paraformaldehyde, and a fluorescence microscope image was acquired.

Editing efficiency was calculated in the same manner as in Example 17-1.

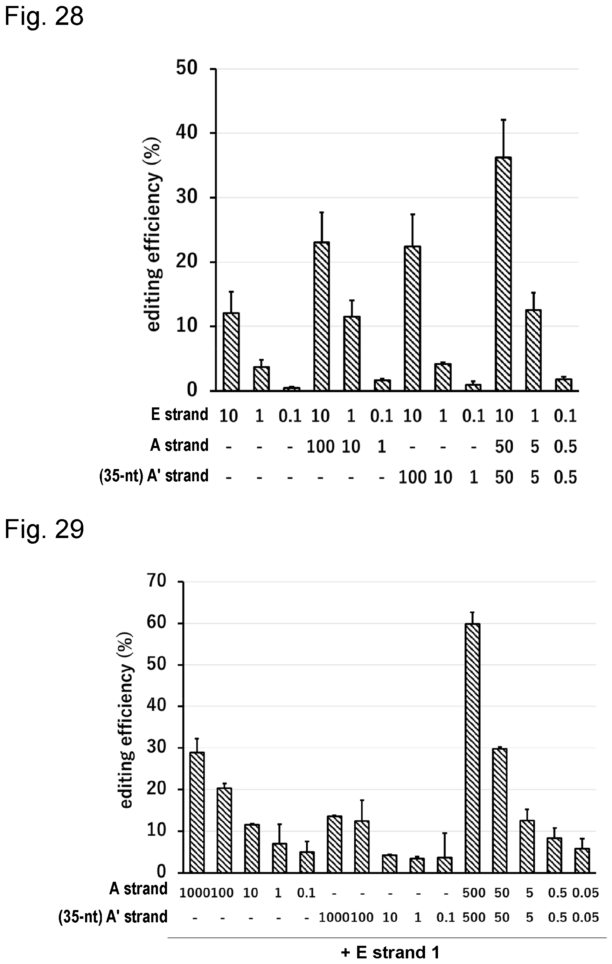

Fig. 28 shows the results. Increased editing efficiency was obtained in a manner depending on the amount of the E strand. As compared with the editing efficiency obtained using only the E strand, addition of the A strand or the A' strand having a length of 35 nucleotides gave enhanced editing efficiency. As compared with the editing efficiency obtained by addition of the A strand or A' strand alone, addition of the two gave much enhanced editing efficiency.

### Example 17-3: Influence of amounts of introduction of A strand and A' strand having length of 35 nucleotides to E strand on base sequence editing efficiency

Examination was performed on the influence of change in amounts of introduction of an A strand and an A' strand having a length of 35 nucleotides with a constant dose of an E strand for a target plasmid on editing efficiency in a base sequence editing method with an E strand by means of a green fluorescent protein.

With the molar ratio between a target plasmid DNA and an E strand set to 1:1, an A strand and an A' strand having a length of 35 nucleotides were introduced into cells at different doses, and the proportions of cells for which green fluorescence was observed through a fluorescence microscope were compared.

The specific experimental method is as follows.

Mixed together were 1 µM solutions of an E strand (SEQ ID NO: 5), an A strand (SEQ ID NO: 6), and an A' strand having a length of 35 nucleotides (AS_Dwn_O, SEQ ID NO: 145) at 1:0.1, 1:1, 1:10, 1:100, or 1:1000 as a ratio between the E strand and the A strand, the A' strand having a length of 35 nucleotides, or a mixture of the A strand and the A' strand having a length of 35 nucleotides (molar ratio: 1:1), and the resultant was left to stand at room temperature for 1 hour to prepare a nucleic acid for editing. An mPlum-T2A-copGFP (Y/H) expression vector plasmid (pDNA; 30.0 ng, 7.33 fmol) containing a mutated copGFP gene for editing efficiency analysis and the nucleic acid for editing prepared were mixed together in such a manner that the molar ratio between the pDNA and the E strand reached 1:1, and the resultant was co-introduced with Lipofectamine 3000 into U2OS cells seeded in each well of a 384-well plate (8 × 10³ cells) and then cultured overnight. After 4 hours, medium exchange was performed, and culture was performed for 48 hours. Nuclear staining was performed with Hoechst 33342, the resultant was fixed with 4% paraformaldehyde, and a fluorescence microscope image was acquired.

Editing efficiency was calculated in the same manner as in Example 17-1.

Fig. 29 shows the results. Addition of the A strand or the A' strand having a length of 35 nucleotides with the molar ratio between the pDNA and the E strand set to 1:1 gave more enhanced editing efficiency as the doses of them increased. Comparison of the A strand and the A' strand having a length of 35 nucleotides for addition at the same dose found that addition of the A strand gave more enhanced editing efficiency. Addition of the A strand and the A' strand in a mixture at 1:1 gave more enhanced editing efficiency than addition of any of them alone at the same dose gave.

### Example 17-4: Influence of introduction ratio between A strand and A' strand having length of 35 nucleotides to E strand on base sequence editing efficiency

Examination was performed on the influence of change in the dose ratio between an A strand and an A' strand having a length of 35 nucleotides with a constant dose of an E strand for a target plasmid on editing efficiency in a base sequence editing method with an E strand by means of a green fluorescent protein.

The molar ratio among a target plasmid DNA, an E strand, and the total of an A strand and an A' strand having a length of 35 nucleotides was set to 1:1:100, and the A strand and the A' strand having a length of 35 nucleotides were introduced into cells at different molar ratios, and the proportions of cells for which green fluorescence was observed through a fluorescence microscope were compared.

The specific experimental method is as follows.

Mixed together were 1 µM solutions of an E strand (SEQ ID NO: 5), an A strand (SEQ ID NO: 6), and an A' strand having a length of 35 nucleotides (AS_Dwn_O, SEQ ID NO: 145) at a molar ratio of 100:0, 90:10, 80:20, 70:30, 60:40, 50:50, 40:60, 30:70, 20:80, 10:90, or 0:100 between the A strand and the A' strand having a length of 35 nucleotides as the amount in moles of the E strand was taken as 1, and the resultant was left to stand at room temperature for 1 hour to prepare a nucleic acid for editing. An mPlum-T2A-copGFP (Y/H) expression vector plasmid (pDNA; 30.0 ng, 7.33 fmol) containing a mutated copGFP gene for editing efficiency analysis and the nucleic acid for editing prepared were mixed together in such a manner that the molar ratio between the pDNA and the E strand reached 1:1, and the resultant was co-introduced with Lipofectamine 3000 into U2OS cells seeded in each well of a 384-well plate (8 × 10³ cells) and then cultured overnight. After 4 hours, medium exchange was performed, and culture was performed for 48 hours. Nuclear staining was performed with Hoechst 33342, the resultant was fixed with 4% paraformaldehyde, and a fluorescence microscope image was acquired.

Editing efficiency was calculated in the same manner as in Example 17-1.

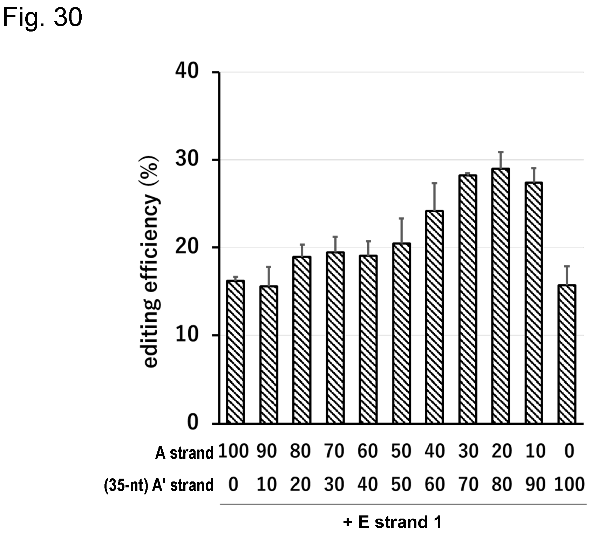

Fig. 30 shows the results. Found was the influence of change in the dose ratio between the A strand and the A' strand having a length of 35 nucleotides on editing efficiency. A tendency of more enhanced editing efficiency was found when the dose of the A' strand having a length of 35 nucleotides was higher than that of the A strand.

### Example 17-5: Influence of difference in length of ODN adjacent to 5' end of E strand (A' strand) on base sequence editing efficiency

Editing efficiency when an ODN adjacent to the 5' end of an E strand (A' strand; length: 15 to 75 nucleotides) was added was analyzed in a base sequence editing method with an E strand by means of a green fluorescent protein, and examination was performed on the influence of the length of the A' strand on editing efficiency.

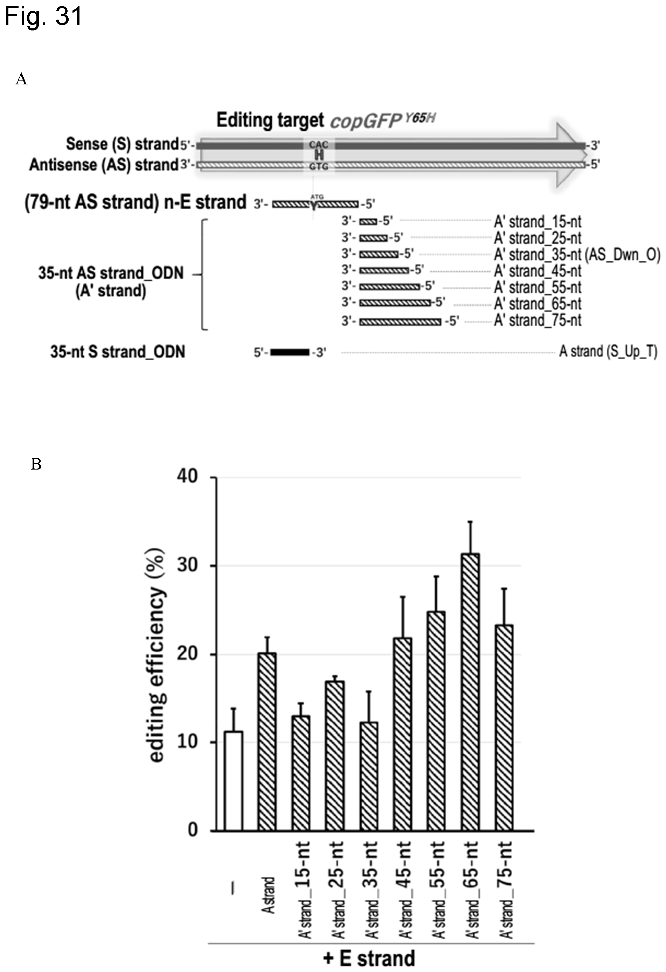

An A strand (SEQ ID NO: 6) or an A' strand having a length of 15 nucleotides (A' strand_15-nt), 25 nucleotides (A' strand_25-nt), 35 nucleotides (A' strand_35-nt, SEQ ID NO: X11), 45 nucleotides (A' strand_45-nt), 55 nucleotides (A' strand_55-nt), 65 nucleotides (A' strand_65-nt), or 75 nucleotides (A' strand_75-nt) was added to a target plasmid DNA and an E strand (SEQ ID NO: 5), the resultant was introduced into cells, and the proportions of cells for which green fluorescence was observed through a fluorescence microscope were compared. The sequences of the ODNs used are shown below. Fig. 31A shows the arrangement of the E strand and the ODNs relative to the target region.
copGFP-153-79b-AS-35TD3'-ODN (A strand): SEQ ID NO: 6
5'-CTGACCTTCAGCCCCTACCTGCTGAGCCACGTGAT -3'
   copGFP_A_prime15 (A' strand_15-nt): SEQ ID NO: 151
5'-GCAGGAAGGGGTTCT -3'
   copGFP_A_prime25 (A' strand_25-nt): SEQ ID NO: 152
5'-TTGATGGCGTGCAGGAAGGGGTTCT -3'
   copGFP_A_prime35 (copGFP-153-79b-S-35TDdown-ODN) (A' strand_35-nt): SEQ ID NO: 145
5'-GCCGCCGTTGTTGATGGCGTGCAGGAAGGGGTTCT -3'
   copGFP_A_prime45 (A' strand_45-nt): SEQ ID NO: 153
5'-TGTTGGTGTAGCCGCCGTTGTTGATGGCGTGCAGGAAGGGGTTCT -3'
   copGFP_A_prime55 (A' strand_55-nt): SEQ ID NO: 154 
5'-
TCGATGCGGGTGTTGGTGTAGCCGCCGTTGTTGATGGCGTGCAGGAAGGGGTTCT
-3'
copGFP_A_prime65 (A' strand_65-nt) (A' strand): SEQ ID NO: 7 
5'-
CTCGTACTTCTCGATGCGGGTGTTGGTGTAGCCGCCGTTGTTGATGGCGTGCAGG
AAGGGGTTCT -3'
copGFP_A_prime75 (A' strand_75-nt): SEQ ID NO: 155

5'-
CGCCGCCGTCCTCGTACTTCTCGATGCGGGTGTTGGTGTAGCCGCCGTTGTTGAT
GGCGTGCAGGAAGGGGTTCT -3'

The specific experimental method is as follows.

Mixed together were 1 µM solutions of the E strand and the A strand or any of the A' strands of different lengths at a molar ratio of 1:10, and the resultant was left to stand at room temperature for 1 hour to prepare a nucleic acid for editing. An mPlum-T2A-copGFP (Y/H) expression vector plasmid (pDNA; 30.0 ng, 7.33 fmol) containing a mutated copGFP gene for editing efficiency analysis and the nucleic acid for editing prepared were mixed together in such a manner that the molar ratio between the pDNA and the E strand reached 1:10, and the resultant was co-introduced with Lipofectamine 3000 into U2OS cells seeded in each well of a 384-well plate (8 × 10³ cells) and then cultured overnight. After 4 hours, medium exchange was performed, and culture was performed for 48 hours. Nuclear staining was performed with Hoechst 33342, the resultant was fixed with 4% paraformaldehyde, and a fluorescence microscope image was acquired.

Editing efficiency was calculated in the same manner as in Example 17-1.

Fig. 31B shows the results. At the same dose in moles, the editing efficiency obtained by addition of an A' strand having a length of 45 nucleotides or more was higher than that obtained using a 5'-tailed duplex (TD) obtained by adding the A strand to the E strand, and addition of the A' strand having a length of 65 nucleotides gave the highest editing efficiency.

### Example 17-6: Influence of difference in length of A' strand on base sequence editing efficiency

Editing efficiency obtained by addition of an A' strand having a length of 15 to 75 nucleotides was analyzed in a base sequence editing method with an E strand or a TD (E strand and A strand) by means of a green fluorescent protein, and examination was performed on the influence of the length of an A' strand on editing efficiency.

An A' strand having a length of 15 nucleotides (A' strand_15-nt), 25 nucleotides (A' strand_25-nt), 35 nucleotides (A' strand_35-nt, SEQ ID NO: X11), 45 nucleotides (A' strand_45-nt), 55 nucleotides (A' strand_55-nt), 65 nucleotides (A' strand_65-nt), or 75 nucleotides (A' strand_75-nt) was added to a target plasmid DNA and an E strand or a TD, the resultant was introduced into cells, and the proportions of cells for which green fluorescence was observed through a fluorescence microscope were compared. The ODN used were the same as those used in Example 17-5 (SEQ ID NOs: 7, 145, and 151 to 155).

The specific experimental method is as follows.

For each of A' strands having different lengths A' strand (SEQ ID NOs: 145 and 151 to 155), solutions of an E strand (SEQ ID NO: 5) and an A strand (SEQ ID NO: 6) or solutions of an E strand (SEQ ID NO: 5) and the A' strand were mixed together at an E strand:A strand or E strand:A' strand molar ratio of 1:10, or solutions of an E strand (SEQ ID NO: 5), an A strand (SEQ ID NO: 6), and the A' strand were mixed together at an E strand:A strand:A' strand molar ratio of 1:5:5, and the resultant was left to stand at room temperature for 1 hour to prepare a nucleic acid for editing. An mPlum-T2A-copGFP (Y/H) expression vector plasmid (pDNA; 30.0 ng, 7.33 fmol) containing a mutated copGFP gene for editing efficiency analysis and the nucleic acid for editing prepared were mixed together in such a manner that the molar ratio between the pDNA and the E strand reached 1:10, and the resultant was co-introduced with Lipofectamine 3000 into U2OS cells seeded in each well of a 384-well plate (8 × 10³ cells) and then cultured overnight. After 4 hours, medium exchange was performed, and culture was performed for 48 hours. Nuclear staining was performed with Hoechst 33342, the resultant was fixed with 4% paraformaldehyde, and a fluorescence microscope image was acquired.

Editing efficiency was calculated in the same manner as in Example 17-1.

Fig. 32 shows the results. The editing efficiency obtained by addition of an A' strand having a length of 35 nucleotides or more to the TD was higher than that obtained using the TD.

### Example 17-7: Establishment of method for calculating quantitative editing efficiency with cumulative total of fluorescence intensity in cells

Each editing efficiency in Examples 17-1 and 17-2 was calculated as a value obtained by dividing copGFP-positive cell count by mPlum-positive cell count. In this calculation method, the copy number of a target plasmid DNA introduced into cells is not reflected. In addition, in the case that a majority of cell nuclei as analysis targets have been determined as copGFP-positive cells by virtue of enhanced editing efficiency (e.g., Example 17-6), it is difficult to compare editing efficiencies. In view of this, examination was performed on whether editing efficiency reflecting the copy number of a target plasmid DNA introduced was successfully calculated from the sum of the cumulative totals of the fluorescence intensities of copGFP and mPlum in cell nuclei as analysis targets in different wells.

The specific experimental method is as follows.

An mPlum-T2A-copGFP (normal) expression vector plasmid and an mPlum-T2A-copGFP (Y/H) expression vector plasmid were mixed together at a molar ratio of any of 1:0 (proportion of normal copGFP: 100%), 2:1 (66.7%), 5:5 (50.0%), 1:2 (33.3%), 1:3 (25.0%), 1:7 (12.5%), 1:15 (6.25%), 1:31 (3.13%), 1:63 (1.56%), 1:127 (0.781%), 1:255 (0.391%), 1:511 (0.195%), 1:1023 (0.0977%), 1:2047 (0.0488%), 1:4095 (0.244%), and 0:1 (0%). Into U2OS cells seeded in each well of a 384-well plate (8 × 10³ cells) and then cultured overnight, 30.0 ng of the plasmid mixture was introduced with Lipofectamine 3000. After 4 hours, medium exchange was performed, and culture was performed for 48 hours. Nuclear staining was performed with Hoechst 33342, the resultant was fixed with 4% paraformaldehyde, and a fluorescence microscope image was acquired.

Analytical values of fluorescence intensity for the wells were determined from the fluorescence microscope images in the following manner.

Fluorescence microscope images were acquired by photographing the wells of a 384-well plate with an Opera Phenix. From data on the fluorescence intensity of Hoechst 33342 for all wells, cell nuclei were identified by using the image analysis software Harmony, and median/cumulative total of values at the pixels was calculated for each of fluorescence intensities at fluorescence wavelengths of Hoechst 33342, mPlum, and copGFP in the cell nuclei. From the fluorescence intensity data for Hoechst 33342 in all wells, cell nuclei having average fluorescence intensity and form were selected as analysis targets. From fluorescence intensity data for mPlum and copGFP in cell nuclei in the well having only the mPlum-T2A-copGFP (Y/H) expression vector plasmid introduced therein, a threshold line between cells negative for the fluorescence of copGFP (cells not containing the pDNA having the fluorescent copGFP gene) and cells positive for the fluorescence of copGFP (cells containing the pDNA having the fluorescent copGFP gene) was determined. From the fluorescence intensities of mPlum in cell nuclei as analysis targets in all wells, a threshold between cells positive for mPlum (cells containing the pDNA) and cells negative for mPlum (cells not containing the pDNA) was determined. The cumulative total of the fluorescence intensity of copGFP in copGFP-positive cells and the cumulative total of the fluorescence intensity of mPlum in mPlum-positive cells were determined for each of the wells having the mPlum-T2A-copGFP (normal) expression vector plasmid and the mPlum-T2A-copGFP (Y/H) expression vector plasmid introduced at different proportions. The cumulative total for copGFP in each well was corrected with the fluorescence intensity data for the well having only the mPlum-T2A-copGFP (Y/H) expression vector plasmid introduced, and the resulting value was used as the analytical value.

Fig. 33 shows the results, in which the analytical values derived from the fluorescence intensity data were plotted on the ordinate, and the proportions (%) of the mPlum-T2A-copGFP (normal) expression vector plasmid in the plasmids introduced were plotted on the abscissa. A regression line was determined with the analytical values up to 50% of the copGFP (normal) expression vector, and the coefficient of determination for the linear relationship, R², was found to be 0.9961. From these results, analytical values for fluorescence intensity data obtained by using the present method are expected to allow comparison of editing efficiencies, in particular, accurate comparison of editing efficiencies up to 50%.

### Example 17-8: Influence of difference in length of A' strand on base sequence editing efficiency

For the results in Example 17-6, reanalysis was performed by the method in Example 17-7, and comparison was made on the influence of the length of an A' strand on editing efficiency.

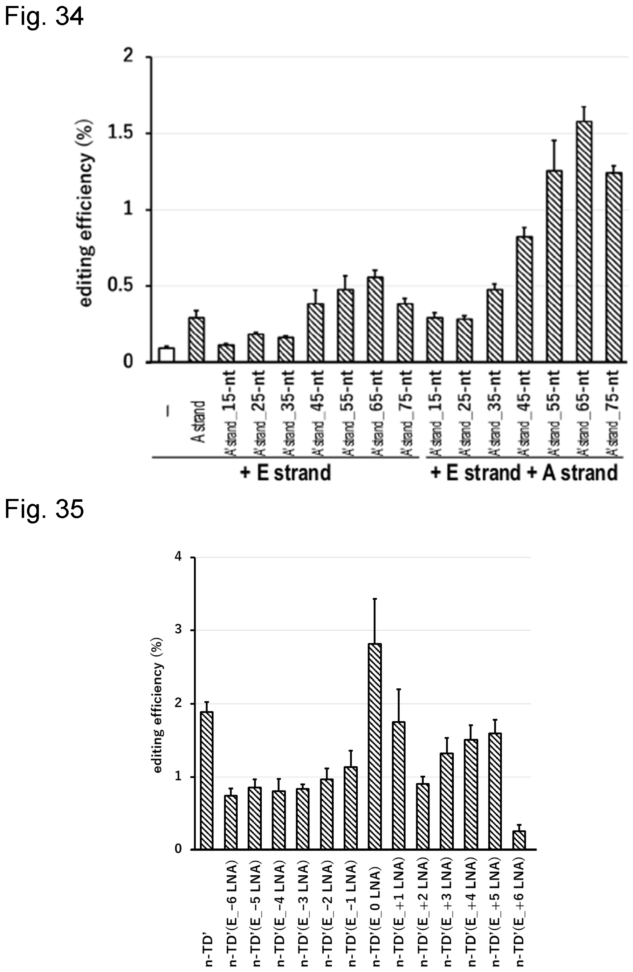

Fig. 34 shows the results. As with the case of the results shown in Fig. 32, addition of an A' strand having a length of 35 nucleotides or more to the TD was confirmed to give editing efficiency higher than that obtained using the TD. Addition of an A' strand having a length of 65 nucleotides to the TD gave the highest editing efficiency.

### Example 18-1: Influence of LNA modification in E strand on base sequence editing efficiency - Part (2)

Examination was performed in the same manner as in Example 1 on base sequence editing efficiency when an LNA modification was introduced at one position around the base for editing in an E strand.

In Example 3 (3-6), examination was performed on editing efficiency when an LNA modification was introduced at specific positions symmetric with respect to the base for editing. Introduction of LNA modifications at six positions of -5, -3, -1, +1, +3, and +5 (the position of the base for editing in an E strand was defined as 0, and the position of a base 5' to position 0 was specified with a minus sign (-) and the position of a base 3' to position 0 was specified with a plus sign (+)) gave the most enhanced editing efficiency (Fig. 11 and Fig. 12).

The nucleic acids for editing that were used are shown in the following (having an LNA modification on the nucleotide at each underlined part).

E strand
copGFP-153-79b-AS (AS_E strand): SEQ ID NO: 5 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-153-79b-AS-35TD3'-ODN (A strand): SEQ ID NO: 6
   5'-CTGACCTTCAGCCCCTACCTGCTGAGCCACGTGAT -3'
copGFP-153-79b-AS-65TDdown-ODN (alias: copGFP_A_prime65) (A' strand for AS_E strand): SEQ ID NO: 7 
5'-
CTCGTACTTCTCGATGCGGGTGTTGGTGTAGCCGCCGTTGTTGATGGCGTGCAGG
AAGGGGTTCT -3'
copGFP-79_AS_E LNA0: SEQ ID NO: 46 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA+1: SEQ ID NO: 156 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA+2: SEQ ID NO: 157 
5'-

CGTAGCCGCTGGGGTAGGTGCCGAAGTGGTAGAAGCCGTAGCCCATCACGTGGC

TCAGCAGGTAGGGGCTGAAGGTCAG -3'
copGFP-79_AS_E LNA+3: SEQ ID NO: 158 
5'-

CGTAGCCGCTGGGGTAGGTGCCGAAGTGGTAGAAGCCGTAGCCCATCACGTGGC

TCAGCAGGTAGGGGCTGAAGGTCAG -3'
copGFP-79_AS_E LNA+4: SEQ ID NO: 159 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA+5: SEQ ID NO: 160 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA+6: SEQ ID NO: 161 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-1: SEQ ID NO: 162 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-2: SEQ ID NO: 163 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-3: SEQ ID NO: 164 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-4: SEQ ID NO: 165 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-5: SEQ ID NO: 166 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-6: SEQ ID NO: 167

5'-

CGTAGCCGCTGGGGTAGGTGCCGAAGTGGTAGAAGCCGTAGCCCATCACGTGGC

TCAGCAGGTAGGGGCTGAAGGTCAG -3'

Fig. 35 shows the results. For introduction of an LNA modification at one position, only the case of introducing an LAN modification at the position of the base for editing (0) resulted in enhanced editing efficiency. The same results were obtained in editing an E strand alone.

### Example 18-2: Influence of LNA modification in E strand on base sequence editing efficiency - Part (3)

Examination was performed in the same manner as in Example 1 on base sequence editing efficiency when an LNA modification was introduced at two positions symmetric with respect to the position of the base for editing (0) as the center in an E strand.

The nucleic acids for editing that were used are shown in the following (having an LNA modification on the nucleotide at each underlined part).

E strand
copGFP-153-79b-AS (AS_E strand): SEQ ID NO: 5 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-153-79b-AS-35TD3'-ODN (A strand): SEQ ID NO: 6
   5'-CTGACCTTCAGCCCCTACCTGCTGAGCCACGTGAT -3'
copGFP-153-79b-AS-65TDdown-ODN (alias: copGFP_A_prime65) (A' strand for AS_E strand): SEQ ID NO: 7 
5'-
CTCGTACTTCTCGATGCGGGTGTTGGTGTAGCCGCCGTTGTTGATGGCGTGCAGG
AAGGGGTTCT -3'
copGFP-79_AS_E LNA0: SEQ ID NO: 46 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-1+1: SEQ ID NO: 48 
5'-

CGTAGCCGCTGGGGTAGGTGCCGAAGTGGTAGAAGCCGTAGCCCATCACGTGGC

TCAGCAGGTAGGGGCTGAAGGTCAG -3'
copGFP-79_AS_E LNA-2+2: SEQ ID NO: 168 
5'-

CGTAGCCGCTGGGGTAGGTGCCGAAGTGGTAGAAGCCGTAGCCCATCACGTGGC

TCAGCAGGTAGGGGCTGAAGGTCAG -3'
copGFP-79_AS_E LNA-3+3: SEQ ID NO: 169 
5'-

CGTAGCCGCTGGGGTAGGTGCCGAAGTGGTAGAAGCCGTAGCCCATCACGTGGC

TCAGCAGGTAGGGGCTGAAGGTCAG -3'
copGFP-79_AS_E LNA-4+4: SEQ ID NO: 170 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-5+5: SEQ ID NO: 171 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-6+6: SEQ ID NO: 172

5'-
CGTAGCCGCTGGGGTAGGTGCCGAAGTGGTAGAAGCCGTAGCCCATCACGTGGC
TCAGCAGGTAGGGGCTGAAGGTCAG -3'

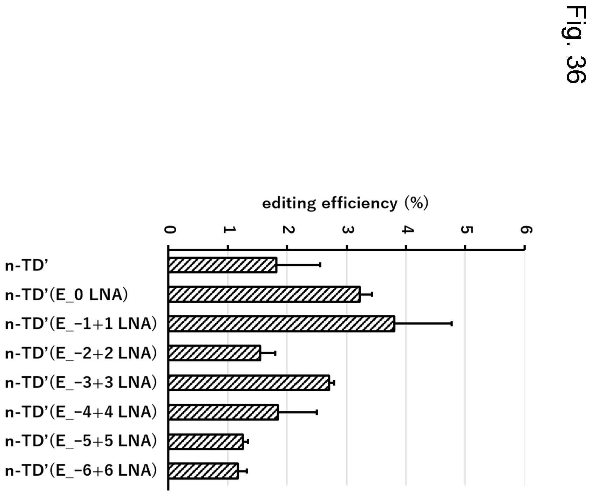

Fig. 36 shows the results. Introduction of LNA modifications at -1 and +1 gave particularly enhanced editing efficiency. The same results were obtained in editing an E strand alone.

### Example 18-3: Influence of LNA modification in E strand on base sequence editing efficiency - Part (4)

Examination was performed in the same manner as in Example 1 on base sequence editing efficiency when an LNA modification was introduced at two positions with one base intervening therebetween around the base for editing in an E strand.

The nucleic acids for editing that were used are shown in the following (having an LNA modification on the nucleotide at each underlined part).

E strand
copGFP-153-79b-AS (AS_E strand): SEQ ID NO: 5 5'- 
CGTAGCCGCTGGGGTAGGTGCCGAAGTGGTAGAAGCCGTAGCCCATCACGTGGC
TCAGCAGGTAGGGGCTGAAGGTCAG -3'
copGFP-153-79b-AS-35TD3'-ODN (A strand): SEQ ID NO: 6
   5'-CTGACCTTCAGCCCCTACCTGCTGAGCCACGTGAT -3'
copGFP-153-79b-AS-65TDdown-ODN (alias: copGFP_A_prime65) (A' strand for AS_E strand): SEQ ID NO: 7 
5'-
CTCGTACTTCTCGATGCGGGTGTTGGTGTAGCCGCCGTTGTTGATGGCGTGCAGG
AAGGGGTTCT -3'
copGFP-79_AS_E LNA-5-3: SEQ ID NO: 173 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-4-2: SEQ ID NO: 174 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-3-1: SEQ ID NO: 175 
5'-

CGTAGCCGCTGGGGTAGGTGCCGAAGTGGTAGAAGCCGTAGCCCATCACGTGGC

TCAGCAGGTAGGGGCTGAAGGTCAG -3'
copGFP-79_AS_E LNA+1+3: SEQ ID NO: 176 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA+2+4: SEQ ID NO: 177 
5'-
CGTAGCCGCTGGGGTAGGTGCCGAAGTGGTAGAAGCCGTAGCCCATCACGTGGC
 TCAGCAGGTAGGGGCTGAAGGTCAG -3'
copGFP-79_AS_E LNA+3+5: SEQ ID NO: 178

5'-
CGTAGCCGCTGGGGTAGGTGCCGAAGTGGTAGAAGCCGTAGCCCATCACGTGGC
TCAGCAGGTAGGGGCTGAAGGTCAG -3'

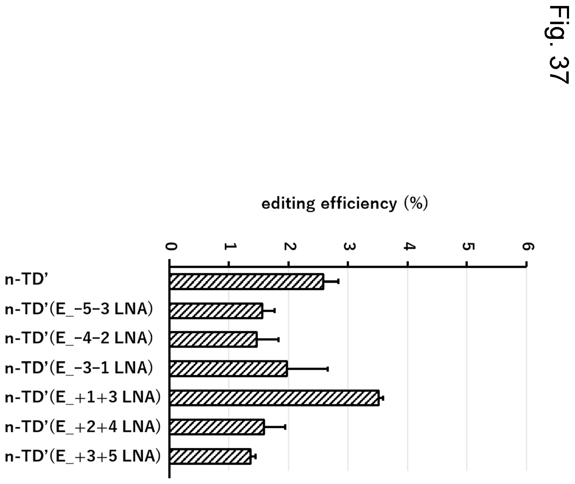

Fig. 37 shows the results. Introduction of LNA modifications at two positions of +1 and +3 gave enhanced editing efficiency. The same results were obtained in editing an E strand alone.

### Example 18-4: Influence of LNA modification in E strand on base sequence editing efficiency - Part (5)

Examination was performed in the same manner as in Example 1 on base sequence editing efficiency when an LNA modification was introduced at the position of the base for editing (0) and one additional position in an E strand.

The nucleic acids for editing that were used are shown in the following (having an LNA modification on the nucleotide at each underlined part).

E strand
copGFP-153-79b-AS (AS_E strand): SEQ ID NO: 5 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-153-79b-AS-35TD3'-ODN (A strand): SEQ ID NO: 6
   5'-CTGACCTTCAGCCCCTACCTGCTGAGCCACGTGAT -3'
copGFP-153-79b-AS-65TDdown-ODN (alias: copGFP_A_prime65) (A' strand for AS_E strand): SEQ ID NO: 7 
5'-
CTCGTACTTCTCGATGCGGGTGTTGGTGTAGCCGCCGTTGTTGATGGCGTGCAGG
AAGGGGTTCT -3'
copGFP-79_AS_E LNA0: SEQ ID NO: 46 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA0+1: SEQ ID NO: 179 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA0+2: SEQ ID NO: 180 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA0+3: SEQ ID NO: 181 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA0+4: SEQ ID NO: 182 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA0+5: SEQ ID NO: 183 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA0+6: SEQ ID NO: 184 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA0+7: SEQ ID NO: 185 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-1+0: SEQ ID NO: 186 
5'-

CGTAGCCGCTGGGGTAGGTGCCGAAGTGGTAGAAGCCGTAGCCCATCACGTGGC

TCAGCAGGTAGGGGCTGAAGGTCAG -3'
copGFP-79_AS_E LNA-2+0: SEQ ID NO: 187 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-3+0: SEQ ID NO: 188 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-4+0: SEQ ID NO: 189 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-5+0: SEQ ID NO: 190 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-6+0: SEQ ID NO: 191 
5'-

CGTAGCCGCTGGGGTAGGTGCCGAAGTGGTAGAAGCCGTAGCCCATCACGTGGC

TCAGCAGGTAGGGGCTGAAGGTCAG -3'
copGFP-79_AS_E LNA-7+0: SEQ ID NO: 192

5'-
CGTAGCCGCTGGGGTAGGTGCCGAAGTGGTAGAAGCCGTAGCCCATCACGTGGC
TCAGCAGGTAGGGGCTGAAGGTCAG -3'

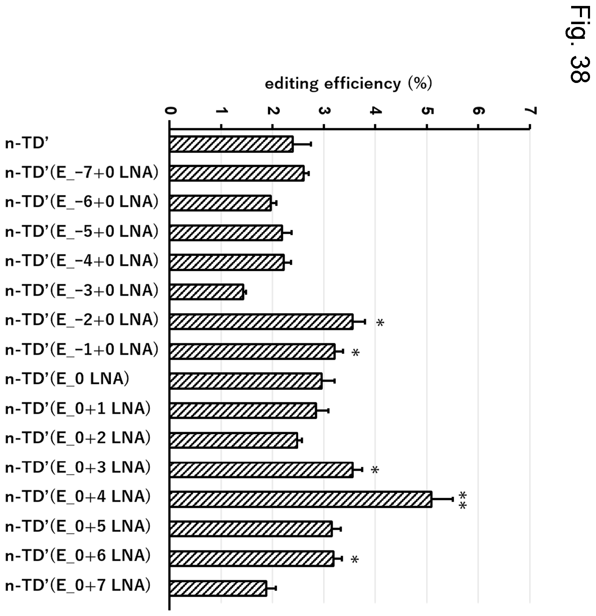

Fig. 38 shows the results. Introduction of LNA modifications at the position of the base for editing (0) and one additional position gave enhanced editing efficiency for some combinations (in particular, -2+0, -1+0, 0+3, 0+4, and 0+6) as compared with introduction of no LAN modification. Introduction of LNA modifications at positions of 0 and +4 (0+4) gave particularly enhanced editing efficiency. The same results were obtained in editing an E strand alone.

### Example 18-5: Influence of LNA modification in E strand on base sequence editing efficiency - Part (6)

Examination was performed in the same manner as in Example 1 on base sequence editing efficiency when an LNA modification was introduced at the position of the base for editing (0) and two additional positions in an E strand.

The nucleic acids for editing that were used are shown in the following (having an LNA modification on the nucleotide at each underlined part).

E strand
copGFP-153-79b-AS (AS_E strand): SEQ ID NO: 5 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-153-79b-AS-35TD3'-ODN (A strand): SEQ ID NO: 6
   5'-CTGACCTTCAGCCCCTACCTGCTGAGCCACGTGAT -3'
copGFP-153-79b-AS-65TDdown-ODN (alias: copGFP_A_prime65) (A' strand for AS_E strand): SEQ ID NO: 7 
5'-
CTCGTACTTCTCGATGCGGGTGTTGGTGTAGCCGCCGTTGTTGATGGCGTGCAGG
AAGGGGTTCT -3'
copGFP-79_AS_E LNA0: SEQ ID NO: 46 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-1+0+1: SEQ ID NO: 47 
5'-

CGTAGCCGCTGGGGTAGGTGCCGAAGTGGTAGAAGCCGTAGCCCATCACGTGGC

TCAGCAGGTAGGGGCTGAAGGTCAG -3'
copGFP-79_AS_E LNA-2+0+2: SEQ ID NO: 193 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-3+0+3: SEQ ID NO: 194 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-4+0+4: SEQ ID NO: 195 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-5+0+5: SEQ ID NO: 196 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-6+0+6: SEQ ID NO: 197 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-7+0+7: SEQ ID NO: 198

5'-
CGTAGCCGCTGGGGTAGGTGCCGAAGTGGTAGAAGCCGTAGCCCATCACGTGGC
TCAGCAGGTAGGGGCTGAAGGTCAG -3'

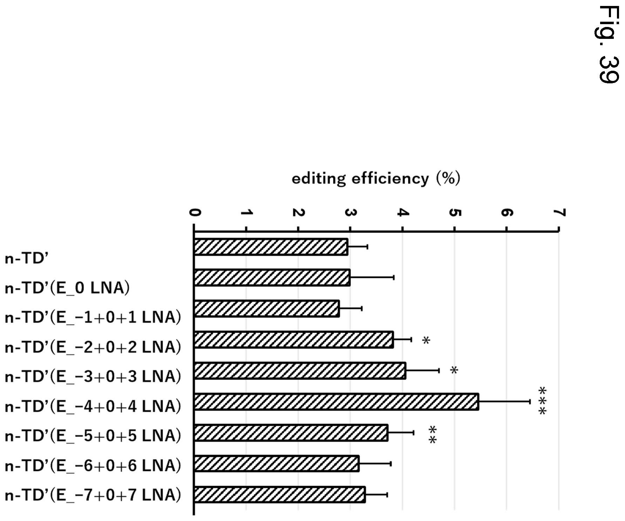

Fig. 39 shows the results. Some combinations gave enhanced editing efficiency (in particular, -2+0+2, -3+0+3, -4+0+4, and -5+0+5) as compared with introduction of no LAN modification. Introduction of LNA modifications at the position of the base for editing (0), -4, and +4 (-4+0+4) gave particularly enhanced editing efficiency. The same results were obtained in editing an E strand alone.

### Example 18-6: Influence of LNA modification in E strand on base sequence editing efficiency - Part (7)

Examination was performed in the same manner as in Example 1 on base sequence editing efficiency when an LNA modification was introduced on two or three consecutive bases in two parts symmetric with respect to the position of the base for editing (0) as the center in an E strand.

The nucleic acids for editing that were used are shown in the following (having an LNA modification on the nucleotide at each underlined part).

E strand
copGFP-153-79b-AS (AS_E strand): SEQ ID NO: 5 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-153-79b-AS-35TD3'-ODN (A strand): SEQ ID NO: 6
   5'-CTGACCTTCAGCCCCTACCTGCTGAGCCACGTGAT -3'
copGFP-153-79b-AS-65TDdown-ODN (alias: copGFP_A_prime65) (A' strand for AS_E strand): SEQ ID NO: 7 
5'-
CTCGTACTTCTCGATGCGGGTGTTGGTGTAGCCGCCGTTGTTGATGGCGTGCAGG
 AAGGGGTTCT -3'
copGFP-79_AS_E LNA-2-1+1+2: SEQ ID NO: 199 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-3-2+2+3: SEQ ID NO: 200 
5'-

CGTAGCCGCTGGGGTAGGTGCCGAAGTGGTAGAAGCCGTAGCCCATCACGTGGC

TCAGCAGGTAGGGGCTGAAGGTCAG -3'
copGFP-79_AS_E LNA-3-2-1+1+2+3: SEQ ID NO: 201

5'-
CGTAGCCGCTGGGGTAGGTGCCGAAGTGGTAGAAGCCGTAGCCCATCACGTGGC
TCAGCAGGTAGGGGCTGAAGGTCAG -3'

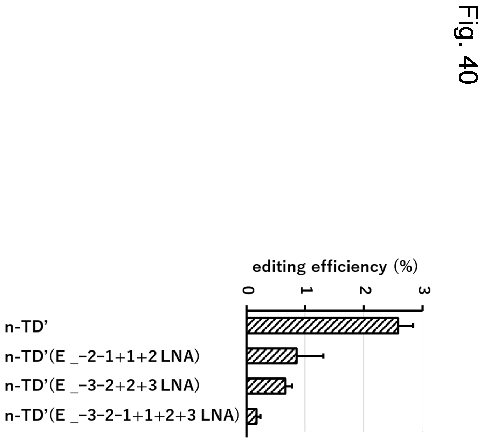

Fig. 40 shows the results. Any of the combinations gave lowered editing efficiency. The same results were obtained in editing an E strand alone.

### Example 18-7: Influence of LNA modification in E strand on base sequence editing efficiency - Part (8)

Examination was performed in the same manner as in Example 1 on base sequence editing efficiency when an LNA modification at +4 was combined with the introduction of any LNA modification that gave enhanced editing efficiency in Examples 3 (3-6) and 18-1 to 18-6 (i.e., the position of the base for editing (0), -1+1, -3-1+1+3, -2+0, -1+0, 0+1, and 0+2).

The nucleic acids for editing that were used are shown in the following (having an LNA modification on the nucleotide at each underlined part).

E strand
copGFP-153-79b-AS (AS_E strand): SEQ ID NO: 5 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-153-79b-AS-35TD3'-ODN (A strand): SEQ ID NO: 6
   5'-CTGACCTTCAGCCCCTACCTGCTGAGCCACGTGAT -3'
copGFP-153-79b-AS-65TDdown-ODN (alias: copGFP_A_prime65) (A' strand for AS_E strand): SEQ ID NO: 7 
5'-
CTCGTACTTCTCGATGCGGGTGTTGGTGTAGCCGCCGTTGTTGATGGCGTGCAGG
AAGGGGTTCT -3'
copGFP-79_AS_E LNA-1+1: SEQ ID NO: 48 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-1+1+4: SEQ ID NO: 202 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-3-1+1+3: SEQ ID NO: 49 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-3-1+1+3+4: SEQ ID NO: 203 
5'-

CGTAGCCGCTGGGGTAGGTGCCGAAGTGGTAGAAGCCGTAGCCCATCACGTGGC

TCAGCAGGTAGGGGCTGAAGGTCAG -3'
copGFP-79_AS_E LNA0+1: SEQ ID NO: 179 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA0+1+4: SEQ ID NO: 204 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-1+0: SEQ ID NO: 186 
5'-

CGTAGCCGCTGGGGTAGGTGCCGAAGTGGTAGAAGCCGTAGCCCATCACGTGGC

TCAGCAGGTAGGGGCTGAAGGTCAG -3'
copGFP-79_AS_E LNA-1+0+4: SEQ ID NO: 205 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA0+2: SEQ ID NO: 180 
5'-

CGTAGCCGCTGGGGTAGGTGCCGAAGTGGTAGAAGCCGTAGCCCATCACGTGGC

TCAGCAGGTAGGGGCTGAAGGTCAG -3'
copGFP-79_AS_E LNA0+2+4: SEQ ID NO: 206 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-2+0: SEQ ID NO: 187 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-2+0+4: SEQ ID NO: 207 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA0+4: SEQ ID NO: 182

5'-
CGTAGCCGCTGGGGTAGGTGCCGAAGTGGTAGAAGCCGTAGCCCATCACGTGGC
TCAGCAGGTAGGGGCTGAAGGTCAG -3'

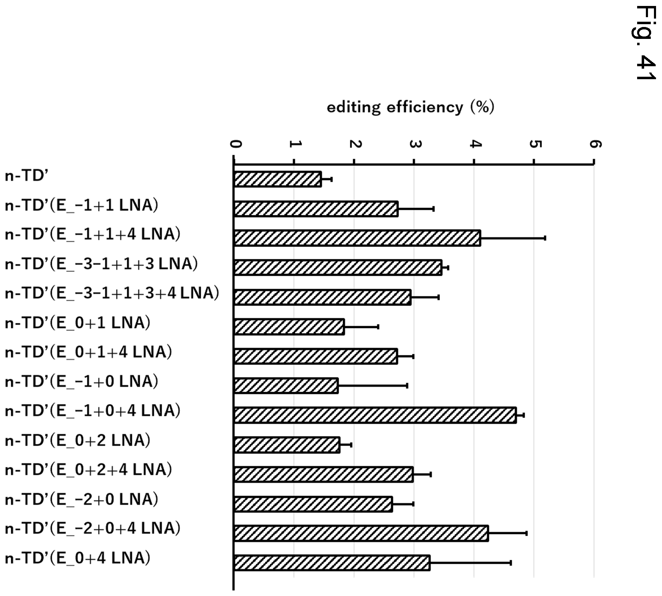

Fig. 41 shows the results. Except for -3-1+1+3, combining with an LNA modification at +4 gave enhanced editing efficiency.

### Example 18-8: Influence of LNA modification in E strand on base sequence editing efficiency - Part (9)

Examination was performed in the same manner as in Example 1 on base sequence editing efficiency when an unmodified E strand or an E strand obtained by additionally introducing an LNA modification at a position of +4, +5, +6, or +7 in an E strand having an LNA modification at the position of the base for editing, 0, and any of a series of A strands having different lengths (31 to 35 nucleotides) obtained by successively truncating at the 3' end by one nucleotide were used.

The experimental results in Example 18-7 and the former examples showed that combining an LNA modification at a position of + 4 and an LNA modification at another position in an E strand gave particularly enhanced editing efficiency. In using an ODN consisting of a complementary sequence to a sequence of 35 nucleotides from the 3' end of an E strand as an A strand, the position of +4 in the E strand corresponds to the base adjacent to the base at the 3' end of the A strand, that is, the 3' end of the sequence that does not form base pairs with the A strand in the E strand. Accordingly, examination was performed on the relationship between the position of an LNA modification in an E strand and the 3' end of an A strand.

The nucleic acids for editing that were used are shown in the following (having an LNA modification on the nucleotide at each underlined part).

E strand
copGFP-153-79b-AS (AS_E strand): SEQ ID NO: 5 
5'-
CGTAGCCGCTGGGGTAGGTGCCGAAGTGGTAGAAGCCGTAGCCCATCACGTGGC
 TCAGCAGGTAGGGGCTGAAGGTCAG -3'
copGFP-153-79b-AS-35TD3'-ODN (A strand, 35-nt A strand): SEQ ID NO: 6 5'-CTGACCTTCAGCCCCTACCTGCTGAGCCACGTGAT -3'
copGFP-153-79b-AS-65TDdown-ODN (alias: copGFP_A_prime65) (A' strand for AS_E strand): SEQ ID NO: 7 
5'-
CTCGTACTTCTCGATGCGGGTGTTGGTGTAGCCGCCGTTGTTGATGGCGTGCAGG
AAGGGGTTCT -3'
copGFP-79_AS_E LNA0+4: SEQ ID NO: 182 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-153-79b-AS-34TD3'-ODN (34-nt A strand): SEQ ID NO: 208 5'-CTGACCTTCAGCCCCTACCTGCTGAGCCACGTGA -3'
copGFP-153-79b-AS-33TD3'-ODN (33-nt A strand): SEQ ID NO: 209 5'-CTGACCTTCAGCCCCTACCTGCTGAGCCACGTG -3'
copGFP-153-79b-AS-32TD3'-ODN (32-nt A strand): SEQ ID NO: 210 5'-CTGACCTTCAGCCCCTACCTGCTGAGCCACGT -3'
copGFP-153-79b-AS-31TD3'-ODN (31-nt A strand): SEQ ID NO: 211 5'-CTGACCTTCAGCCCCTACCTGCTGAGCCACG -3'
copGFP-79_AS_E LNA0+5: SEQ ID NO: 183 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA0+6: SEQ ID NO: 184 
5'-

CGTAGCCGCTGGGGTAGGTGCCGAAGTGGTAGAAGCCGTAGCCCATCACGTGGC

TCAGCAGGTAGGGGCTGAAGGTCAG -3'
copGFP-79_AS_E LNA0+7: SEQ ID NO: 185

5'-
CGTAGCCGCTGGGGTAGGTGCCGAAGTGGTAGAAGCCGTAGCCCATCACGTGGC
TCAGCAGGTAGGGGCTGAAGGTCAG -3'

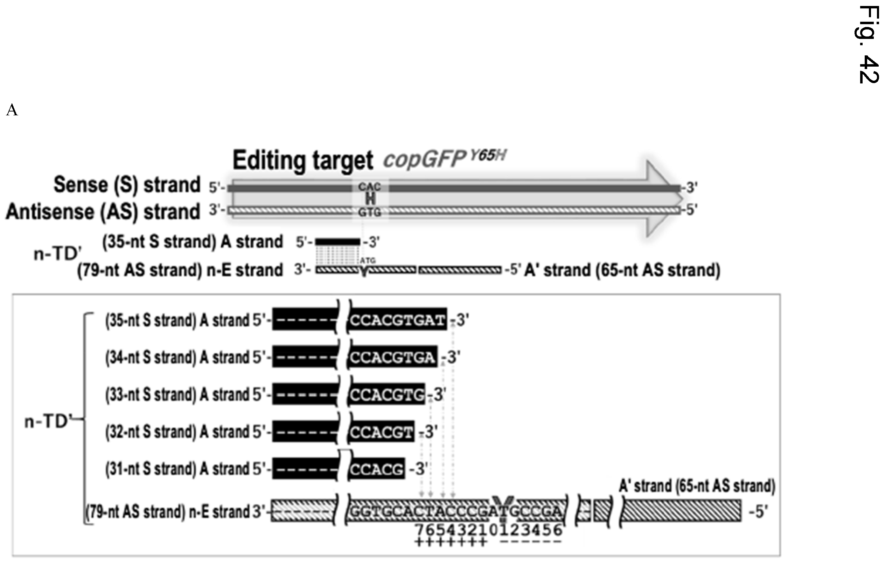

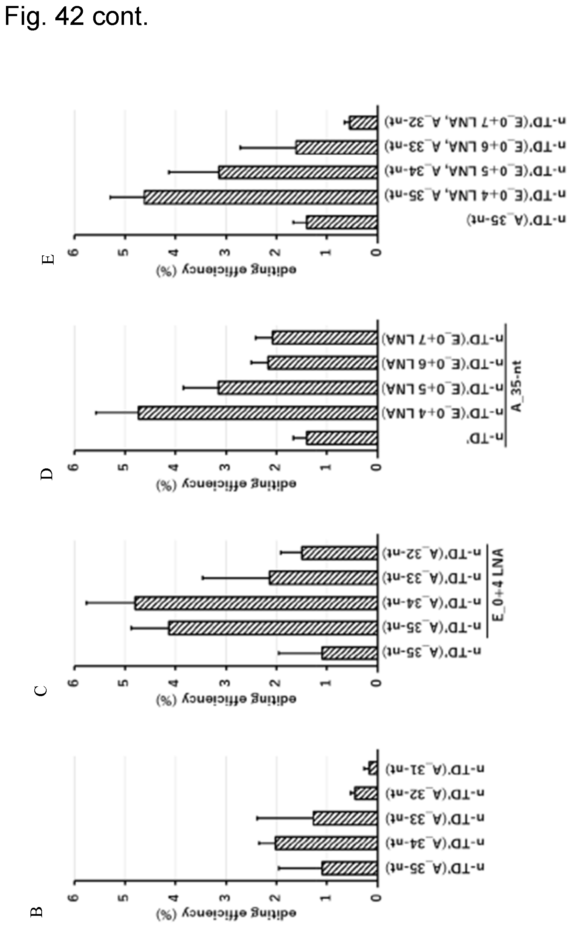

Fig. 42 shows the results. Each n-TD' with an unmodified E strand or an E strand having LNA modifications introduced at two positions of 0 and +4 gave the highest editing efficiency in the case of an A strand having a length of 34 nucleotides, and remarkably lowered editing efficiency in the case of an A strand having a length of 32 nucleotides or less. For A strands having lengths different from them, the n-TD' with an E strand having LNA modifications introduced at two positions of 0 and +4 gave higher editing efficiency than the n-TD' with an unmodified E strand. The enhancement of editing efficiency obtained by LNA modifications in an E strand depended not on the positional relationship between the positions of the LNA modifications in the E strand and the 3' end of the A strand, but on the positions of the LNA modifications in the E strand and on proper length for the A strand.

### Example 18-9: Influence of LNA modification in E strand on base sequence editing efficiency - Part (10)

Examination was performed in the same manner as in Example 1 on base sequence editing efficiency obtained using an n-TD' with an E strand having LNA modifications at two positions that gave enhanced editing efficiency in Example 18-4 and an A strand of 33, 34, or 35 nucleotides in length.

The nucleic acids for editing that were used are shown in the following (having an LNA modification on the nucleotide at each underlined part).

E strand
copGFP-153-79b-AS (AS_E strand): SEQ ID NO: 5 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-153-79b-AS-35TD3'-ODN (A strand, 35-nt A strand): SEQ ID NO: 6 5'-CTGACCTTCAGCCCCTACCTGCTGAGCCACGTGAT -3'
copGFP-153-79b-AS-34TD3'-ODN (34-nt A strand): SEQ ID NO: 208 5'-CTGACCTTCAGCCCCTACCTGCTGAGCCACGTGA -3'
copGFP-153-79b-AS-33TD3'-ODN (33-nt A strand): SEQ ID NO: 209 5'-CTGACCTTCAGCCCCTACCTGCTGAGCCACGTG -3'
copGFP-153-79b-AS-65TDdown-ODN (alias: copGFP_A_prime65) (A' strand for AS_E strand): SEQ ID NO: 7 
5'-
CTCGTACTTCTCGATGCGGGTGTTGGTGTAGCCGCCGTTGTTGATGGCGTGCAGG
AAGGGGTTCT -3'
copGFP-79_AS_E LNA0+4: SEQ ID NO: 182 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA0+5: SEQ ID NO: 183 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA0+6: SEQ ID NO: 184 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA0+7: SEQ ID NO: 185

5'-
CGTAGCCGCTGGGGTAGGTGCCGAAGTGGTAGAAGCCGTAGCCCATCACGTGGC
TCAGCAGGTAGGGGCTGAAGGTCAG -3'

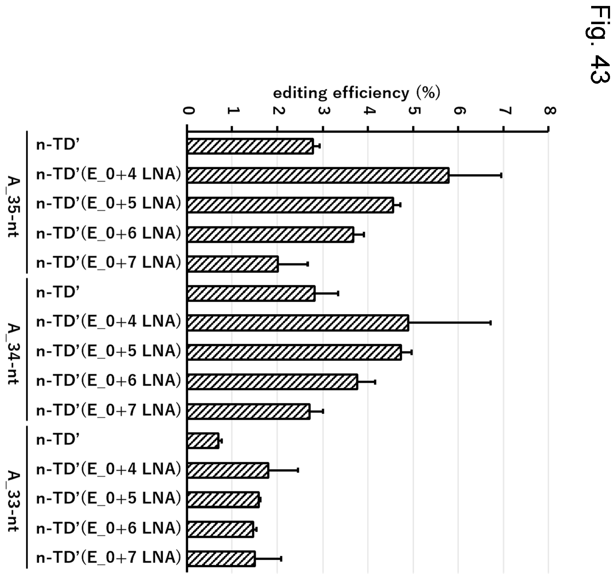

Fig. 43 shows the results. An n-TD' containing an A strand of 35 nucleotides in length or 34 nucleotides in length gave enhanced editing efficiency as compared with the case without any LNA modification when an LNA modification was introduced at two positions of 0 and +4, +5, or +6. The highest editing efficiency resulted when an E strand having LNA modifications introduced at positions of 0 and +4 was used, and lower editing efficiency was obtained as the modified position moved farther to +5, +6, and so on. No difference in the tendency of enhanced editing efficiency was found between the A strand of 35 nucleotides in length and the A strand of 34 nucleotides in length. The E strand having LNA modifications introduced at 0 and +7 did not give enhanced editing efficiency. The A strand of 33 nucleotides in length gave enhanced editing efficiency through introduction of LNA modifications at two positions in the E strand, but no difference depending on the positions of LNA modifications was found.

### Example 18-10: Influence of LNA modification in E strand on base sequence editing efficiency - Part (11)

Examination was performed in the same manner as in Example 1 on base sequence editing efficiency obtained using an n-TD' with an E strand that gave enhanced editing efficiency in the experimental results in Example 18-7 and an A strand of 33, 34, or 35 nucleotides in length.

The nucleic acids for editing that were used are shown in the following (having an LNA modification on the nucleotide at each underlined part).

E strand
copGFP-153-79b-AS (AS_E strand): SEQ ID NO: 5 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-153-79b-AS-35TD3'-ODN (A strand, 35-nt A strand): SEQ ID NO: 6 5'-CTGACCTTCAGCCCCTACCTGCTGAGCCACGTGAT -3'
copGFP-153-79b-AS-34TD3'-ODN (34-nt A strand): SEQ ID NO: 208 5'-CTGACCTTCAGCCCCTACCTGCTGAGCCACGTGA -3'
copGFP-153-79b-AS-33TD3'-ODN (33-nt A strand): SEQ ID NO: 209
   5'-CTGACCTTCAGCCCCTACCTGCTGAGCCACGTG -3'
copGFP-153-79b-AS-65TDdown-ODN (alias: copGFP_A_prime65) (A' strand for AS_E strand): SEQ ID NO: 7
copGFP-79_AS_E LNA0: SEQ ID NO: 46 
5'-
CGTAGCCGCTGGGGTAGGTGCCGAAGTGGTAGAAGCCGTAGCCCATCACGTGGC
TCAGCAGGTAGGGGCTGAAGGTCAG -3'
 
5'-
CTCGTACTTCTCGATGCGGGTGTTGGTGTAGCCGCCGTTGTTGATGGCGTGCAGG
AAGGGGTTCT -3'
copGFP-79_AS_E LNA0+4: SEQ ID NO: 182 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-1+1+4: SEQ ID NO: 202 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-1+0+4: SEQ ID NO: 205 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-2+0+4: SEQ ID NO: 207

5'-
CGTAGCCGCTGGGGTAGGTGCCGAAGTGGTAGAAGCCGTAGCCCATCACGTGGC
TCAGCAGGTAGGGGCTGAAGGTCAG -3'

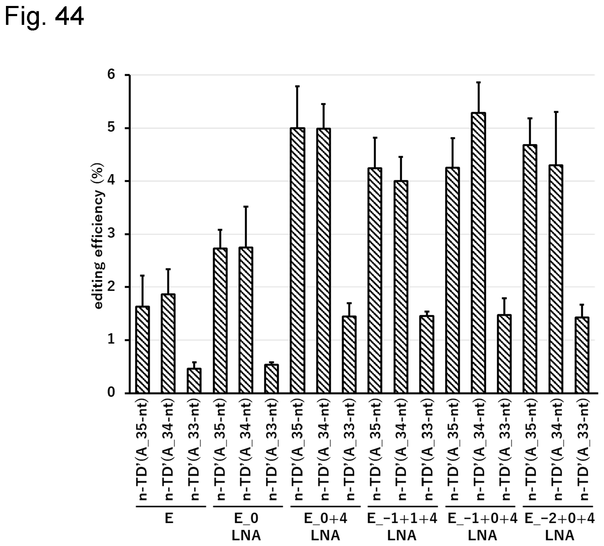

Fig. 44 shows the results. An n-TD' containing an E strand having an LNA modification introduced gave enhanced editing efficiency, and the degree of enhancement for the A strand of 35 nucleotides in length and that for the A strand of 34 nucleotides in length were almost equivalent.

### Example 18-11: Influence of LNA modification in E strand on base sequence editing efficiency - Part (12)

Examination was performed on the influence of the positions of LNA modifications in an E strand that affect base sequence editing efficiency for copGFP gene on the nonsense mutations in DMD gene and WRN gene that were used in the experiment in Example 16. Examination was performed in the same manner as in Example 1 on base sequence editing efficiency obtained using an n-TD' for the genes. An E strand, an A strand, and an A' strand were introduced into cells at molar ratios of 10, 20, and 30, respectively, to a pDNA as 1 for copGFP gene and DMD gene, and at molar ratios of 0.1, 0.2, and 0.3, respectively, to a pDNA as 1 for WRN gene, and analysis of editing efficiency was conducted.

The nucleic acids for editing that were used are shown in the following (having an LNA modification on the nucleotide at each underlined part).

E strand
copGFP-153-79b-AS (AS_E strand): SEQ ID NO: 5 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-153-79b-AS-35TD3'-ODN (A strand, 35-nt A strand): SEQ ID NO: 6 5'-CTGACCTTCAGCCCCTACCTGCTGAGCCACGTGAT -3'
copGFP-153-79b-AS-65TDdown-ODN (alias: copGFP_A_prime65) (A' strand for AS_E strand): SEQ ID NO: 7 
5'-
CTCGTACTTCTCGATGCGGGTGTTGGTGTAGCCGCCGTTGTTGATGGCGTGCAGG
AAGGGGTTCT -3'
copGFP-79_AS_E LNA0+4: SEQ ID NO: 182 5'- 
CGTAGCCGCTGGGGTAGGTGCCGAAGTGGTAGAAGCCGTAGCCCATCACGTGGC

TCAGCAGGTAGGGGCTGAAGGTCAG -3'
copGFP-79_AS_E LNA-4+0: SEQ ID NO: 189 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-1+1: SEQ ID NO: 48 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA0: SEQ ID NO: 46 
5'-
CGTAGCCGCTGGGGTAGGTGCCGAAGTGGTAGAAGCCGTAGCCCATCACGTGGC
TCAGCAGGTAGGGGCTGAAGGTCAG -3'
copGFP-79_AS_E LNA-2+2: SEQ ID NO: 51 
5'-

CGTAGCCGCTGGGGTAGGTGCCGAAGTGGTAGAAGCCGTAGCCCATCACGTGGC

TCAGCAGGTAGGGGCTGAAGGTCAG -3'
DMD-79_AS_E: SEQ ID NO: 212 
5'-
TGATTACATTAACCTGTGGATAATTACGAGTTGATTGTCGGACCCAGCTCAGGAG
AATCTTTTCACTGTTGGTTTGTTG -3'
DMD-35_forAS-E_A: SEQ ID NO: 213
   5'-CAACAAACCAACAGTGAAAAGATTCTCCTGAGCTG -3'
DMD-65_forAS-E_Aprime: SEQ ID NO: 214 
5'-
GCCGGGATTCTCCTCCACGTCACCGCATGTTAGAAGACTTCCTCTGCCCTCGCTG
GTGGTGAAGT -3'
DMD-79_AS_E LNA0+4: SEQ ID NO: 215 
5'-
TGATTACATTAACCTGTGGATAATTACGAGTTGATTGTCGGACCCAGCTCAGGAG
AATCTTTTCACTGTTGGTTTGTTG -3'
DMD-79_AS_E LNA-4+0: SEQ ID NO: 216 
5'-
TGATTACATTAACCTGTGGATAATTACGAGTTGATTGTCGGACCCAGCTCAGGAG
AATCTTTTCACTGTTGGTTTGTTG -3'
DMD-79_AS_E LNA-1+1: SEQ ID NO: 217 
5'-
TGATTACATTAACCTGTGGATAATTACGAGTTGATTGTCGGACCCAGCTCAGGAG
AATCTTTTCACTGTTGGTTTGTTG -3'
DMD-79_AS_E LNA0: SEQ ID NO: 218 
5'-
TGATTACATTAACCTGTGGATAATTACGAGTTGATTGTCGGACCCAGCTCAGGAG
AATCTTTTCACTGTTGGTTTGTTG -3'
DMD-79_AS_E LNA-2+2: SEQ ID NO: 219 
5'-
TGATTACATTAACCTGTGGATAATTACGAGTTGATTGTCGGACCCAGCTCAGGAG
AATCTTTTCACTGTTGGTTTGTTG -3'
WRN-79_AS_E: SEQ ID NO: 220 
5'-
CAGCAATAATCTTCTGAACCTCTGGAGTCAGGCCTGCTCGCTCCAAATCAAGGGG
GCAGCCAGCTTTCACCGCTTGGGA -3'
WRN-35_forAS-E_A: SEQ ID NO: 221
   5'-TCCCAAGCGGTGAAAGCTGGCTGCCCCCTTGATTT -3'
WRN-65_forAS-E_Aprime: SEQ ID NO: 222 GCCGGGATTCTCCTCCACGTCACCGCATGTTAGAAGACTTCCTCTGCCCTCGTTTC GGATAACAT
WRN-79_AS_E LNA0+4: SEQ ID NO: 223 
5'-
CAGCAATAATCTTCTGAACCTCTGGAGTCAGGCCTGCTCGCTCCAAATCAAGGGG
GCAGCCAGCTTTCACCGCTTGGGA -3'
WRN-79_AS_E LNA-4+0: SEQ ID NO: 224 
5'-
CAGCAATAATCTTCTGAACCTCTGGAGTCAGGCCTGCTCGCTCCAAATCAAGGGG
GCAGCCAGCTTTCACCGCTTGGGA -3'
WRN-79_AS_E LNA-1+1: SEQ ID NO: 225 
5'-
CAGCAATAATCTTCTGAACCTCTGGAGTCAGGCCTGCTCGCTCCAAATCAAGGGG
GCAGCCAGCTTTCACCGCTTGGGA -3'
WRN-79_AS_E LNA0: SEQ ID NO: 226 
5'-
CAGCAATAATCTTCTGAACCTCTGGAGTCAGGCCTGCTCGCTCCAAATCAAGGGG
GCAGCCAGCTTTCACCGCTTGGGA -3'
WRN-79_AS_E LNA-2+2: SEQ ID NO: 227

5'-
CAGCAATAATCTTCTGAACCTCTGGAGTCAGGCCTGCTCGCTCCAAATCAAGGGG
GCAGCCAGCTTTCACCGCTTGGGA -3'

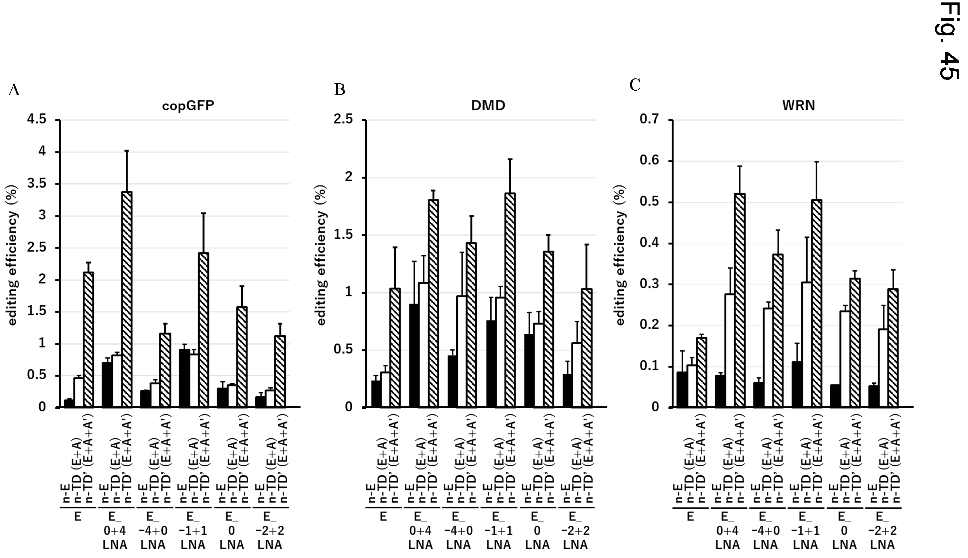

Fig. 45 shows the results. For any of the genes, an n-TD' containing an E strand having LNA modifications introduced at two positions of 0 and +4 or -1 and +1 gave particularly enhanced editing efficiency as compared with an n-TD' containing an unmodified E strand. For WRN gene, an n-TD' containing an E strand having an LNA modification introduced gave enhanced editing efficiency as compared with the n-TD' containing an unmodified E strand. Regarding n-TDs (E+A), for DMD gene and WRN gene, an n-TD containing an E strand having an LNA modification introduced gave enhanced editing efficiency as compared with an n-TD containing an unmodified E strand.

### Example 19: Examination on mutagenesis by ethylphosphotriester bond, phosphorothioate bond, or LNA modification

The examples shown above have demonstrated that modification of an ODN results in enhanced editing efficiency obtained using an n-TD'. Examination was performed on the presence or absence of possibility that a polynucleotide subjected to such modification causes mutagenesis when used as a template for DNA replication in human cells.

The specific experimental method is as follows.

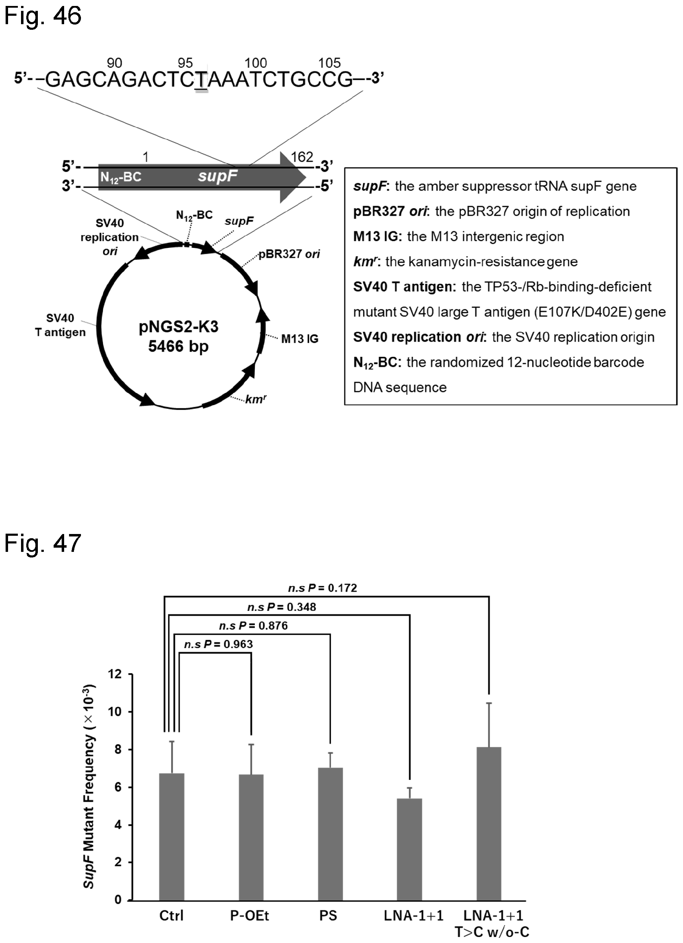

A supF-NGS method (H. Kawai, R. Iwata, S. Ebi, R. Sugihara, S. Masuda, C. Fujiwara, S. Kimura, H. Kamiya. Development of a versatile high-throughput mutagenesis assay with multiplexed short-read NGS using DNA-barcoded supF shuttle vector library amplified in E. coli. Elife. 11, e83780 (2022)) was used for analysis of mutations induced by modified polynucleotides. The supF-NGS method is a technique using pNGS2-K3 (Fig. 46), which is a shuttle vector plasmid (replicated both in mammalian cells and in *Escherichia coli*) containing supF (amber suppressor tRNA) gene as a marker for mutation detection and a molecular barcode (a 12-base random base sequence for use in analysis of mutation data), and RF01, which is indicator *Escherichia coli* for mutation detection for supF gene (R. Fukushima, T. Suzuki, H. Kamiya. New indicator Escherichia coli strain for rapid and accurate detection of supF mutations. Genes Environ. 42, 28 (2020)), to analyze mutations in the supF gene sequence and the frequencies thereof through next-generation sequencer (NGS) analysis.

First, double-stranded circular DNAs were constructed from pNGS2-K3 that had been formed into a single-stranded circlular DNA through *in vitro* enzymatic reaction (R. Fukushima, T. Suzuki, Y. Komatsu, H. Kamiya. Biased distribution of action-at-a-distance mutations by 8-oxo-7,8-dihydroguanine. Mutation Res. (Fundam. Mol. Mech. Mutagen.), 825, 111794 (2022)) with use of five ODNs each having the 5' end subjected to phosphorylation modification (Phos-) (SEQ ID NO: 228 to 232; each modified part was indicated by an underline).
SupF top_strand: SEQ ID NO: 228
   5'-Phos-GAGCAGACTCTAAATCTGCCG -3'
SupF top_strand p-etho (ethylphosphotriester bond): SEQ ID NO: 229 5'-Phos-GAGCAGACTCTAAATCTGCCG -3'
SupF top_strand S (phosphorothioate bond): SEQ ID NO: 230 5'-Phos-GAGCAGACTCTAAATCTGCCG -3'
SupF top_strand LNA-1+1: SEQ ID NO: 231
   5'-Phos-GAGCAGACTCTAAATCTGCCG -3'
SupF top_strand LNA-1+1 T>C: SEQ ID NO: 232
   5'- Phos-GAGCAGACTCCAAATCTGCCG -3'

pNGS2-K3 (Ctrl) for control experiment was constructed with an unmodified ODN set forth in SEQ ID NO: 228, and a plasmid having an ethylphosphotriester bond introduced into the supF gene sequence, a plasmid having a phosphorothioate bond introduced into the supF gene sequence, and a plasmid having LNA modifications (at two positions) introduced into the supF gene sequence (pNGS2-K3(P-OEt), pNGS2-K3(PS), and pNGS2-K3(LNA-1+1), respectively) were constructed with an ODN set forth in SEQ ID NO: 229, an ODN set forth in SEQ ID NO: 230, and an ODN set forth in SEQ ID NO: 231, respectively. In addition, pNGS2-K3 (LNA-1+1 T>C) with a base pair between LNA modifications at two positions being a C:A mismatch was constructed with an ODN set forth in SEQ ID NO: 232.

Each of the double-stranded circular DNAs constructed (400 ng) was introduced with Lipofectamine 2000 into U2OS cells (seeded at 5 × 10⁵ cell/well on the previous day) in a 6-well plate. The cells were cultured for 48 hours, and the plasmids replicated from the double-stranded circular DNAs in cells were collected and each introduced into RF01. Each RF01 was seeded in a Kanamycin-containing LB plate, and the plasmids were extracted from colonies formed, sequence information on supF gene was acquired by the supF-NGS method, and the mutant frequencies were calculated.

Fig. 47 shows the results. For pNGS2-K3 (Ctrl), pNGS2-K3 (P-OEt), pNGS2-K3 (PS), and pNGS2-K3 (LNA-1+1), the means of three experimental data on mutant frequency in supF gene were 6.73 × 10⁻³, 6.67 × 10⁻³, 7.04 × 10⁻³, and 5.41 × 10⁻³, respectively, and those modifications were revealed to cause no significant induction of a mutation. For pNGS2-K3(LNA-1+1 T>C), the mean of mutant frequency was 5.52 × 10⁻¹, and 5.49 × 10⁻¹ thereof was attributed to T-to-C mutations derived from the C:A mismatch between LNA modifications. This result suggested that a DNA strand containing LNA modifications at two positions in pNGS2-K3 and an unmodified DNA strand were replicated at almost the same efficiencies, and thus the possibility that LNA modifications do not inhibit DNA replication. The result that the mean of mutant frequency with the T-to-C mutations (LNA-1+1 T>C w/o-C) excluded was 8.12 × 10⁻³ revealed that even if a mismatching base pair for editing is present, the LNA modifications on both sides thereof do not cause significant induction of a mutation.

### Example 20: Enhancement of base sequence editing efficiency by combination of LNA modification in E strand and P-OEt modification in A strand - Part (2)

LNA modifications in an E strand for which enhanced editing efficiency had been found were combined with a P-OEt modification in an A strand, and examination was performed on base sequence editing efficiency in the same manner as in Example 1. The nucleic acids for editing that were used are shown in the following.

E strand
copGFP-153-79b-AS (AS_E strand): SEQ ID NO: 5
copGFP-153-79b-AS-35TD3'-ODN (A strand, 35-nt A strand): SEQ ID NO: 6 copGFP_forAS_E79 A35 3' p-etho × 1 : SEQ ID NO: 44
copGFP-153-79b-AS-65TDdown-ODN (alias: copGFP_A_prime65) (A' strand for AS_E strand): SEQ ID NO: 7
copGFP-79_AS_E LNA-1+1: SEQ ID NO: 48
copGFP-79_AS_E LNA-1+1+4: SEQ ID NO: 202
copGFP-79_AS_E LNA-1+0: SEQ ID NO: 186
copGFP-79_AS_E LNA-1+0+4: SEQ ID NO: 205
copGFP-79_AS_E LNA-2+0: SEQ ID NO: 187
copGFP-79_AS_E LNA-2+0+4: SEQ ID NO: 207
copGFP-79_AS_E LNA0: SEQ ID NO: 46
copGFP-79_AS_E LNA0+4: SEQ ID NO: 182

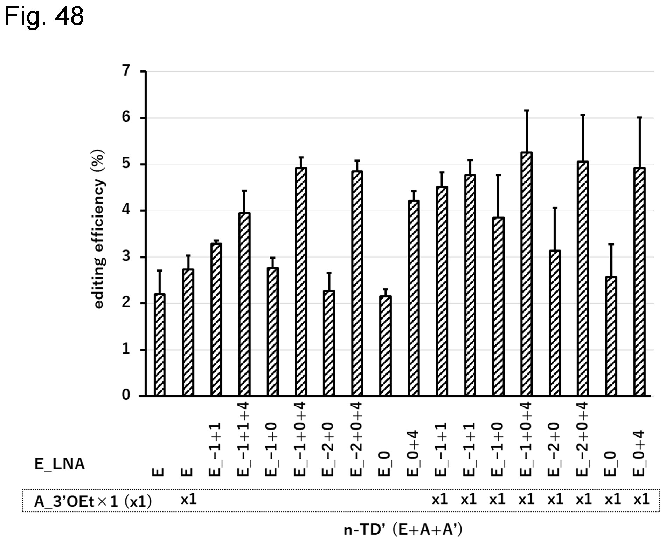

Fig. 48 shows the results. It was found that any of the cases with introduction of an LNA modification into an E strand tended to give enhanced editing efficiency in an additive manner when combined with a P-OEt modification in an A strand.

### INDUSTRIAL APPLICABILITY

The present invention enables efficient editing of a target base sequence in a genomic DNA without use of any artificial nuclease. Accordingly, the present invention can be used for genome editing in the field of cell engineering, bioengineering, healthcare, and so on. In particular, the present invention can be used for treating or preventing a disease due to a mutation in a gene.

### SEQUENCE LISTING FREE TEXT

SEQ ID NO: 1: nucleotide sequence for mPlum-T2A-copGFP (Y/H) (sequence containing mutated copGFP gene)
SEQ ID NO: 2: amino acid sequence of mPlum-T2A-copGFP (Y/H)
SEQ ID NO: 3: nucleotide sequence for mPlum-T2A-mEGFP (Y/H) (sequence containing mutated mEGFP gene)
SEQ ID NO: 4: amino acid sequence of mPlum-T2A-mEGFP (Y/H)
SEQ ID NO: 5: copGFP-153-79b-AS (AS_E strand)
SEQ ID NO: 6: copGFP-153-79b-AS-35TD3'-ODN (A strand for AS_E strand)
SEQ ID NO: 7: copGFP-153-79b-AS-65TDdown-ODN (alias: copGFP_A_prime65) (A' strand for AS_E strand)
SEQ ID NO: 8: mEGFP-79_AS_E
SEQ ID NO: 9: mEGFP-35_S_A
SEQ ID NO: 10: mEGFP-65_AS_Ap
SEQ ID NO: 11: copGFP-79_AS_E 5'3'S
SEQ ID NO: 12: copGFP-79_AS_E 5'S
SEQ ID NO: 13: copGFP-79_AS_E 3'S
SEQ ID NO: 14: copGFP-79_AS_E 5'3'SS
SEQ ID NO: 15: copGFP-79_AS_E 5'SS
SEQ ID NO: 16: copGFP -79_AS_E 3'SS
SEQ ID NO: 17: copGFP-35_S_A 5'3'S
SEQ ID NO: 18: copGFP-35_S_A 5'S
SEQ ID NO: 19: copGFP-35_S_A 3'S
SEQ ID NO: 20: copGFP-35_S_A 5'3'SS
SEQ ID NO: 21: copGFP-35_S_A 5'SS
SEQ ID NO: 22: copGFP-35_S_A 3'SS
SEQ ID NO: 23: copGFP-79_AS_Ap 5'3'S
SEQ ID NO: 24: copGFP-79_AS_Ap 5'S
SEQ ID NO: 25: copGFP-79_AS_Ap 3'S
SEQ ID NO: 26: copGFP-79_AS_Ap 5'3'SS
SEQ ID NO: 27: copGFP-79_AS_Ap 5'SS
SEQ ID NO: 28: copGFP-79_AS_Ap 3'SS
SEQ ID NO: 29: copGFP-79_AS_E 3'SSS
SEQ ID NO: 30: copGFP-79_AS_E 3'SSSS
SEQ ID NO: 31: copGFP-35_S_A 3'SSS
SEQ ID NO: 32: copGFP-35_S_A 3'SSSS
SEQ ID NO: 33: copGFP-79_AS_E 3'SSp11
SEQ ID NO: 34: copGFP-79_AS_E 3'SSp21
SEQ ID NO: 35: copGFP-79_AS_E 3'SSp31
SEQ ID NO: 36: copGFP-79_AS_E 3'SSp41
SEQ ID NO: 37: copGFP-79_AS_E 3'SSp51
SEQ ID NO: 38: copGFP-79_AS_E 3'SSp61
SEQ ID NO: 39: copGFP-79_AS_E 3'SSp71
SEQ ID NO: 40: copGFP_forAS_E79 A35 5' p-met ×2
SEQ ID NO: 41: copGFP_forAS_E79 A35 3' p-met ×2
SEQ ID NO: 42: copGFP_forAS_E79 A35 5' p-etho ×2
SEQ ID NO: 43: copGFP_forAS_E79 A35 3' p-etho ×2
SEQ ID NO: 44: copGFP_forAS_E79 A35 3' p-etho ×1
SEQ ID NO: 45: copGFP_forAS_E79 A35 3' p-etho ×2
SEQ ID NO: 46: copGFP-79_AS_E LNA0
SEQ ID NO: 47: copGFP-79_AS_E LNA-1+0+1
SEQ ID NO: 48: copGFP-79_AS_E LNA-1+1
SEQ ID NO: 49: copGFP-79_AS_E LNA-3-1+1+3
SEQ ID NO: 50: copGFP-79_AS_E LNA-5-3-1+1+3+5
SEQ ID NO: 51: copGFP-79_AS_E LNA-2+2
SEQ ID NO: 52: copGFP-79_AS_E LNA-2-1+1+2
SEQ ID NO: 53: copGFP-79_AS_E LNA-3-2+2+3
SEQ ID NO: 54: copGFP-79_AS_E LNA-3-2-1+1+2+3
SEQ ID NO: 55: copGFP-79_AS_E LNA-7-5-3-1+1+3+5+7
SEQ ID NO: 56: copGFP-79_AS_E LNA-9-7-5-3-1+1+3+5+7+9
SEQ ID NO: 57: mEGFP-79_AS_E LNA-2,0
SEQ ID NO: 58: mEGFP-35_S_A 3' p-etho×1
SEQ ID NO: 59: mEGFP-65_AS_Ap
SEQ ID NO: 60: 65Ap_AS_Gap1
SEQ ID NO: 61: 65Ap_AS_Gap2
SEQ ID NO: 62: 65Ap_AS_Gap3
SEQ ID NO: 63: 65Ap_AS_Gap6
SEQ ID NO: 64: 65Ap_AS_Gap9
SEQ ID NO: 65: copGFP-79_AS_Ap 3-overlap
SEQ ID NO: 66: copGFP-79_AS_Ap 6-overlap
SEQ ID NO: 67: copGFP-79_AS_Ap 9-overlap
SEQ ID NO: 68: copGFP-79_AS_Ap 12-overlap
SEQ ID NO: 69: copGFP-79_AS_Ap 15-overlap
SEQ ID NO: 70: copGFP-79_AS_Ap 19-overlap
SEQ ID NO: 71: copGFP-79_AS_Ap 29-overlap
SEQ ID NO: 72: copGFP-79_AS_Ap 39-overlap
SEQ ID NO: 73: copGFP-153-79b-AS-45TD3'-ODN5'
SEQ ID NO: 74: copGFP-153-79b-AS-55TD3'-ODN5'
SEQ ID NO: 75: copGFP-153-79b-AS-65TD3'-ODN5'
SEQ ID NO: 76: copGFP-153-79b-AS-75TD3'-ODN5'
SEQ ID NO: 77: copGFP-153-79b-AS-45TD3'-ODN3'
SEQ ID NO: 78: copGFP-153-79b-AS-55TD3'-ODN3'
SEQ ID NO: 79: copGFP-153-79b-AS-65TD3'-ODN3'
SEQ ID NO: 80: copGFP-153-79b-AS-75TD3'-ODN3'
SEQ ID NO: 81: copGFP-153-79b-AS-79TD3'-ODN3'
SEQ ID NO: 82: 79E_AS_45TD3'-ODN3'
SEQ ID NO: 83: 79E_AS_55TD3'-ODN3'
SEQ ID NO: 84: 79E_AS_65TD3'-ODN3'
SEQ ID NO: 85: 79E_AS_75TD3'-ODN3'
SEQ ID NO: 86: 79E_AS_79TD3'-ODN3'
SEQ ID NO: 87: 79GFP_AS_5'-1-mut(+44-WT)
SEQ ID NO: 88: 35mer_for_79GFP_AS_5'-1-mut(+44-WT)
SEQ ID NO: 89: 65Ap_for_79E_AS_5'-1-mut(+44-WT)
SEQ ID NO: 90: 79GFP_AS_5'-11-mut(+34-WT)
SEQ ID NO: 91: 35mer_for_79GFP_AS_5'-11-mut(+34-WT)
SEQ ID NO: 92: 65Ap_for_79E_AS_5'-11-mut(+34-WT)
SEQ ID NO: 93: 79GFP_AS_5'-21-mut(+24-WT)
SEQ ID NO: 94: 35mer_for_79GFP_AS_5'-21-mut(+24-WT)
SEQ ID NO: 95: 65Ap_for_79E_AS_5'-21-mut(+24-WT)
SEQ ID NO: 96: 79GFP_AS_5'-31-mut(+14-WT)
SEQ ID NO: 97: 35mer_for_79GFP_AS_5'-31-mut(+14-WT)
SEQ ID NO: 98: 65Ap_for_79E_AS_5'-31-mut(+14-WT)
SEQ ID NO: 99: 79GFP_AS_5'-41-mut(+4-WT)
SEQ ID NO: 100: 35mer_for_79GFP_AS_5'-41-mut(+4-WT)
SEQ ID NO: 101: 65Ap_for_79E_AS_5'-41-mut(+4-WT)
SEQ ID NO: 102: 79GFP_AS_5'-44-mut(+1-WT)
SEQ ID NO: 103: 35mer_for_79GFP_AS_5'-44-mut(+1-WT)
SEQ ID NO: 104: 65Ap_for_79E_AS_5'-44-mut(+1-WT)
SEQ ID NO: 105: 79GFP_AS_5'-51-mut(-6)
SEQ ID NO: 106: 35mer_for_79GFP_AS_5'-51-mut(-6-WT)
SEQ ID NO: 107: 35A_AS_5'-51-mut(-6-YH)
SEQ ID NO: 108: 65Ap_for_79E_AS_5'-51-mut(-6)
SEQ ID NO: 109: 79GFP_AS_5'-61-mut(-16)
SEQ ID NO: 110: 35mer_for_79GFP_AS_5'-61-mut(-16-WT)
SEQ ID NO: 111: 35A_AS_5'-61-mut(-16-YH)
SEQ ID NO: 112: 65Ap_for_79E_AS_5'-61-mut(-16)
SEQ ID NO: 113: 79GFP_AS_5'-71-mut(-26)
SEQ ID NO: 114: 35mer_for_79GFP_AS_5'-71-mut(-26-WT)
SEQ ID NO: 115: 35A_AS_5'-71-mut(-26-YH)
SEQ ID NO: 116: 65Ap_for_79E_AS_5'-71-mut(-26)
SEQ ID NO: 117: 79GFP_AS_5'-79-mut(-34)
SEQ ID NO: 118: 35mer_for_79GFP_AS_5'-79-mut(-34-WT)
SEQ ID NO: 119: 35A_AS_5'-79-mut(-34-YH)
SEQ ID NO: 120: 65Ap_for_79E_AS_5'-79-mut(-34)
SEQ ID NO: 121: 144E_AS_5'-105-mut
SEQ ID NO: 122: 109E_AS_5'-70-mut_for_35Ap
SEQ ID NO: 123: 35Ap_for_109E_AS_5'-70-mut
SEQ ID NO: 124: 65E_AS_5'-26-mut_for_79Ap
SEQ ID NO: 125: 79Ap_for_65E_AS_5'-26-mut
SEQ ID NO: 126: 35E_AS_notmut_for_109Ap
SEQ ID NO: 127: 109Ap_5'-105-mut_for_35E_AS_notmut
SEQ ID NO: 128: copGFP-79_AS_E LNA-1+1_5'PS
SEQ ID NO: 129: copGFP_A_prime65_5'PS
SEQ ID NO: 130: mEGFP-79_AS_E LNA-1+1
SEQ ID NO: 131: copGFP-79_AS_E ENA-1+1
SEQ ID NO: 132: nonsense mutation sequence in DMD gene
SEQ ID NO: 133: nonsense mutation sequence in WRN gene
SEQ ID NO: 134: nonsense mutation sequence in HBB gene
SEQ ID NO: 135: nonsense mutation sequence in ADA gene
SEQ ID NO: 136: nonsense mutation sequence in LCA gene
SEQ ID NO: 137: nonsense mutation sequence in PRKN gene
SEQ ID NO: 138: nonsense mutation sequence in BRAF gene
SEQ ID NO: 139: nonsense mutation sequence in NRAS gene
SEQ ID NO: 140: copGFP-153-79b-S-35TDup-ODN(AS_Up_O)
SEQ ID NO: 141: copGFP-153-79b-S-35TD5'-ODN(AS_Up_T)
SEQ ID NO: 142: copGFP-153-79b-S-35TDcn-ODN(AS_Cnt_Y)
SEQ ID NO: 143: copGFP-153-79b-S-35TDcnYH-ODN(AS_Cnt_H)
SEQ ID NO: 144: copGFP-153-79b-S-35TD3'-ODN(AS_Dwn_T)
SEQ ID NO: 145: copGFP-153-79b-S-35TDdown-ODN(AS_Dwn_O)
SEQ ID NO: 146: copGFP-153-79b-AS-35TDup-ODN(S_Up_O)
SEQ ID NO: 147: copGFP-153-79b-AS-35TDcn-ODN(S_Cnt_Y)
SEQ ID NO: 148: copGFP-153-79b-AS-35TDcnYH-ODN(S_Cnt_H)
SEQ ID NO: 149: copGFP-153-79b-AS-35TD5'-ODN(S_Dwn_T)
SEQ ID NO: 150: copGFP-153-79b-AS-35TDdown-ODN(S_Dwn_O)
SEQ ID NO: 151: copGFP_A_prime15
SEQ ID NO: 152: copGFP_A_prime25
SEQ ID NO: 153: copGFP_A_prime45
SEQ ID NO: 154: copGFP_A_prime55
SEQ ID NO: 155: copGFP_A_prime75
SEQ ID NO: 156: copGFP-79_AS_E LNA+1
SEQ ID NO: 157: copGFP-79_AS_E LNA+2
SEQ ID NO: 158: copGFP-79_AS_E LNA+3
SEQ ID NO: 159: copGFP-79_AS_E LNA+4
SEQ ID NO: 160: copGFP-79_AS_E LNA+5
SEQ ID NO: 161: copGFP-79_AS_E LNA+6
SEQ ID NO: 162: copGFP-79_AS_E LNA-1
SEQ ID NO: 163: copGFP-79_AS_E LNA-2
SEQ ID NO: 164: copGFP-79_AS_E LNA-3
SEQ ID NO: 165: copGFP-79_AS_E LNA-4
SEQ ID NO: 166: copGFP-79_AS_E LNA-5
SEQ ID NO: 167: copGFP-79_AS_E LNA-6
SEQ ID NO: 168: copGFP-79_AS_E LNA-2+2
SEQ ID NO: 169: copGFP-79_AS_E LNA-3+3
SEQ ID NO: 170: copGFP-79_AS_E LNA-4+4
SEQ ID NO: 171: copGFP-79_AS_E LNA-5+5
SEQ ID NO: 172: copGFP-79_AS_E LNA-6+6
SEQ ID NO: 173: copGFP-79_AS_E LNA-5-3
SEQ ID NO: 174: copGFP-79_AS_E LNA-4-2
SEQ ID NO: 175: copGFP-79_AS_E LNA-3-1
SEQ ID NO: 176: copGFP-79_AS_E LNA+1+3
SEQ ID NO: 177: copGFP-79_AS_E LNA+2+4
SEQ ID NO: 178: copGFP-79_AS_E LNA+3+5
SEQ ID NO: 179: copGFP-79_AS_E LNA0+1
SEQ ID NO: 180: copGFP-79_AS_E LNA0+2
SEQ ID NO: 181: copGFP-79_AS_E LNA0+3
SEQ ID NO: 182: copGFP-79_AS_E LNA0+4
SEQ ID NO: 183: copGFP-79_AS_E LNA0+5
SEQ ID NO: 184: copGFP-79_AS_E LNA0+6
SEQ ID NO: 185: copGFP-79_AS_E LNA0+7
SEQ ID NO: 186: copGFP-79_AS_E LNA-1+0
SEQ ID NO: 187: copGFP-79_AS_E LNA-2+0
SEQ ID NO: 188: copGFP-79_AS_E LNA-3+0
SEQ ID NO: 189: copGFP-79_AS_E LNA-4+0
SEQ ID NO: 190: copGFP-79_AS_E LNA-5+0
SEQ ID NO: 191: copGFP-79_AS_E LNA-6+0
SEQ ID NO: 192: copGFP-79_AS_E LNA-7+0
SEQ ID NO: 193: copGFP-79_AS_E LNA-2+0+2
SEQ ID NO: 194: copGFP-79_AS_E LNA-3+0+3
SEQ ID NO: 195: copGFP-79_AS_E LNA-4+0+4
SEQ ID NO: 196: copGFP-79_AS_E LNA-5+0+5
SEQ ID NO: 197: copGFP-79_AS_E LNA-6+0+6
SEQ ID NO: 198: copGFP-79_AS_E LNA-7+0+7
SEQ ID NO: 199: copGFP-79_AS_E LNA-2-1+1+2
SEQ ID NO: 200: copGFP-79_AS_E LNA-3-2+2+3
SEQ ID NO: 201: copGFP-79_AS_E LNA-3-2-1+1+2+3
SEQ ID NO: 202: copGFP-79_AS_E LNA-1+1+4
SEQ ID NO: 203: copGFP-79_AS_E LNA-3-1+1+3+4
SEQ ID NO: 204: copGFP-79_AS_E LNA0+1+4
SEQ ID NO: 205: copGFP-79_AS_E LNA-1+0+4
SEQ ID NO: 206: copGFP-79_AS_E LNA0+2+4
SEQ ID NO: 207: copGFP-79_AS_E LNA-2+0+4
SEQ ID NO: 208: copGFP-153-79b-AS-34TD3'-ODN
SEQ ID NO: 209: copGFP-153-79b-AS-33TD3'-ODN
SEQ ID NO: 210: copGFP-153-79b-AS-32TD3'-ODN
SEQ ID NO: 211: copGFP-153-79b-AS-31TD3'-ODN
SEQ ID NO: 212: DMD-79_AS_E
SEQ ID NO: 213: DMD-35_forAS-E_A
SEQ ID NO: 214: DMD-65_forAS-E_Aprime
SEQ ID NO: 215: DMD-79_AS_E LNA0+4
SEQ ID NO: 216: DMD-79_AS_E LNA-4+0
SEQ ID NO: 217: DMD-79_AS_E LNA-1+1
SEQ ID NO: 218: DMD-79_AS_E LNA0
SEQ ID NO: 219: DMD-79_AS_E LNA-2+2
SEQ ID NO: 220: WRN-79_AS_E
SEQ ID NO: 221: WRN-35_forAS-E_A
SEQ ID NO: 222: WRN-65_forAS-E_Aprime
SEQ ID NO: 223: WRN-79_AS_E LNA0+4
SEQ ID NO: 224: WRN-79_AS_E LNA-4+0
SEQ ID NO: 225: WRN-79_AS_E LNA-1+1
SEQ ID NO: 226: WRN-79_AS_E LNA0
SEQ ID NO: 227: WRN-79_AS_E LNA-2+2
SEQ ID NO: 228: SupF top_strand
SEQ ID NO: 229: SupF top_strand p-etho
SEQ ID NO: 230: SupF top_strand S
SEQ ID NO: 231: SupF top_strand LNA-1+1
SEQ ID NO: 232: SupF top_strand LNA-1+1 T>C

Phosphorothioate bond (as modified internucleotide bond)
PCH₃ modification (as modified internucleotide bond)
POCH₂CH₃ modification (as modified internucleotide bond)
2'-O,4'-C-Methylene-crosslinked nucleic acid (as sugar-modified nucleotide)
2'-O,4'-C-Ethylene-crosslinked nucleic acid (as sugar-modified nucleotide)

## Claims

1. A composition comprising a first single-stranded polynucleotide and a third single-stranded polynucleotide, wherein
the first single-stranded polynucleotide is 50 to 200 nucleotides in length, and has a base sequence having a sequence identity of 80% or more with a partial base sequence of an editing target strand in a target region present in an intracellular double-stranded DNA and comprising at least one mutation for editing selected from the group consisting of deletion, substitution, and insertion of one or more nucleotides as compared with a base sequence of the editing target strand, and
the third single-stranded polynucleotide is 15 to 200 nucleotides in length, and has a base sequence having a sequence identity of 90% or more with a base sequence in the editing target strand 5' or 3' to a position corresponding to the mutation for editing in the first single-stranded polynucleotide, wherein, in the editing target strand, the base sequence having sequence identity with the first single-stranded polynucleotide and the base sequence having sequence identity with the third single-stranded polynucleotide are overlapping, adjacent, or separated by one to nine bases.

2. The composition according to claim 1, wherein the position of the mutation for editing in the first single-stranded polynucleotide is present within a range of 25 to 75% from the 5' end, with the full length from the 5' end to the 3' end defined as 100%.

3. The composition according to claim 2, wherein the position of the mutation for editing in the first single-stranded polynucleotide is present within a range of 49 to 51% from the 5' end, with the full length from the 5' end to the 3' end defined as 100%.

4. The composition according to any one of claims 1 to 3, wherein the first single-stranded polynucleotide is 70 to 100 nucleotides in length.

5. The composition according to any one of claims 1 to 4, wherein, in the first single-stranded polynucleotide, a sequence excluding the position of the mutation for editing and a region of 10 bases from the 5' end and a region of 10 bases from the 3' end from the first single-stranded polynucleotide consists of a base sequence 100% identical to a corresponding base sequence in the editing target strand.

6. The composition according to any one of claims 1 to 5, wherein the base sequence of the third single-stranded polynucleotide has a sequence identity of 90% or more with a base sequence in the editing target strand 5' to the position corresponding to the mutation for editing in the first single-stranded polynucleotide.

7. The composition according to any one of claims 1 to 6, wherein, in the editing target strand, the base sequence having sequence identity with the first single-stranded polynucleotide and the base sequence having sequence identity with the third single-stranded polynucleotide are adjacent.

8. The composition according to any one of claims 1 to 7, wherein the third single-stranded polynucleotide is 55 to 75 nucleotides in length.

9. The composition according to any one of claims 1 to 8, wherein the third single-stranded polynucleotide consists of a base sequence 100% identical to a corresponding base sequence in the editing target strand.

10. The composition according to any one of claims 1 to 9, further comprising a second single-stranded polynucleotide, wherein
the second single-stranded polynucleotide is 20 to 200 nucleotides in length, comprises a base sequence complementary to a partial base sequence of a region not comprising the mutation for editing in the first single-stranded polynucleotide, and is capable of forming a double strand with the first single-stranded polynucleotide.

11. The composition according to claim 10, wherein the second single-stranded polynucleotide comprises a base sequence complementary to a part within a region 3' to the position of the mutation for editing in the base sequence of the first single-stranded polynucleotide.

12. The composition according to claim 10 or 11, wherein a molar concentration of the third single-stranded polynucleotide is equal to or higher than a molar concentration of the second single-stranded polynucleotide.

13. The composition according to any one of claims 1 to 12, wherein an abnormal base sequence causative of a disease is comprised in the target region, and the base sequence of the first single-stranded polynucleotide comprises the mutation for editing so that the abnormal base sequence is repaired and thereby the disease is treated or prevented.

14. A pharmaceutical composition for treating or preventing a disease due to an abnormal base sequence comprised in a target region present in an intracellular double-stranded DNA, the pharmaceutical composition comprising the composition according to claim 13.

15. A method for treating or preventing a disease due to an abnormal base sequence comprised in a target region present in an intracellular double-stranded DNA, the method comprising administering a therapeutically or prophylactically effective amount of the composition according to claim 13 to a subject.

16. A method for producing an isolated cell having a mutation introduced thereinto or an organism possessing a cell having a mutation introduced thereinto, the method comprising:
introducing the composition according to any one of claims 1 to 13 into an isolated cell or a cell constituting a living body of an organism, thereby introducing a mutation into a base sequence in a target region present in an intracellular double-stranded DNA.

17. A composition comprising a first single-stranded polynucleotide, wherein
the first single-stranded polynucleotide is 50 to 200 nucleotides in length, and has a base sequence having a sequence identity of 80% or more with a partial base sequence of an editing target strand in a target region present in an intracellular double-stranded DNA and comprising at least one mutation for editing selected from the group consisting of deletion, substitution, and insertion of one or more nucleotides as compared with a base sequence of the editing target strand, and the composition has at least one of the following features:
(a) at least one nucleotide in a region consisting of the position of the mutation for editing and a 10-base sequence 5' adjacent to the position of the mutation for editing and a 10-base sequence 3' adjacent to the position of the mutation for editing in the first single-stranded polynucleotide is a sugar-modified nucleotide; and
(b) the first single-stranded polynucleotide comprises at least one modified internucleotide bond, and the modified internucleotide bond is absent in a range from a third base 5' to the position of the mutation for editing to a third base 3' to the position of the mutation for editing.

18. The composition according to claim 17, wherein the sugar-modified nucleotide is a nucleotide comprising a 2'-O,4'-C-methylene cross-linkage or a 2'-O,4'-C-ethylene cross-linkage in the ribose ring.

19. The composition according to claim 17 or 18, wherein the first single-stranded polynucleotide comprises one to four modified internucleotide bonds within 13 bases from the 5' end and/or the 3' end.

20. The composition according to any one of claims 17 to 19, wherein the position of the mutation for editing in the first single-stranded polynucleotide is present within a range of 25 to 75% from the 5' end, with the full length from the 5' end to the 3' end defined as 100%.

21. The composition according to claim 20, wherein the position of the mutation for editing in the first single-stranded polynucleotide is present within a range of 49 to 51% from the 5' end, with the full length from the 5' end to the 3' end defined as 100%.

22. The composition according to any one of claims 17 to 21, wherein the first single-stranded polynucleotide is 70 to 100 nucleotides in length.

23. The composition according to any one of claims 17 to 22, further comprising a second single-stranded polynucleotide, wherein
the second single-stranded polynucleotide is 20 to 200 nucleotides in length, comprises a base sequence complementary to a partial base sequence of a region not comprising the mutation for editing in the first single-stranded polynucleotide, and is capable of forming a double strand with the first single-stranded polynucleotide.

24. The composition according to claim 23, wherein the second single-stranded polynucleotide comprises at least one modified internucleotide bond.

25. The composition according to claim 24, wherein the second single-stranded polynucleotide comprises one or two modified internucleotide bonds within three bases from the 5' end and/or the 3' end or one modified internucleotide bond within two bases from the 5' end and/or the 3' end.

26. The composition according to any one of claims 23 to 25, wherein the second single-stranded polynucleotide comprises a base sequence complementary to a part within a region 3' to the position of the mutation for editing in the base sequence of the first single-stranded polynucleotide.

27. The composition according to claim 17, 19, 24, or 25, wherein the modified internucleotide bond or modified internucleotide bonds is/are each a phosphorothioate bond, an alkylphosphonate bond, or a phosphotriester bond.

28. The composition according to any one of claims 17 to 27, further comprising a third single-stranded polynucleotide, wherein
the third single-stranded polynucleotide is 15 to 75 nucleotides in length, and has a base sequence having a sequence identity of 90% or more with a base sequence in the editing target strand 5' or 3' to a position corresponding to the mutation for editing in the first single-stranded polynucleotide, wherein, in the editing target strand, the base sequence having sequence identity with the first single-stranded polynucleotide and the base sequence having sequence identity with the third single-stranded polynucleotide are overlapping, adjacent, or separated by one to nine bases.

29. The composition according to claim 28, wherein the base sequence of the third single-stranded polynucleotide has a sequence identity of 90% or more with a base sequence in the editing target strand 5' to the position corresponding to the mutation for editing in the first single-stranded polynucleotide.

30. The composition according to claim 28 or 29, wherein, in the editing target strand, the base sequence having sequence identity with the first single-stranded polynucleotide and the base sequence having sequence identity with the third single-stranded polynucleotide are adjacent.

31. The composition according to any one of claims 28 to 30, wherein the third single-stranded polynucleotide is 55 to 75 nucleotides in length.

32. The composition according to any one of claims 28 to 31, wherein the third single-stranded polynucleotide consists of a base sequence 100% identical to a corresponding base sequence in the editing target strand.

33. The composition according to any one of claims 28 to 32, wherein a molar concentration of the third single-stranded polynucleotide is equal to or higher than a molar concentration of the second single-stranded polynucleotide.

34. The composition according to any one of claims 17 to 33, wherein an abnormal base sequence causative of a disease is comprised in the target region, and the base sequence of the first single-stranded polynucleotide comprises the mutation for editing so that the abnormal base sequence is repaired and thereby the disease is treated or prevented.

35. A pharmaceutical composition for treating or preventing a disease due to an abnormal base sequence comprised in a target region present in an intracellular double-stranded DNA, the pharmaceutical composition comprising the composition according to claim 34.

36. A method for treating or preventing a disease due to an abnormal base sequence comprised in a target region present in an intracellular double-stranded DNA, the method comprising administering a therapeutically or prophylactically effective amount of the composition according to claim 34 to a subject.

37. A method for producing an isolated cell having a mutation introduced thereinto or an organism possessing a cell having a mutation introduced thereinto, the method comprising:
introducing the composition according to any one of claims 17 to 34 into an isolated cell or a cell constituting a living body of an organism, thereby introducing a mutation into a base sequence in a target region present in an intracellular double-stranded DNA.

38. A pharmaceutical composition comprising a third single-stranded polynucleotide for use in combination with a composition comprising a first single-stranded polynucleotide, wherein
the first single-stranded polynucleotide is 50 to 200 nucleotides in length, and has a base sequence having a sequence identity of 80% or more with a partial base sequence of an editing target strand in a target region present in an intracellular double-stranded DNA and comprising at least one mutation for editing selected from the group consisting of deletion, substitution, and insertion of one or more nucleotides as compared with a base sequence of the editing target strand,
the third single-stranded polynucleotide is 15 to 200 nucleotides in length, and has a base sequence having a sequence identity of 90% or more with a base sequence in the editing target strand 5' or 3' to a position corresponding to the mutation for editing in the first single-stranded polynucleotide, wherein, in the editing target strand, the base sequence having sequence identity with the first single-stranded polynucleotide and the base sequence having sequence identity with the third single-stranded polynucleotide are overlapping, adjacent, or separated by one to nine bases, and
an abnormal base sequence causative of a disease is comprised in the target region, and the base sequence of the first single-stranded polynucleotide comprises the mutation for editing so that the abnormal base sequence is repaired and thereby the disease is treated or prevented.

39. The pharmaceutical composition according to claim 38, wherein the base sequence of the third single-stranded polynucleotide has a sequence identity of 90% or more with a base sequence in the editing target strand 5' to the position corresponding to the mutation for editing in the first single-stranded polynucleotide.

40. The pharmaceutical composition according to claim 38 or 39, wherein, in the editing target strand, the base sequence having sequence identity with the first single-stranded polynucleotide and the base sequence having sequence identity with the third single-stranded polynucleotide are adjacent.

41. The pharmaceutical composition according to any one of claims 38 to 40, wherein the third single-stranded polynucleotide is 55 to 75 nucleotides in length.

42. The composition according to any one of claims 38 to 41, wherein the third single-stranded polynucleotide consists of a base sequence 100% identical to a corresponding base sequence in the editing target strand.

43. The pharmaceutical composition according to any one of claims 38 to 42, wherein the position of the mutation for editing in the first single-stranded polynucleotide is present within a range of 25 to 75% from the 5' end, with the full length from the 5' end to the 3' end defined as 100%.

44. The pharmaceutical composition according to claim 43, wherein the position of the mutation for editing in the first single-stranded polynucleotide is present within a range of 49 to 51% from the 5' end, with the full length from the 5' end to the 3' end defined as 100%.

45. The pharmaceutical composition according to any one of claims 38 to 44, wherein the first single-stranded polynucleotide is 70 to 100 nucleotides in length.

46. The composition according to any one of claims 38 to 45, wherein, in the first single-stranded polynucleotide, a sequence excluding the position of the mutation for editing and a region of 10 bases from the 5' end and a region of 10 bases from the 3' end from the first single-stranded polynucleotide consists of a base sequence 100% identical to a corresponding base sequence in the editing target strand.

47. The pharmaceutical composition according to any one of claims 38 to 46, wherein
the composition comprising the first single-stranded polynucleotide further comprises a second single-stranded polynucleotide, and
the second single-stranded polynucleotide is 20 to 200 nucleotides in length, comprises a base sequence complementary to a partial base sequence of a region not comprising the mutation for editing in the first single-stranded polynucleotide, and is capable of forming a double strand with the first single-stranded polynucleotide.

48. The pharmaceutical composition according to claim 47, wherein the second single-stranded polynucleotide comprises a base sequence complementary to a part within a region 3' to the position of the mutation for editing in the base sequence of the first single-stranded polynucleotide.

49. The pharmaceutical composition according to claim 47 or 48, wherein the pharmaceutical composition is used in such a manner that a molar concentration of the third single-stranded polynucleotide is equal to or higher than a molar concentration of the second single-stranded polynucleotide.

50. The pharmaceutical composition according to any one of claims 38 to 49 for treating or preventing the disease due to an abnormal base sequence comprised in a target region present in an intracellular double-stranded DNA.

51. A method for treating or preventing a disease due to an abnormal base sequence comprised in a target region present in an intracellular double-stranded DNA, the method comprising administering a therapeutically or prophylactically effective amount of the pharmaceutical composition according to claim 50 to a subject together with the composition comprising the first single-stranded polynucleotide simultaneously, separately, or consecutively.

52. A method for producing an isolated cell having a mutation introduced thereinto or an organism possessing a cell having a mutation introduced thereinto, the method comprising:
introducing the pharmaceutical composition according to any one of claims 38 to 50 into an isolated cell or a cell constituting a living body of an organism together with the composition comprising the first single-stranded polynucleotide simultaneously, separately, or consecutively, thereby introducing a mutation into a base sequence in a target region present in an intracellular double-stranded DNA.
